# EUROPEAN PATENT APPLICATION

(11) **EP 4 233 884 A2**
(43) Date of publication of application: **30.08.2023**
(21) Application number: 22207065.8
(22) Date of filing: 23.11.2016
(51) Int. Cl.: A61K 35/741, A61K 35/742, A61P 1/00, A61P 31/04

(54) **DESIGNED BACTERIAL COMPOSITIONS**

(30) Priority: 24.11.2015 US 201562259523 P; 17.06.2016 US 201662351696 P; 28.09.2016 US 201662401011 P
(62) Divisional of application: 16819219.3
(71) Applicant: Seres Therapeutics, Inc., Cambridge, MA 02139 (US)
(72) Inventor: BUTTON, Julie, Lincoln, Massachusetts, 01773 (US); HENN, Matthew, Belmont, Massachusetts, 02478 (US); LITCOFSKY, Kevin, Newtonville, Massachusetts, 02458-1948 (US); MCKENZIE, Gregory, Arlington, Massachusetts, 02474 (US); COOK, David, Brookline, Massachusetts, 02445 (US); MCKENZIE, Mary-Jane, Lombardo, Arlington, Massachusetts 02474 (US); MARTINEZ, Asuncion, Arlington, Massachusetts, 02474 (US); NANDAKUMAR, Madhumitha, Arlington, Massachusetts, 02476 (US)
(74) Representative: Garner, Stephen

(57) **Abstract**

Compositions and methods of treating a gastrointestinal dysbiosis using a limited number of defined bacterial species are provided.

## Description

### FIELD OF THE INVENTION

Disclosed herein are bacterial compositions useful for treating a dysbiosis, e.g., in a human.

### BACKGROUND OF THE INVENTION

Dysbiosis has been implicated in a number of diseases including infection such as infection by *Clostridium difficile* and drug-resistant Enterococcus, as well as in metabolic diseases such as diabetes. Methods of treating a dysbiosis-related condition have included fecal microbiome transplantation (FMT), which can provide microorganisms to the gastrointestinal tract (GI). However, fecal transplant presents a number of issues including those related to safety and methods of delivery such as naso-duodenal-, transcolonoscopic-, or enema-based methods that generally require in-clinic procedures and may introduce adverse events. Treatments using FMT have a likelihood of being inherently inconsistent because of the variability between individuals donating the feces for transplant. FMT methods also introduce a risk of infection by pathogenic organisms, including viruses, bacteria, fungi and protists in the source material. Furthermore, there can be issues related to the stability and storage of donated feces, for example, related to the survival of bacterial species. Some treatments using fecal bacteria delivered in capsules have required that patients take large numbers of capsules, which can be difficult for people with GI illnesses and may reduce compliance with complete treatment. Accordingly, there is a need for compositions that deliver a consistent product containing cultured bacteria that are of sufficient complexity to effectively treat a dysbiosis or dysbiosis-related condition, e.g., *Clostridium difficile* infection, for example, preventing or inhibiting infection, or, preventing or inhibiting recurrence of infection. As used herein, treating and preventing can include a reduction in signs or symptoms of disease.

### SUMMARY OF THE INVENTION

The invention relates to the discovery of bacterial compositions, e.g., bacterial spore compositions containing a consortium of defined types of bacteria, which are useful for treating a dysbiosis. Such a consortium is referred to herein as a Designed Bacterial Composition (DBC).

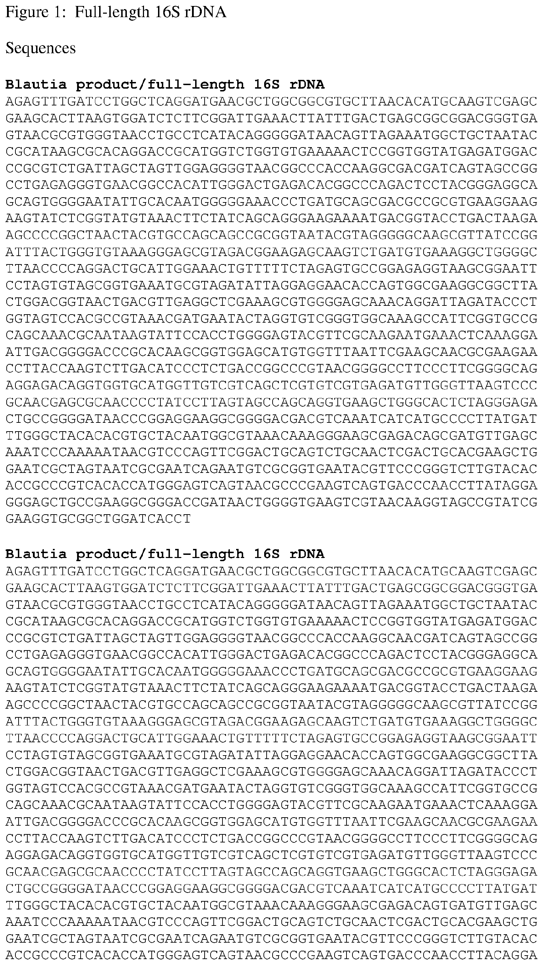

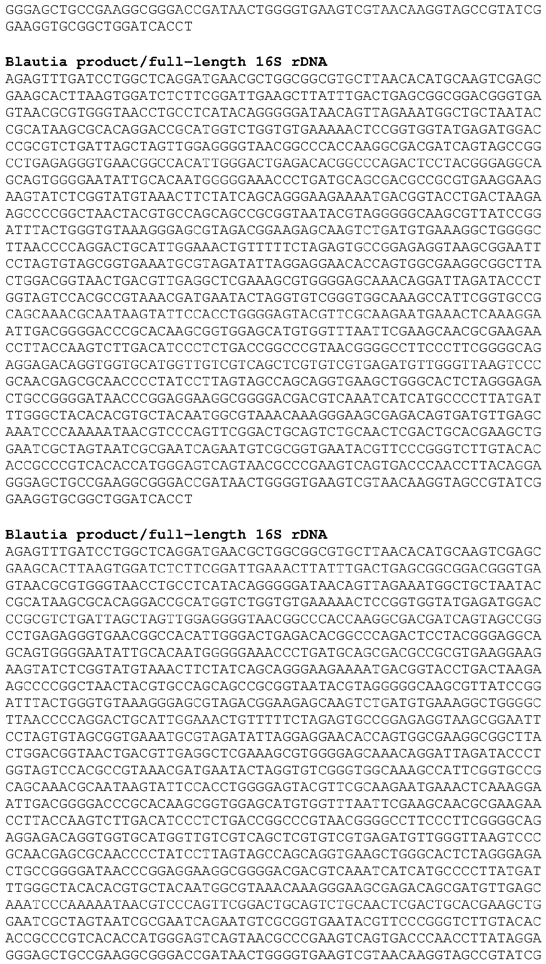

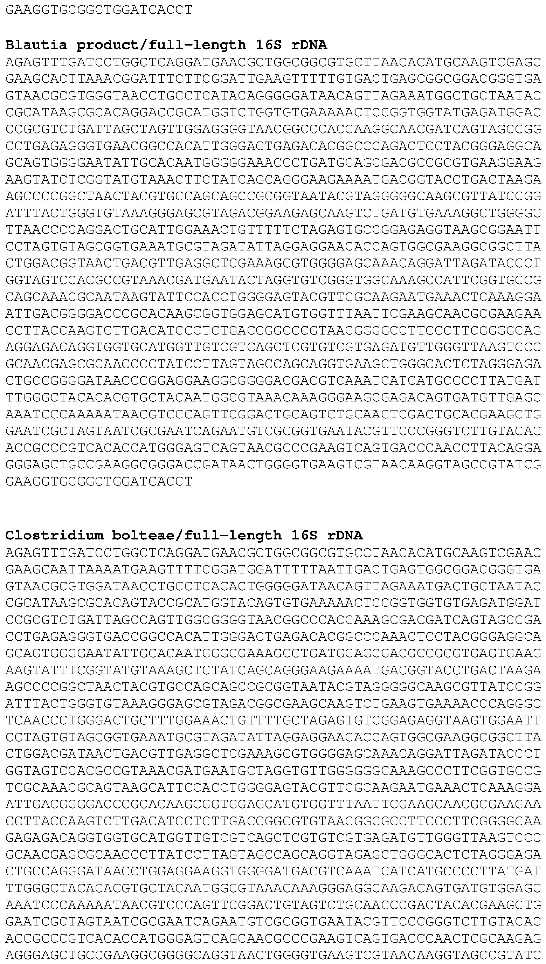

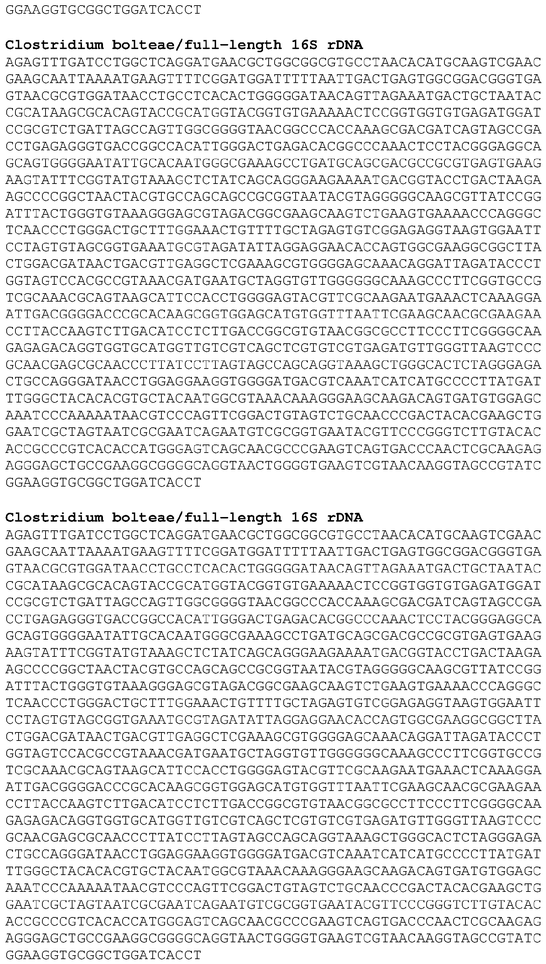

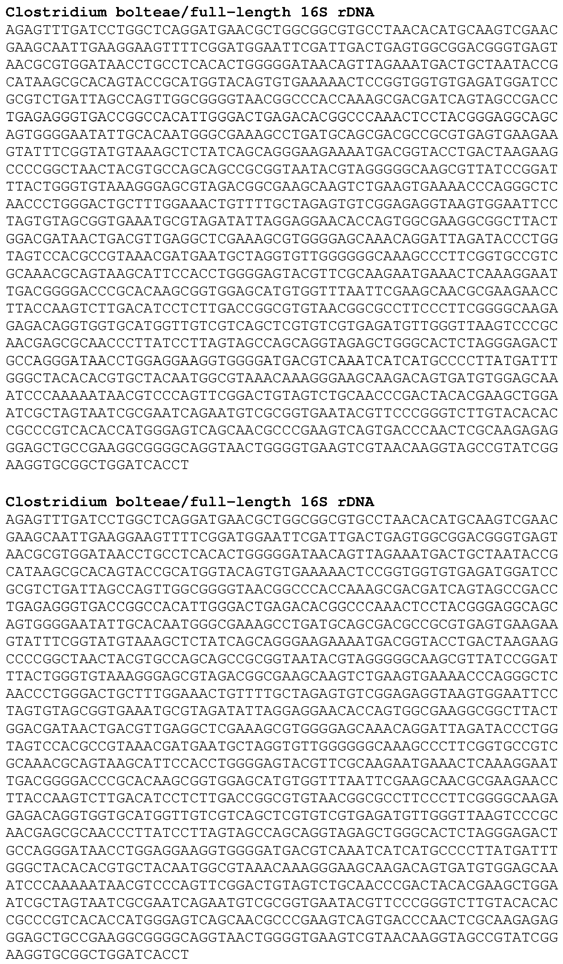

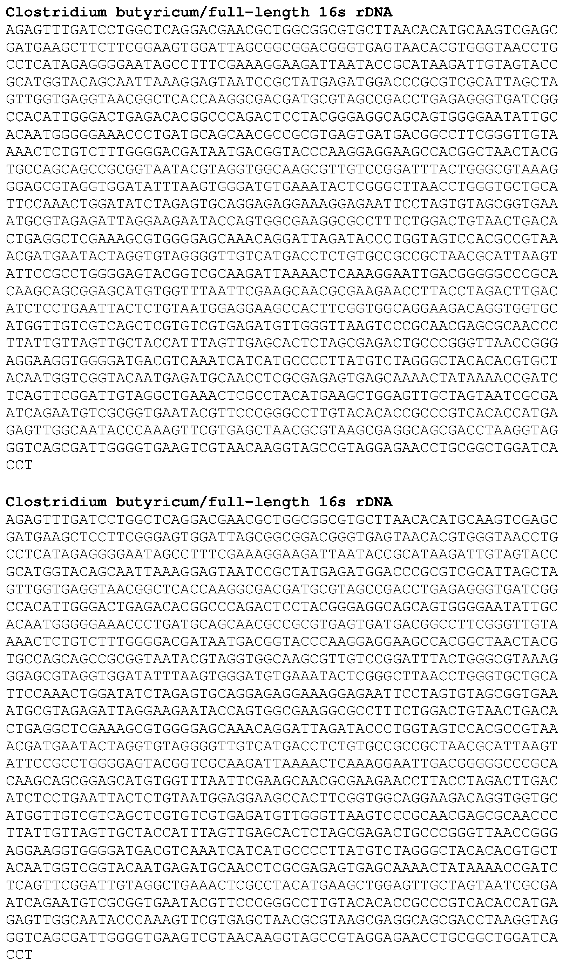

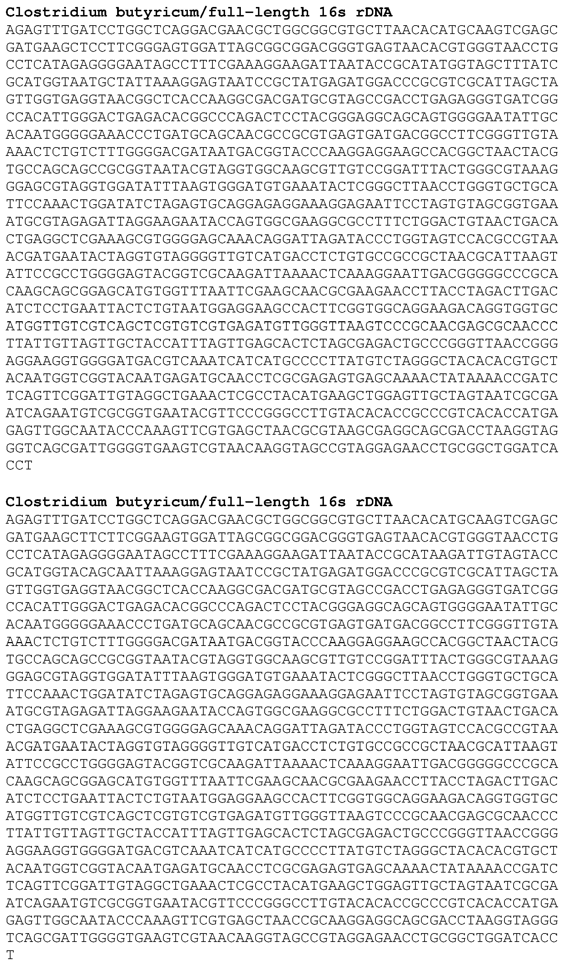

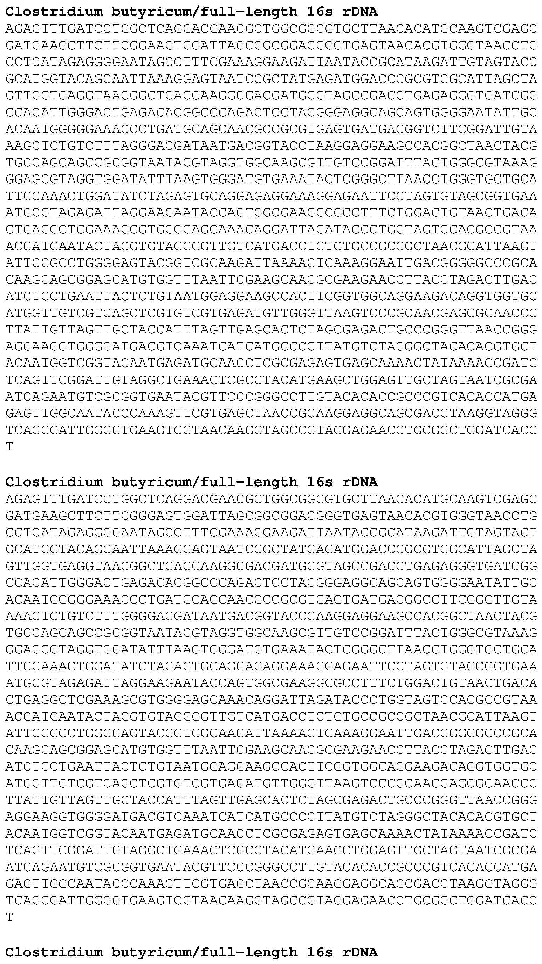

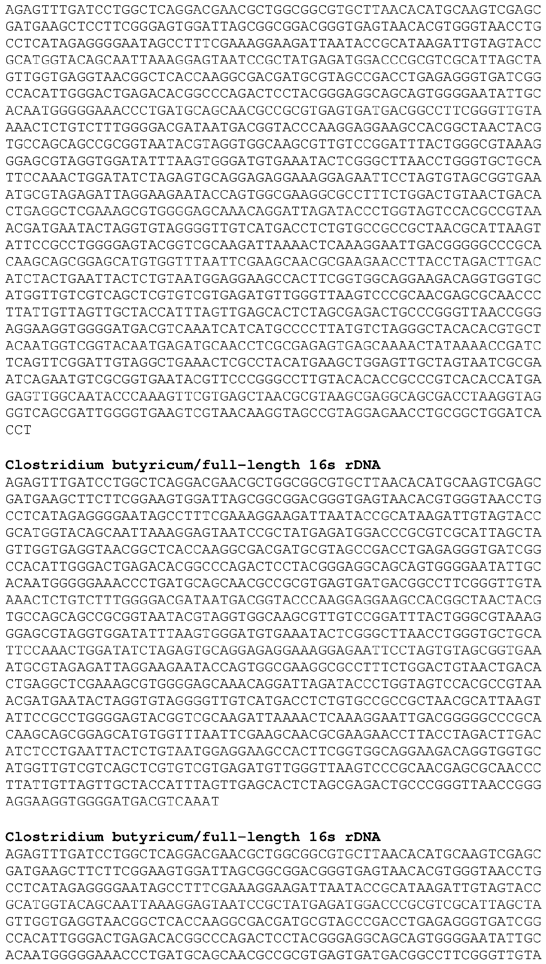

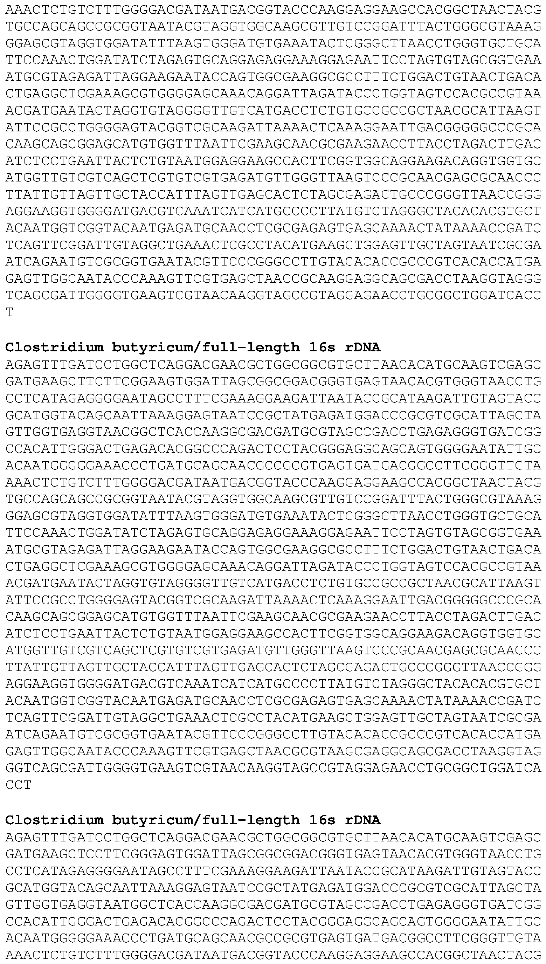

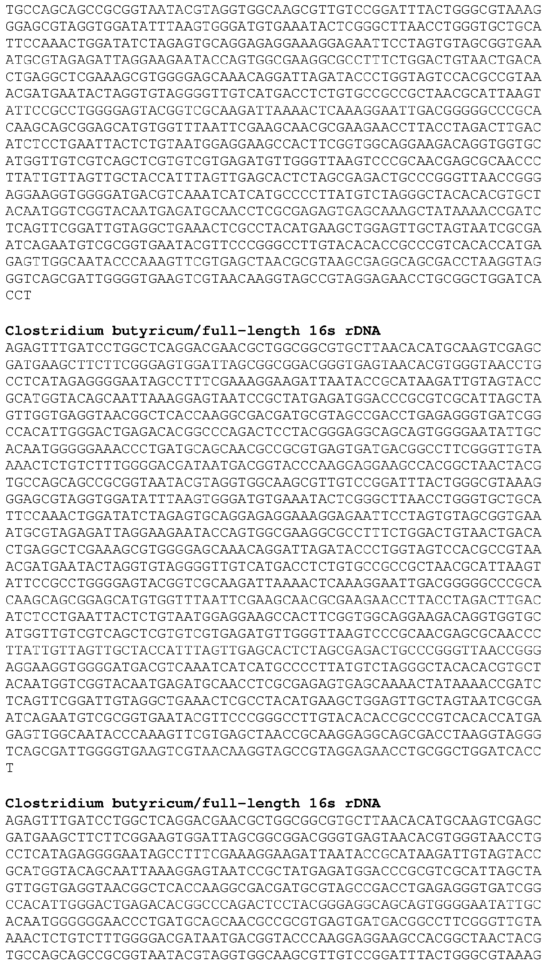

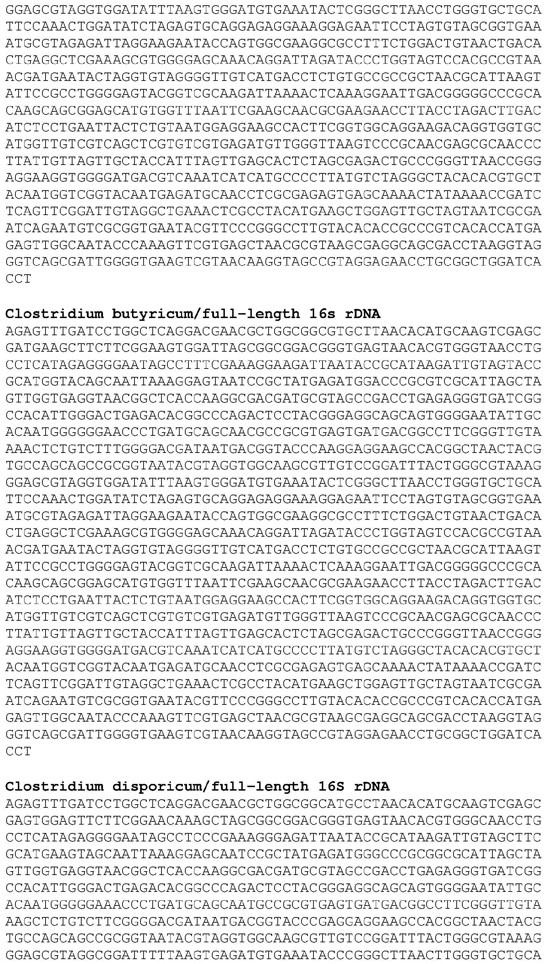

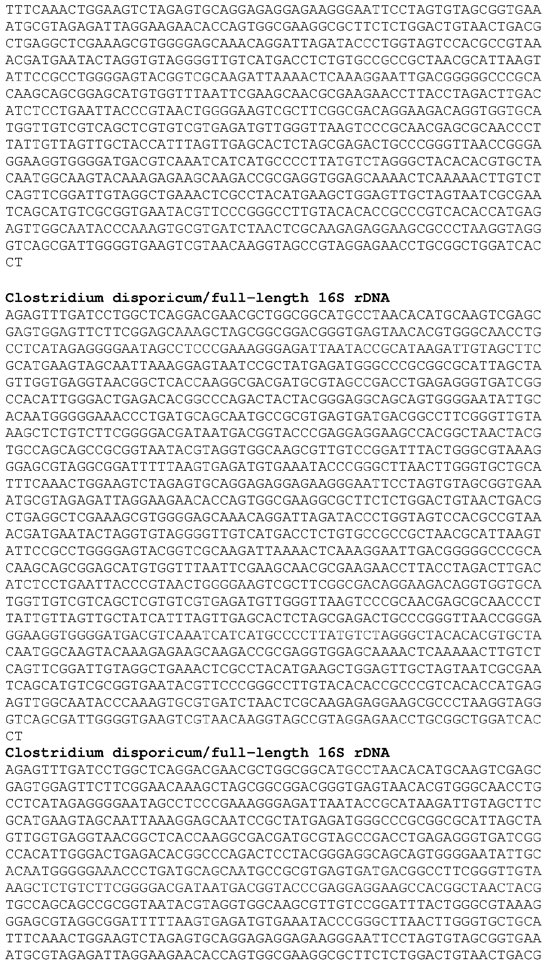

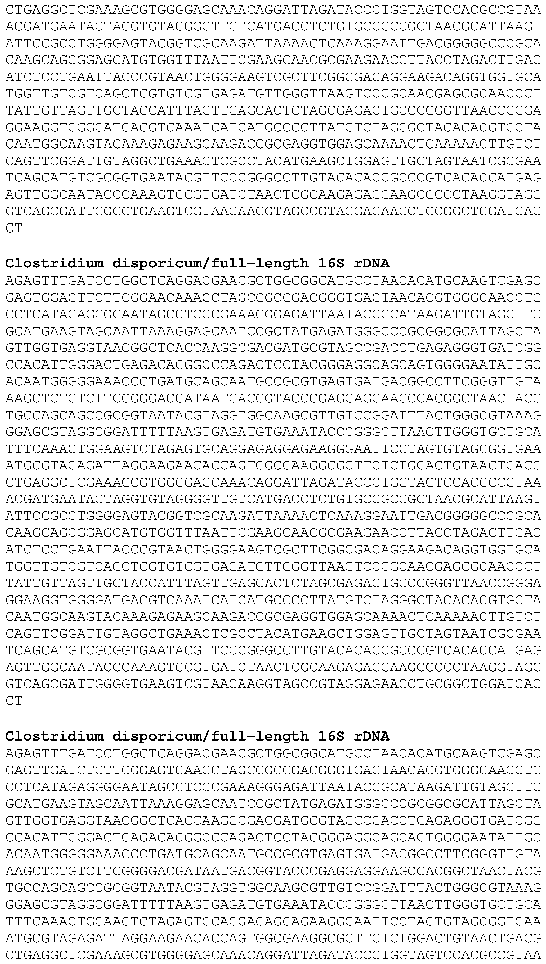

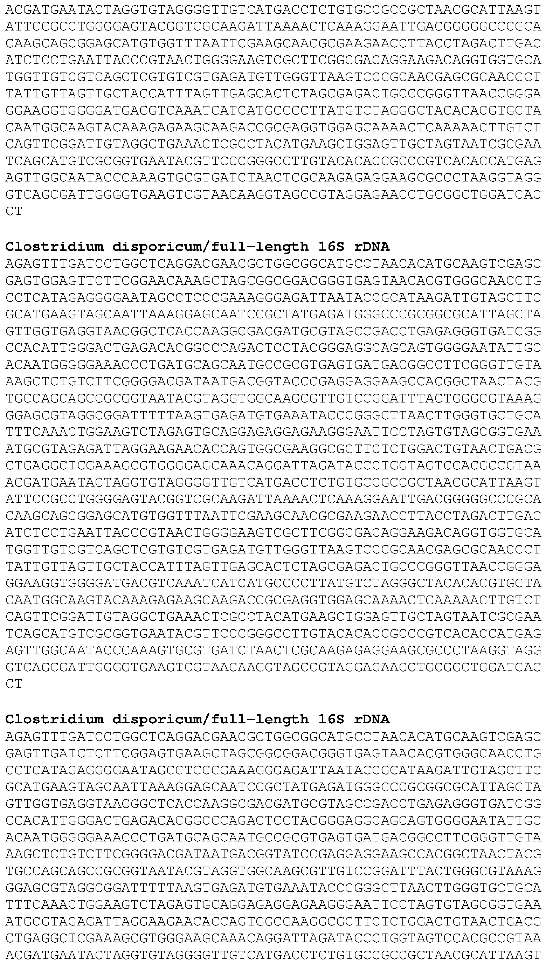

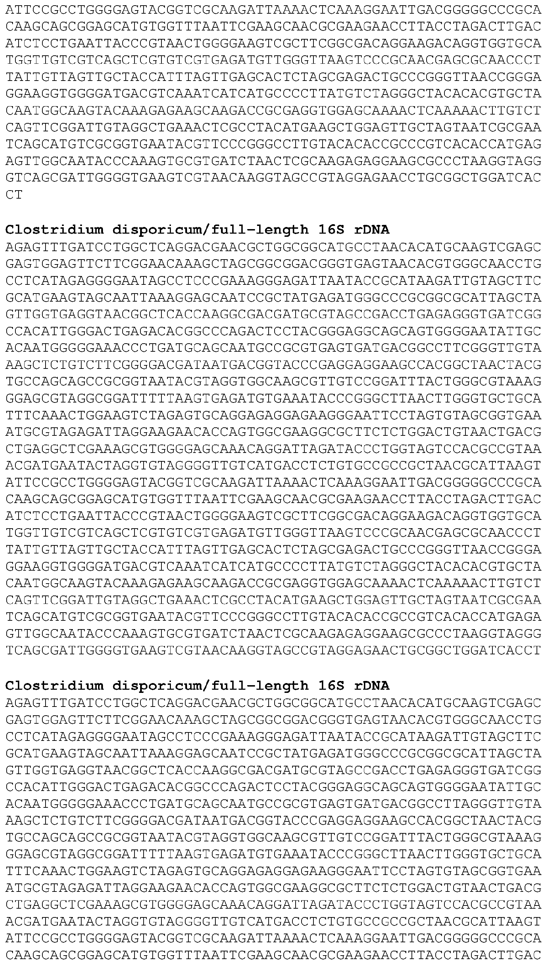

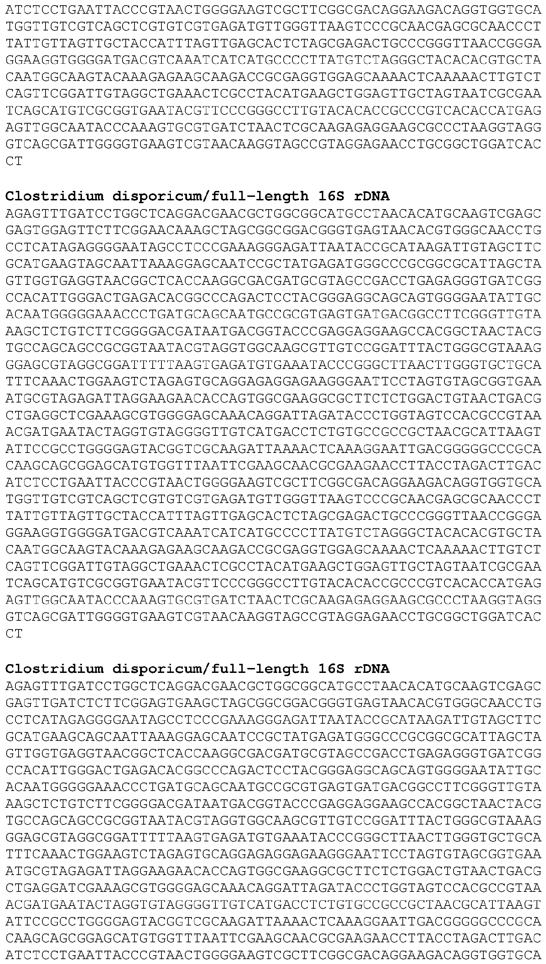

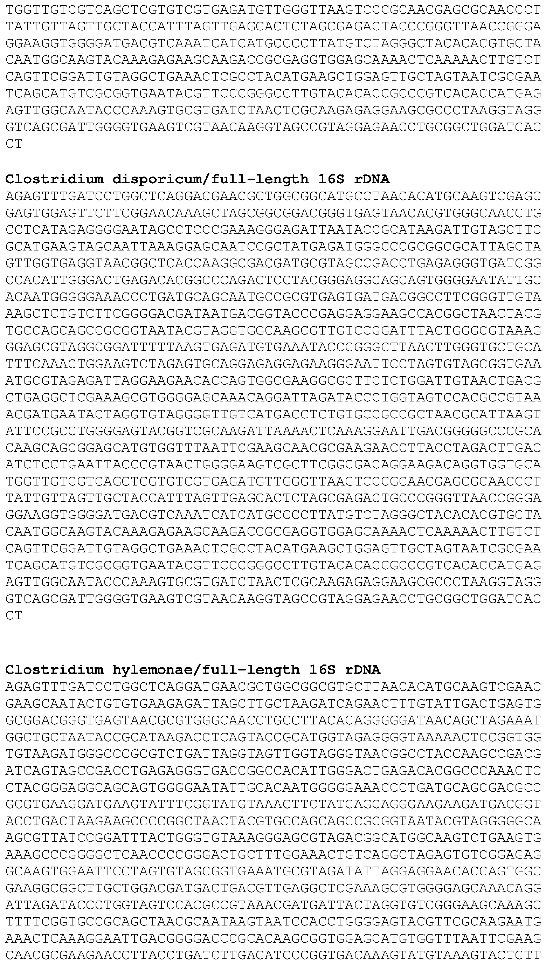

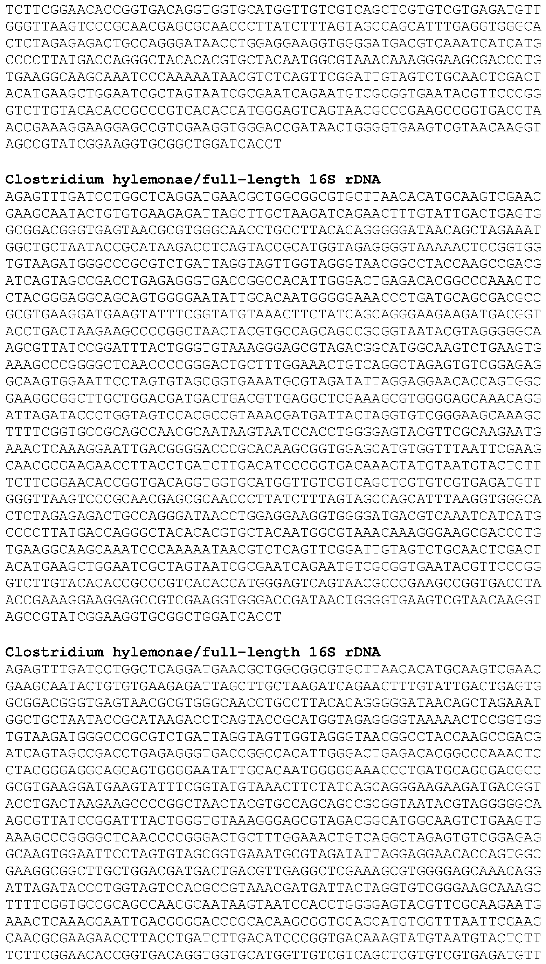

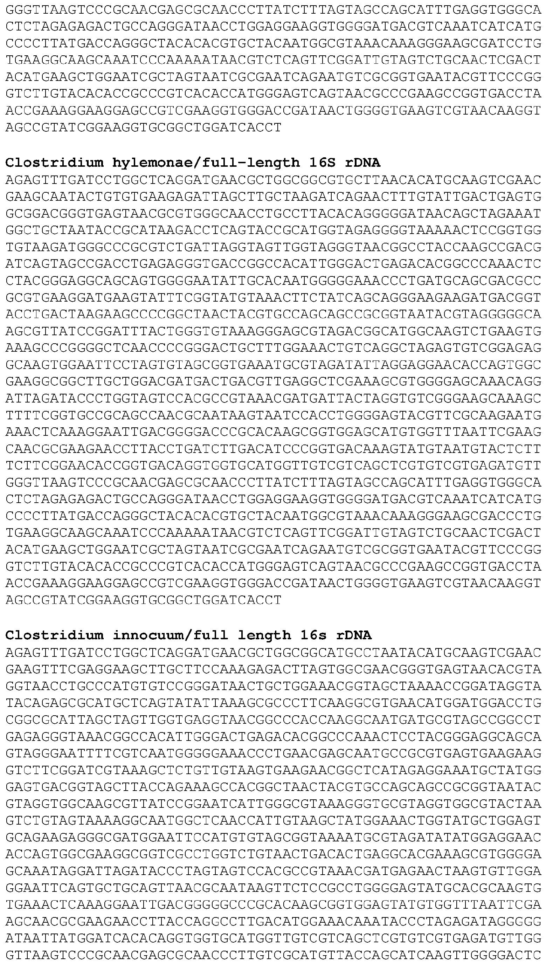

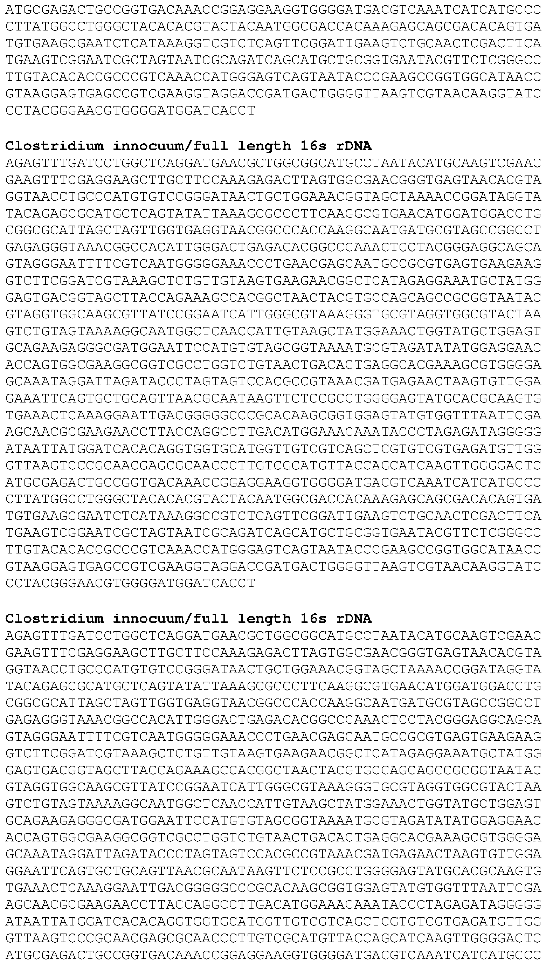

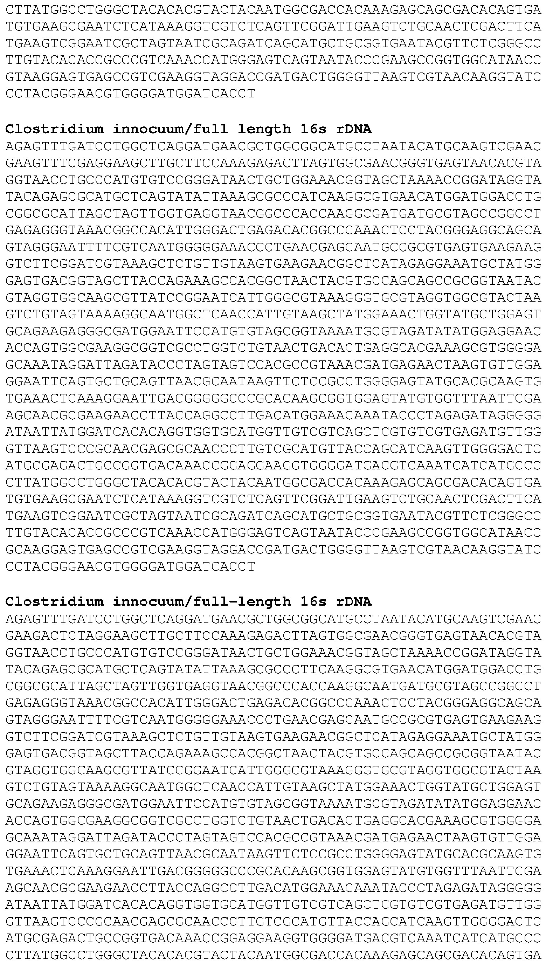

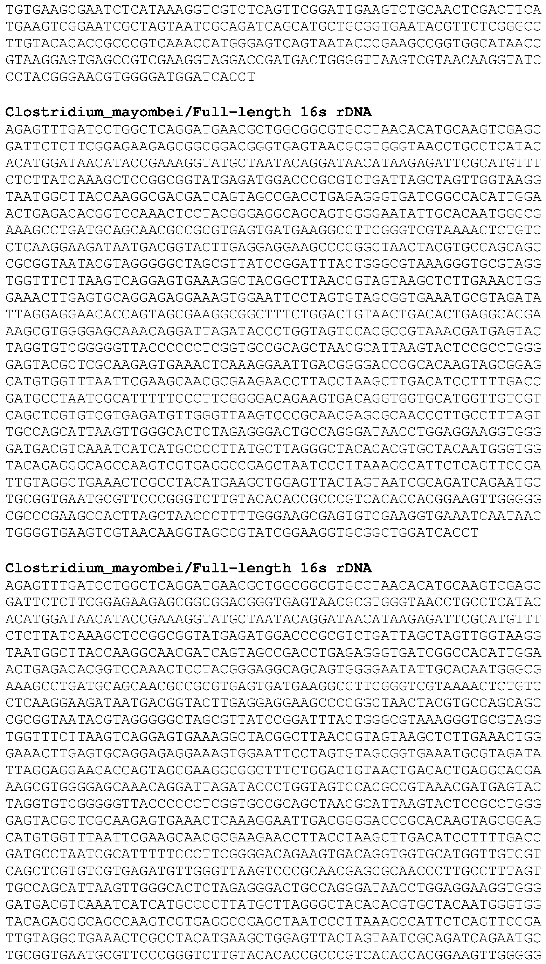

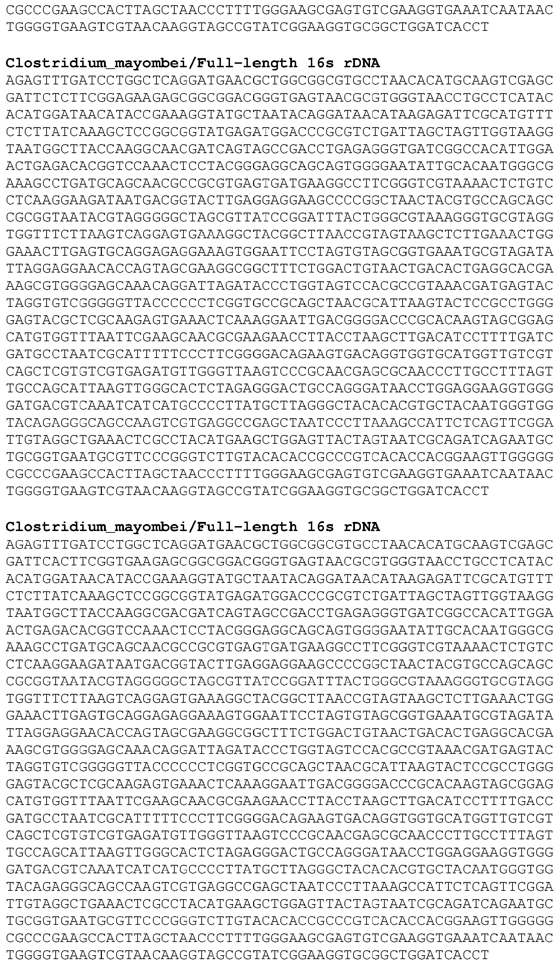

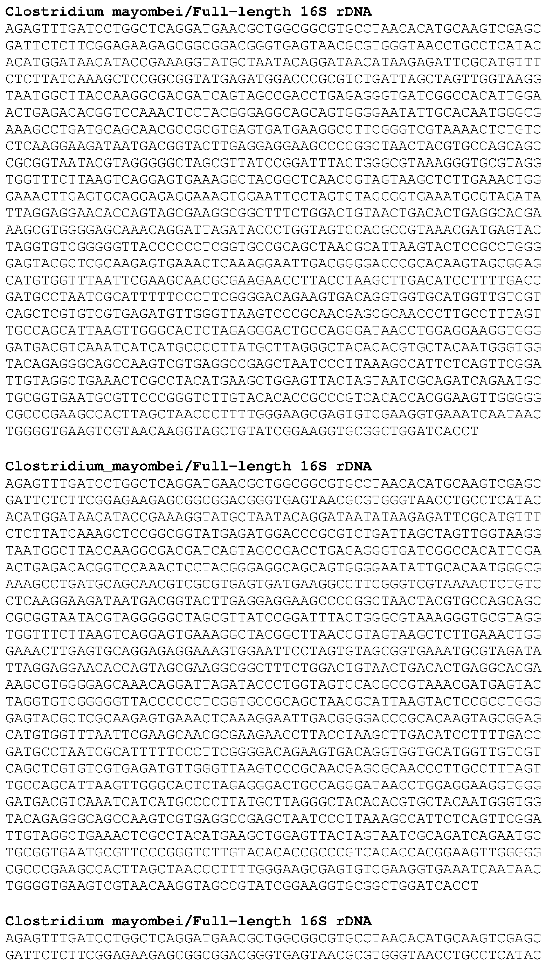

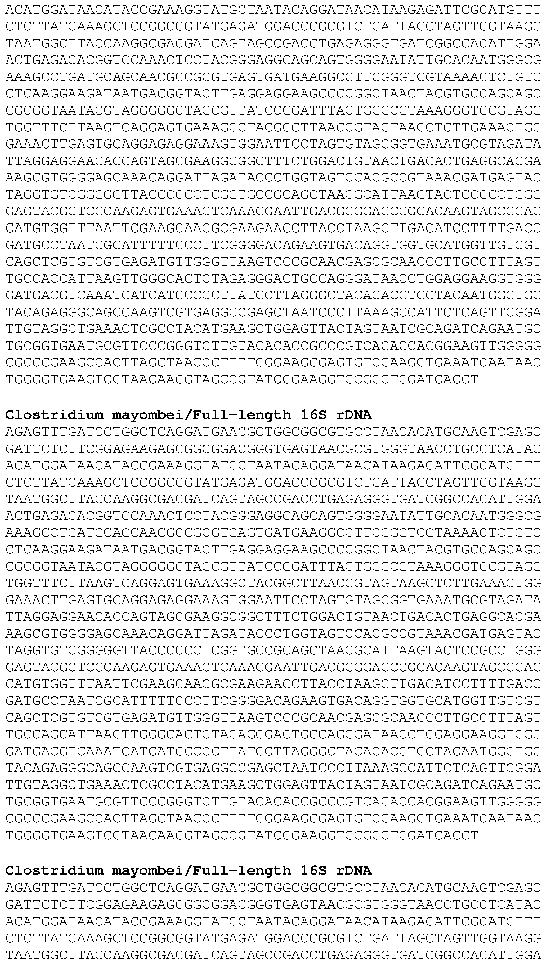

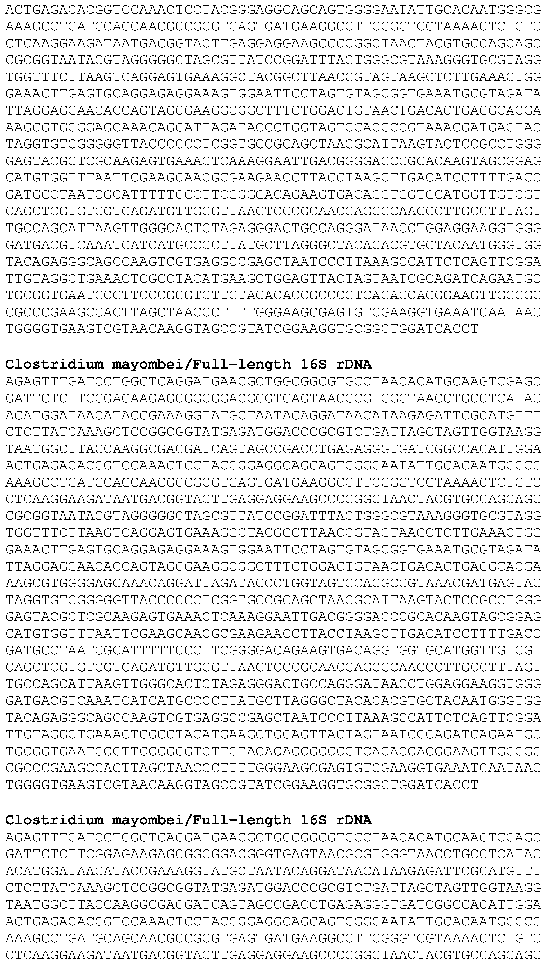

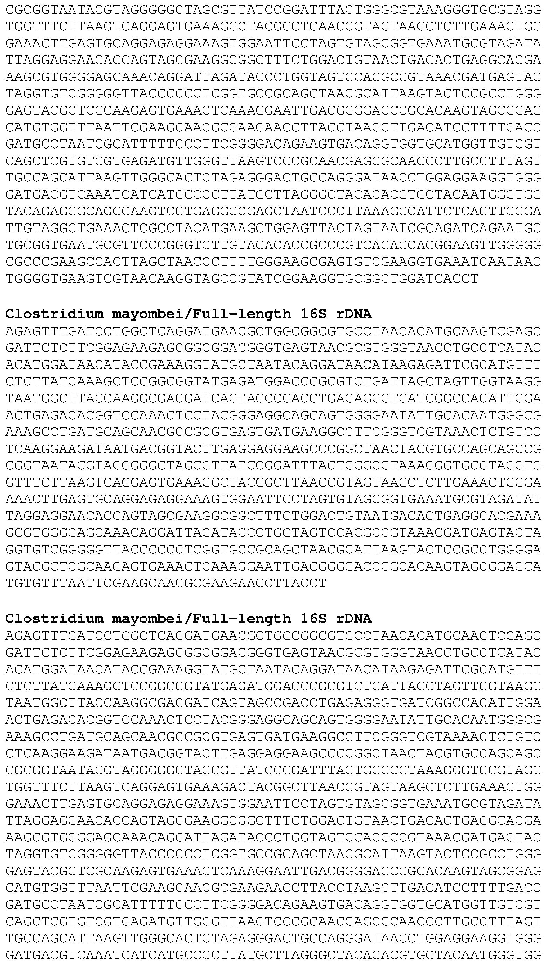

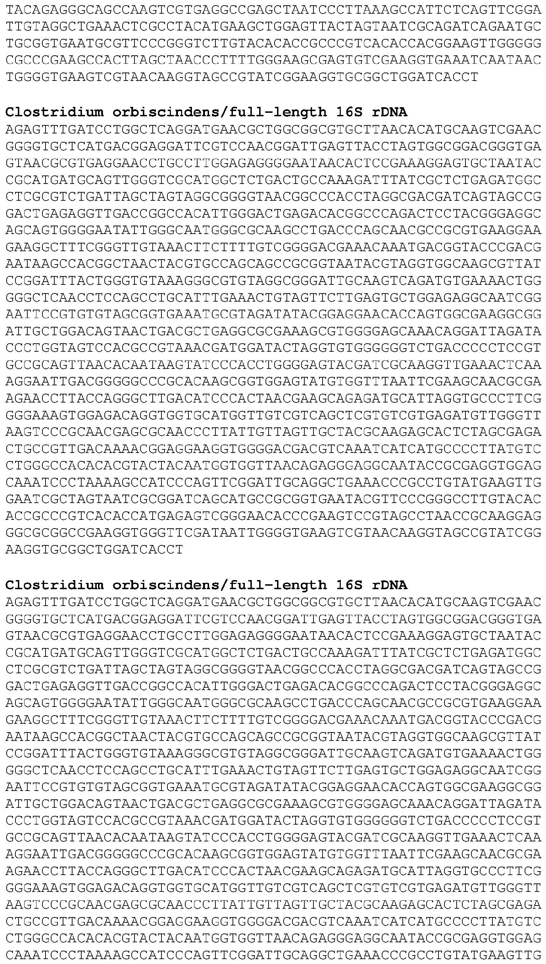

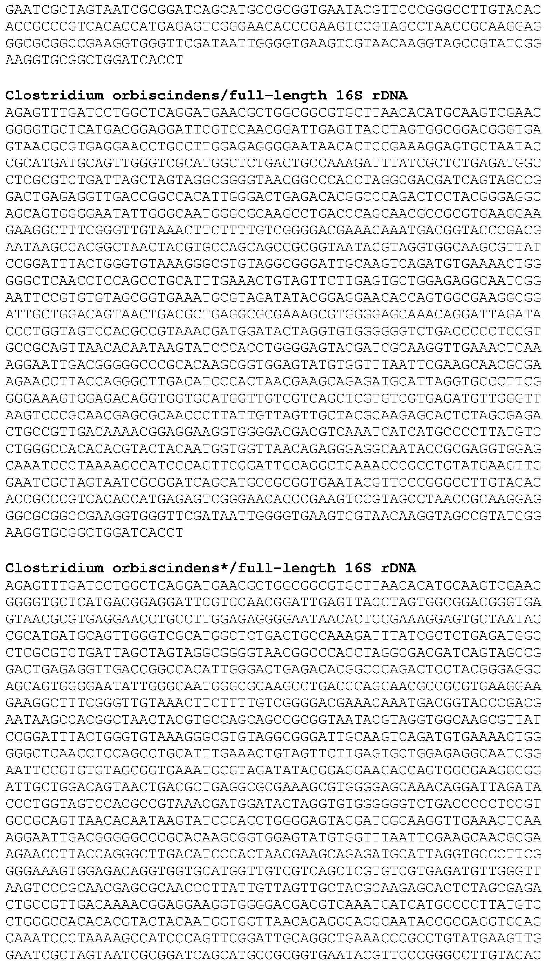

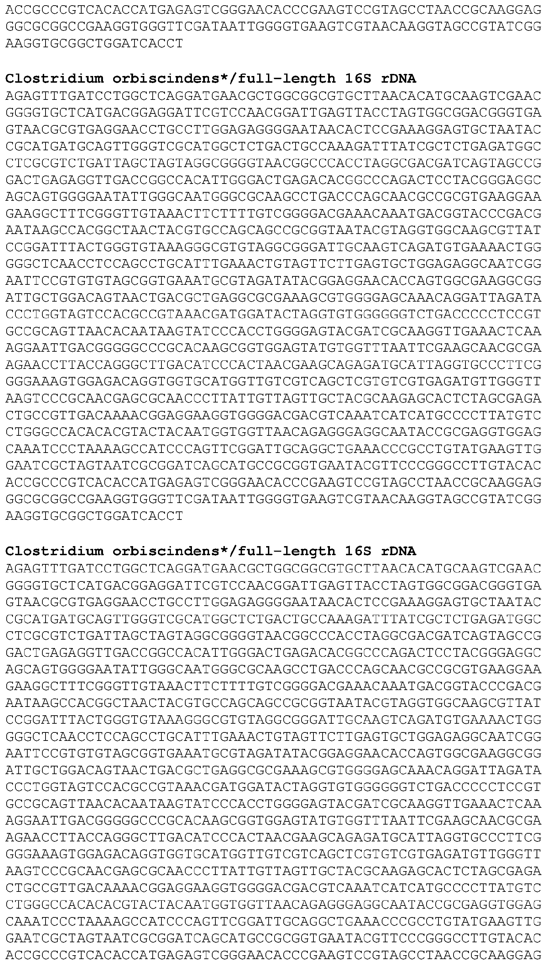

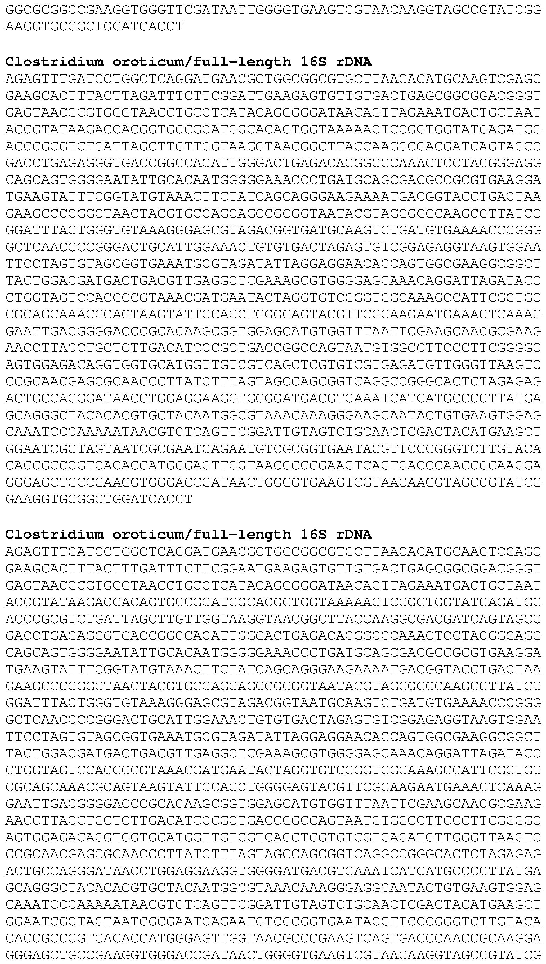

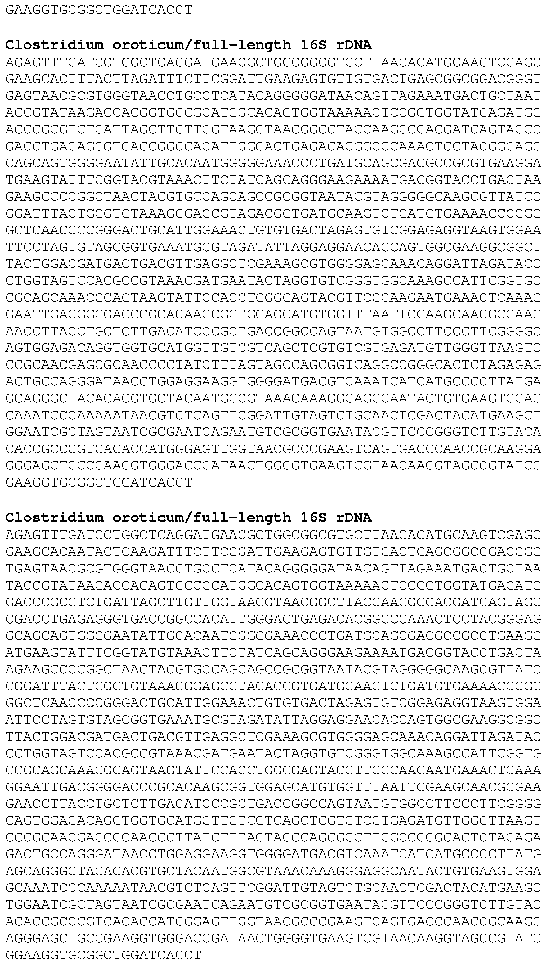

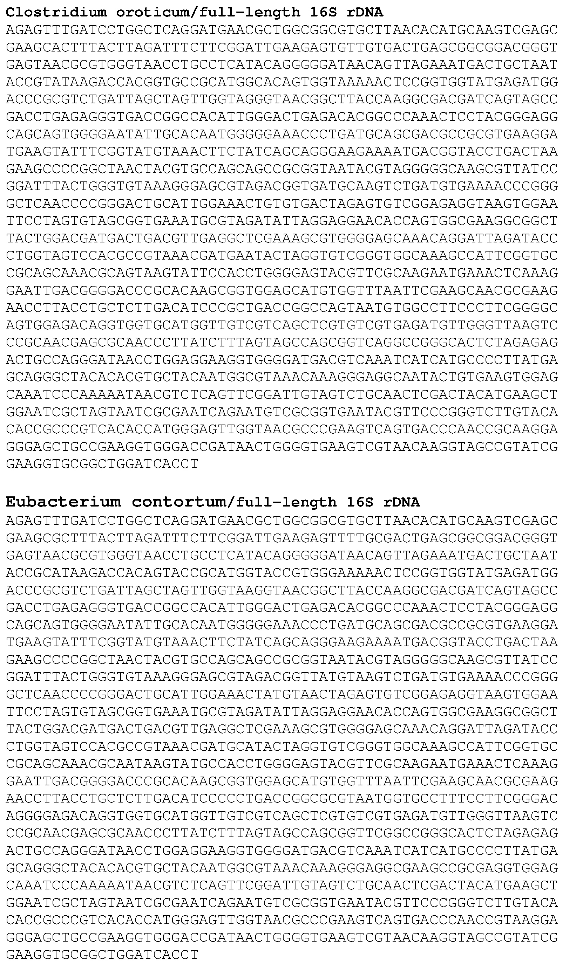

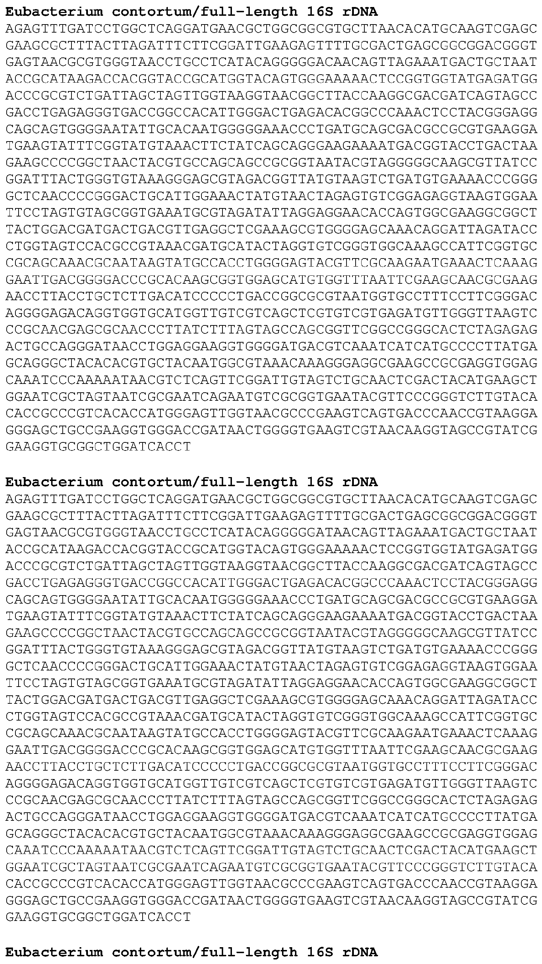

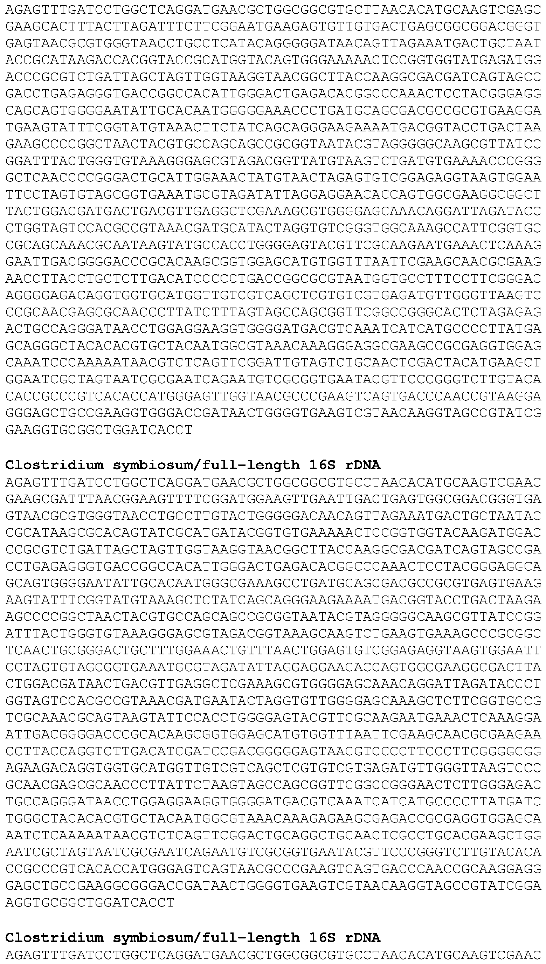

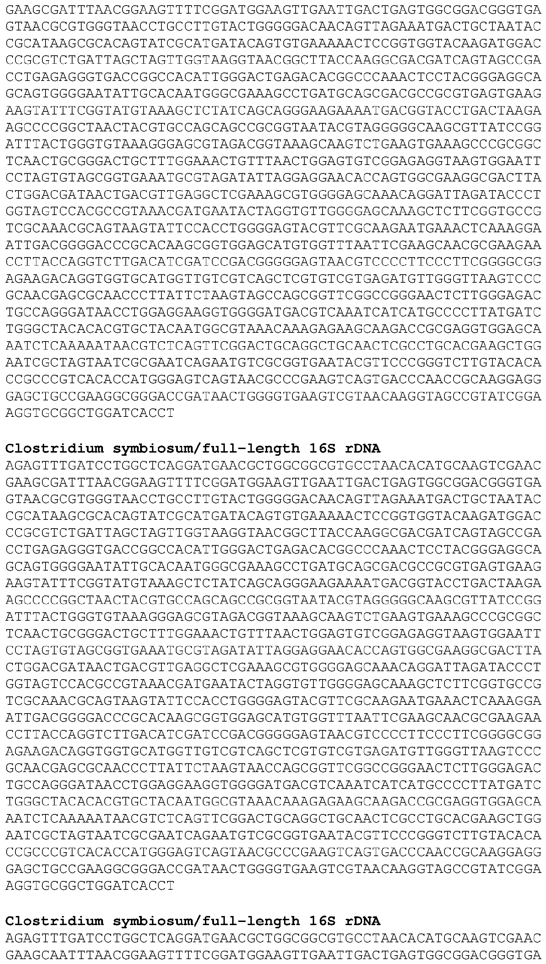

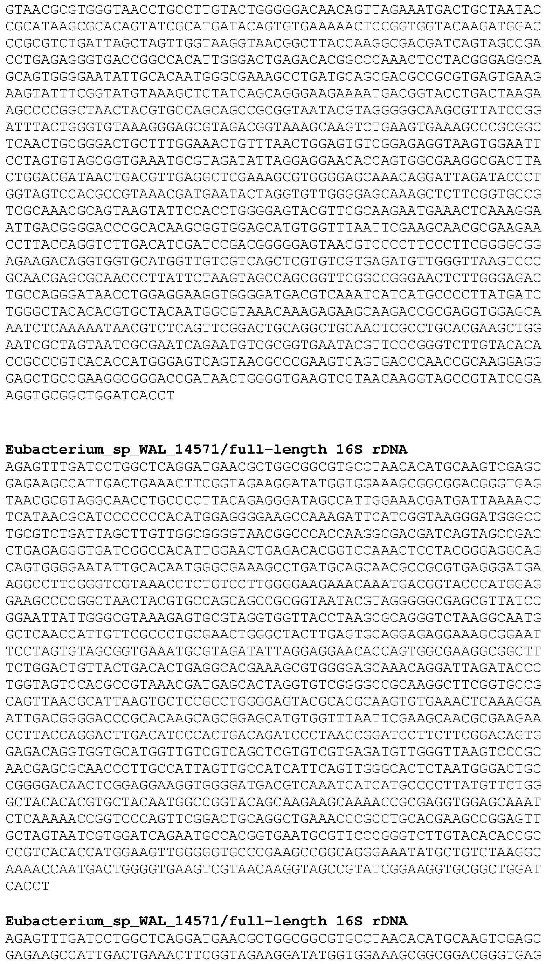

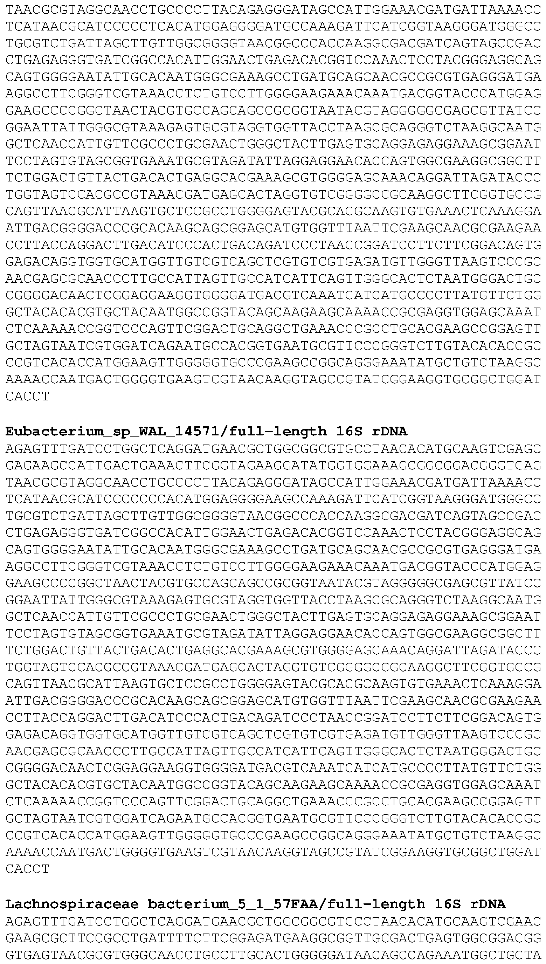

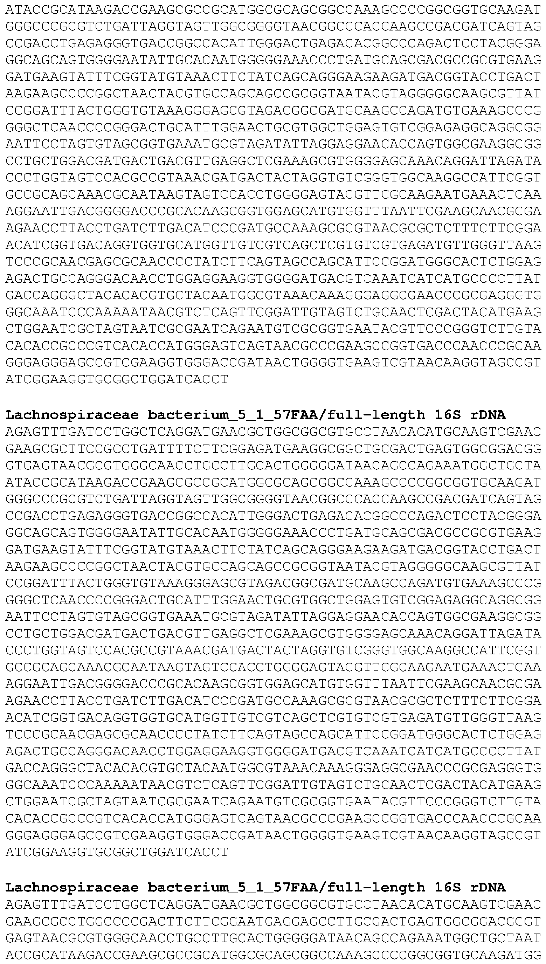

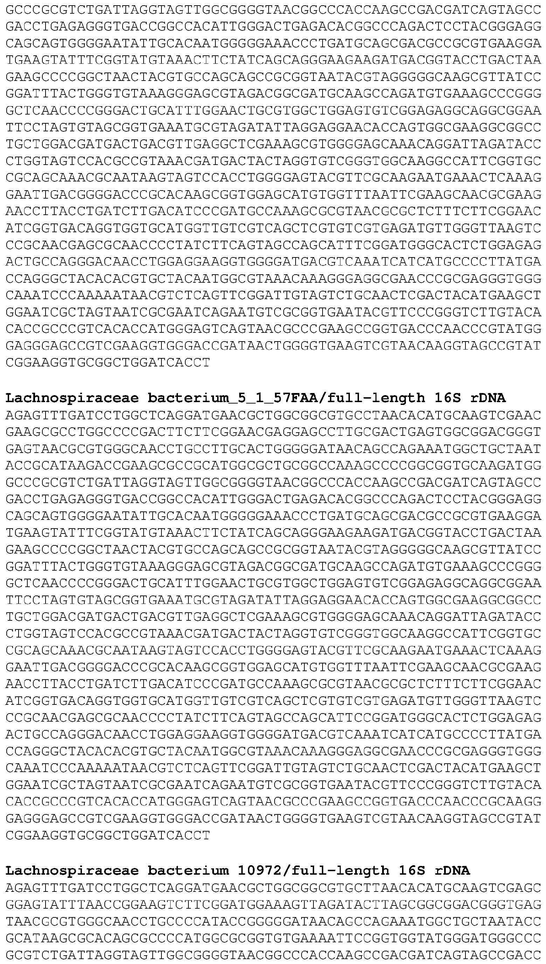

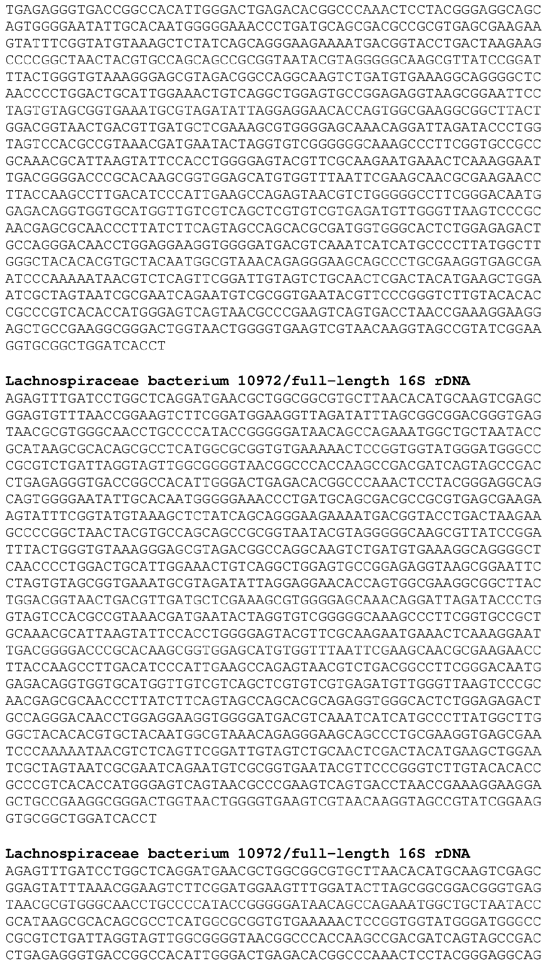

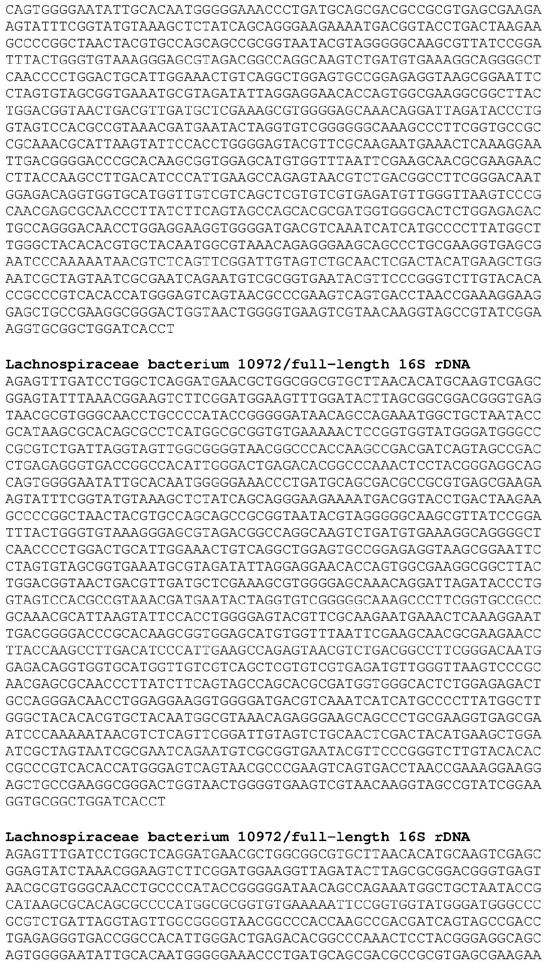

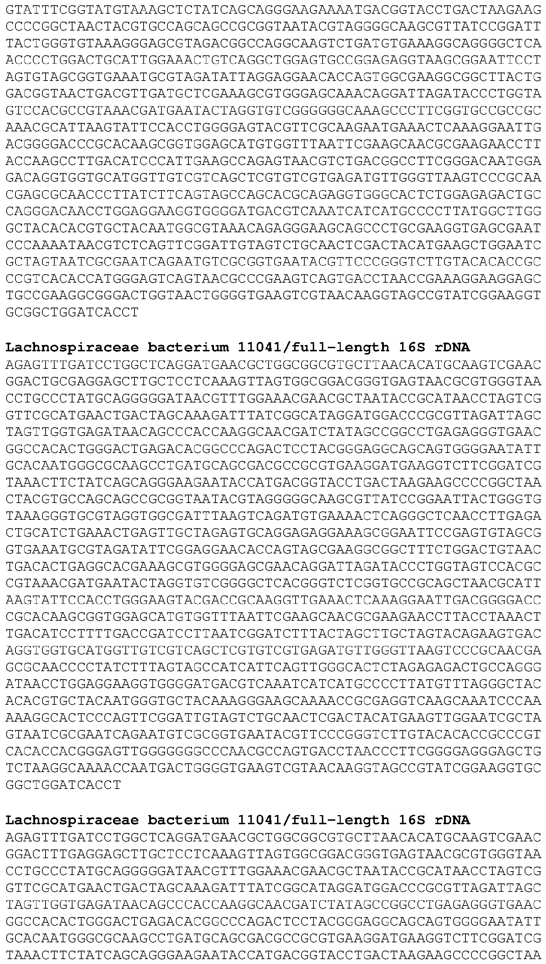

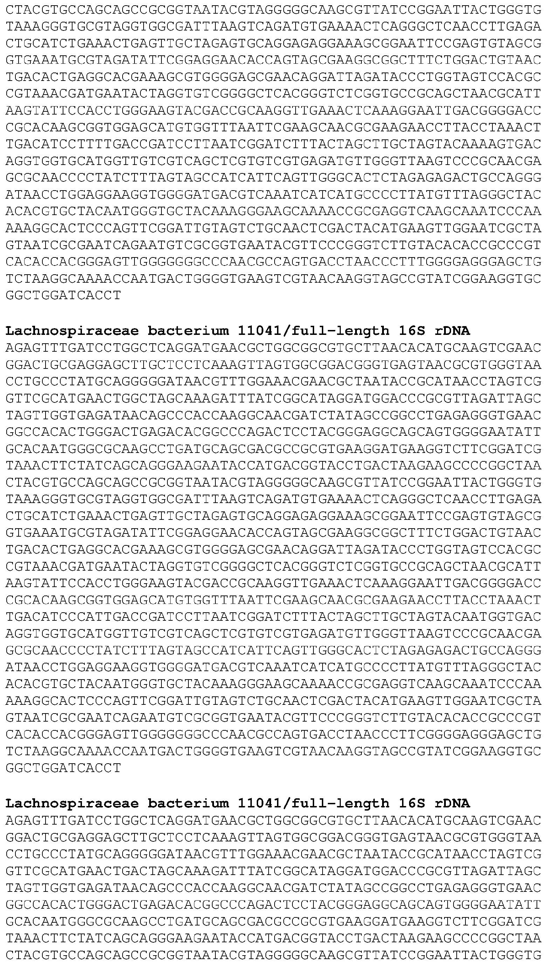

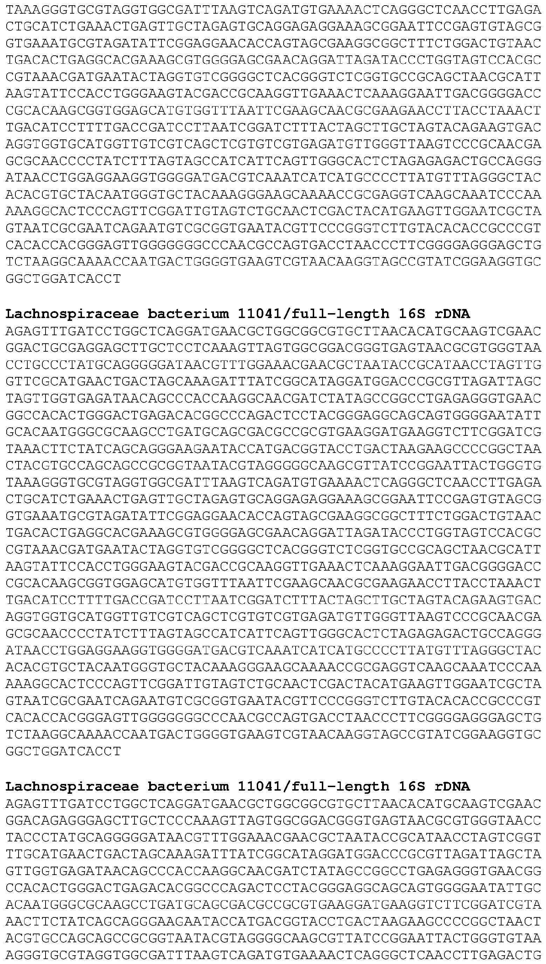

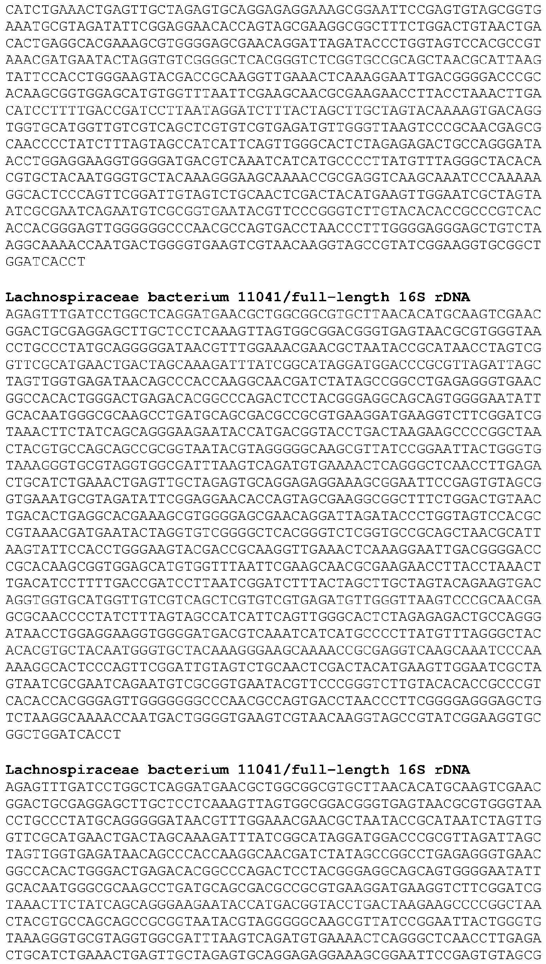

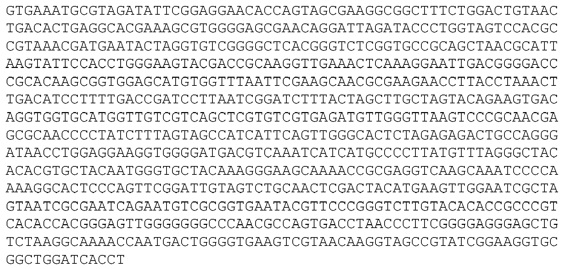

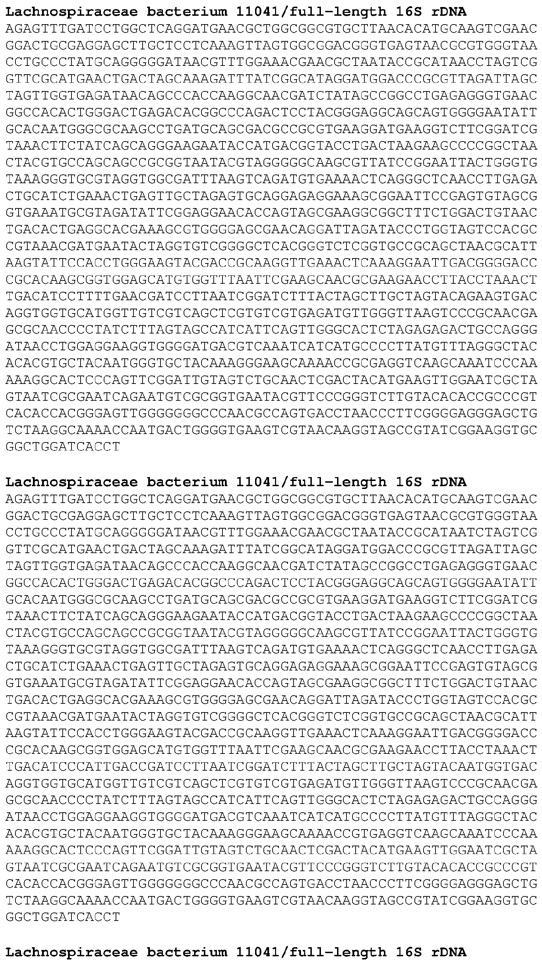

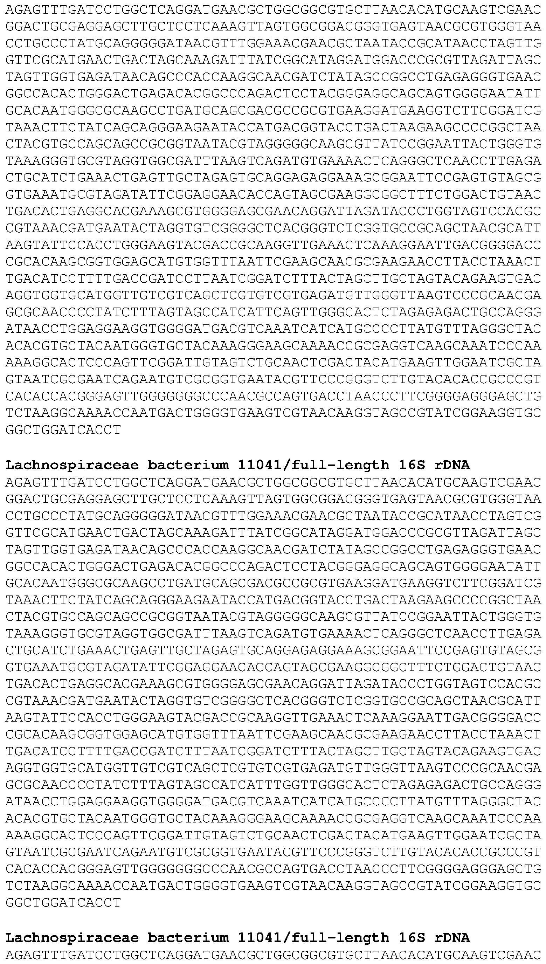

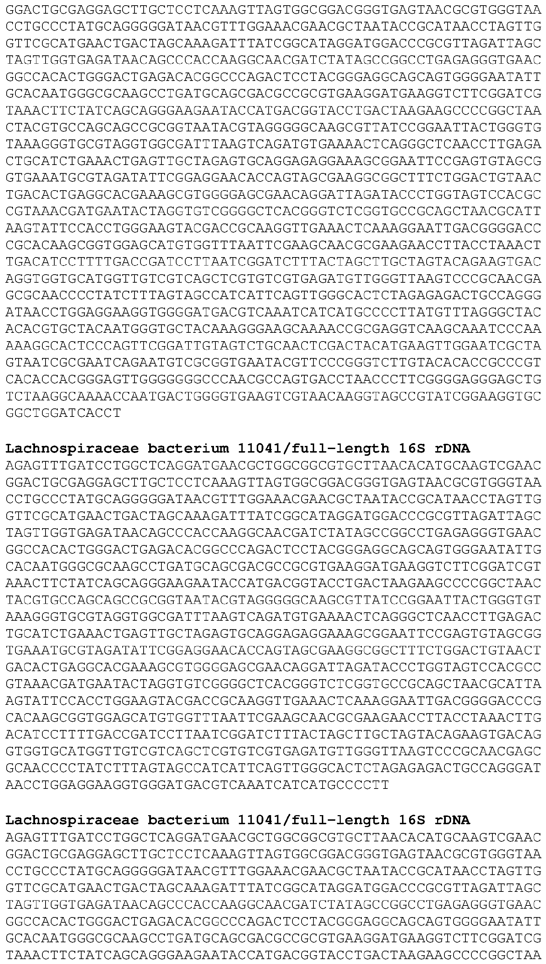

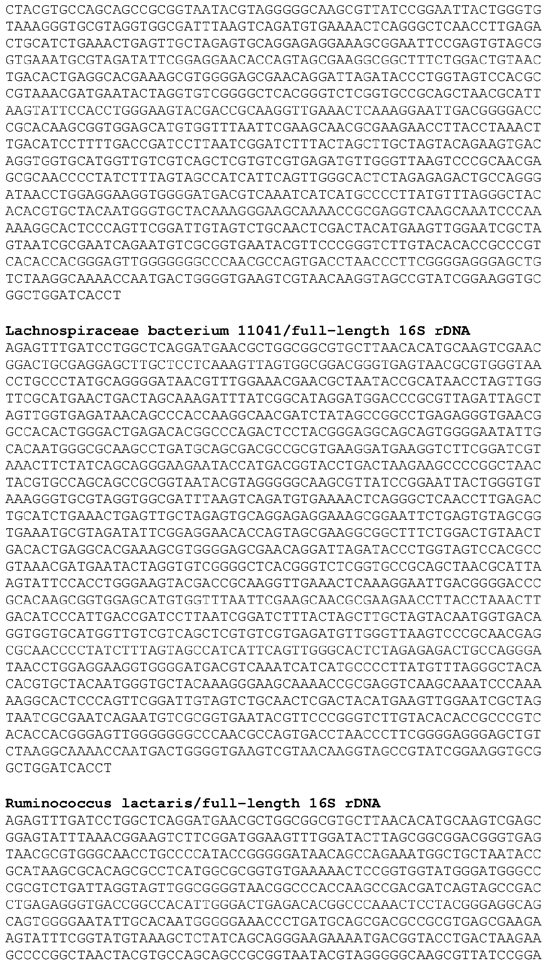

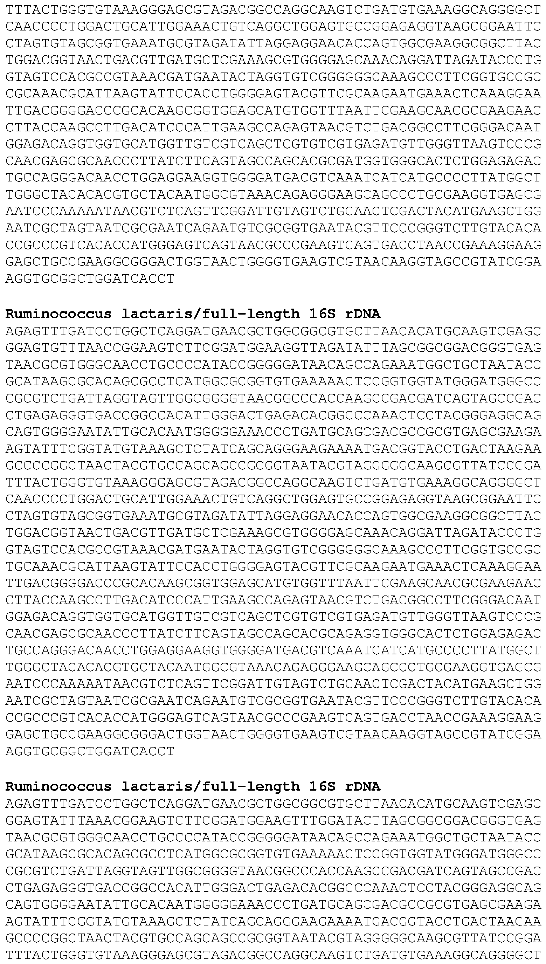

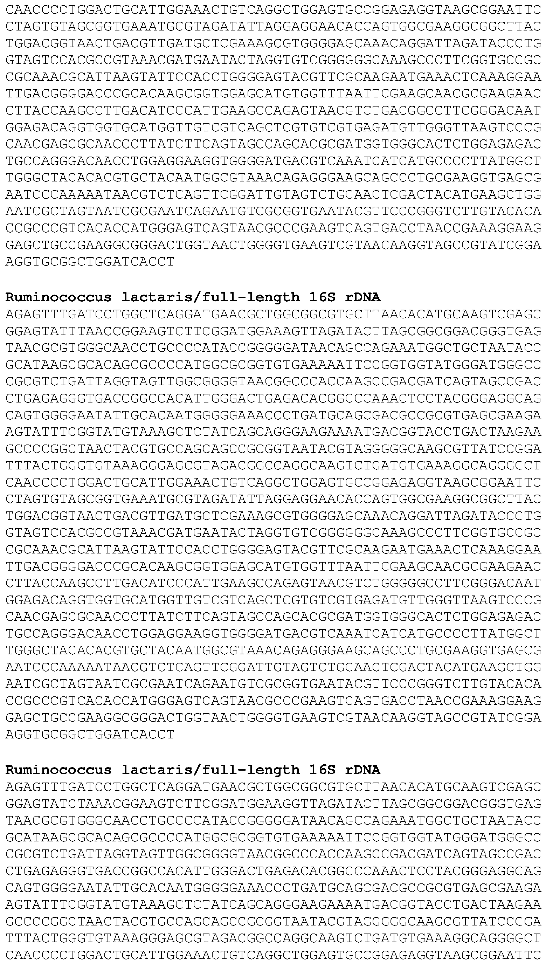

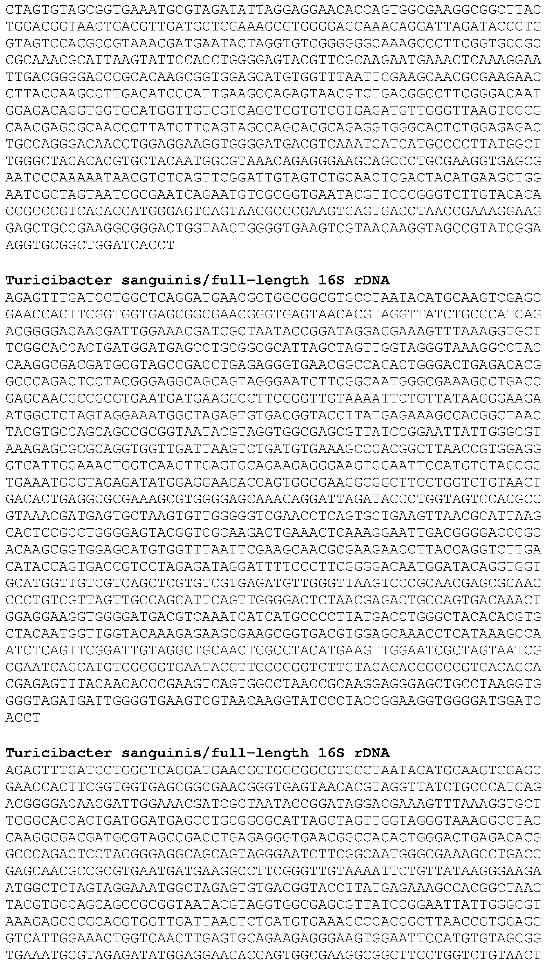

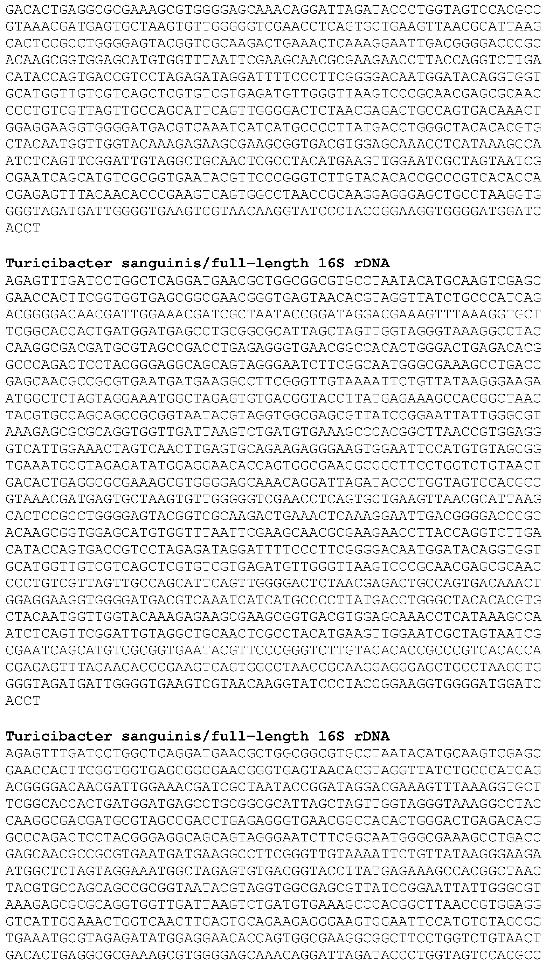

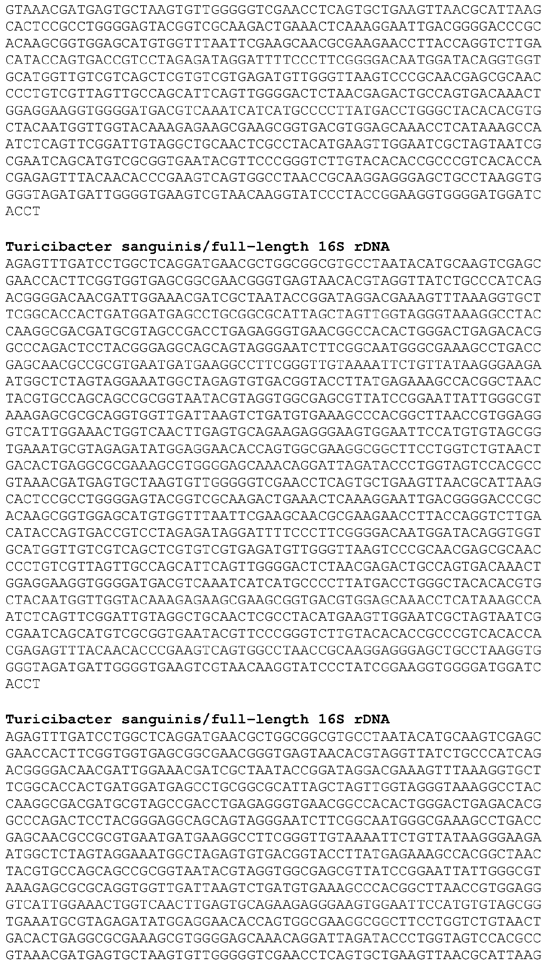

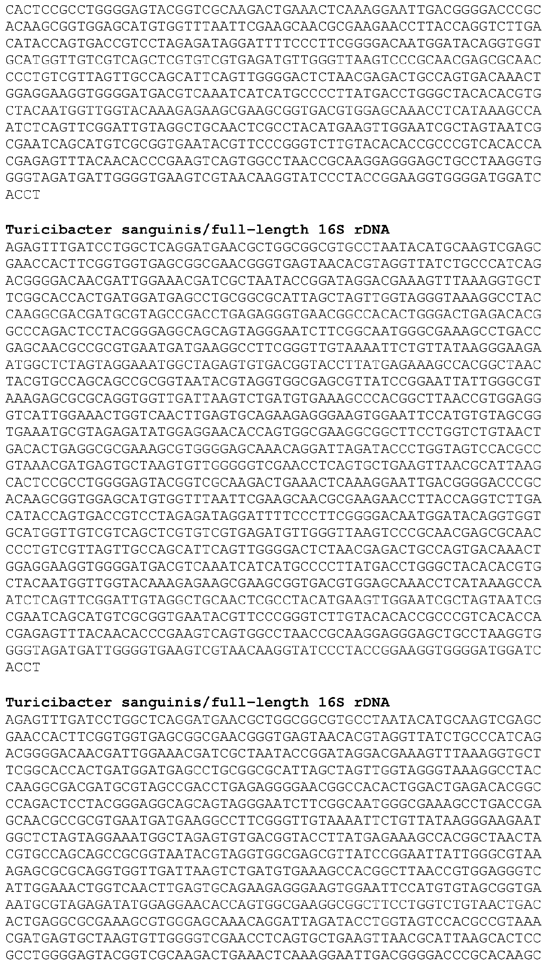

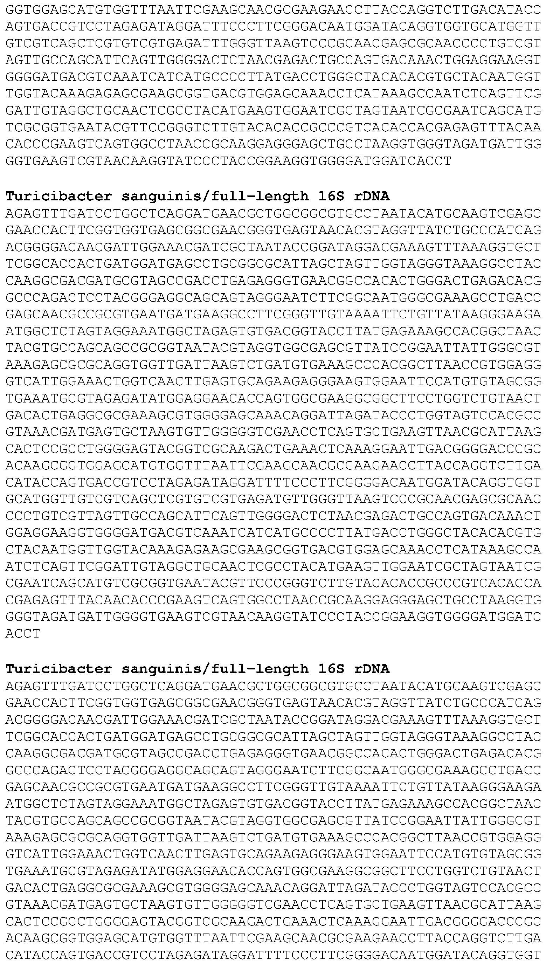

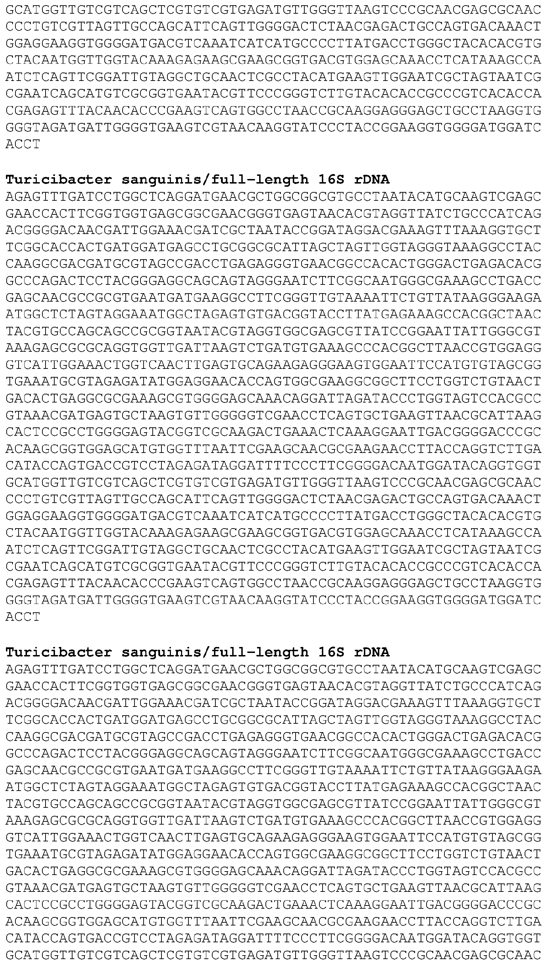

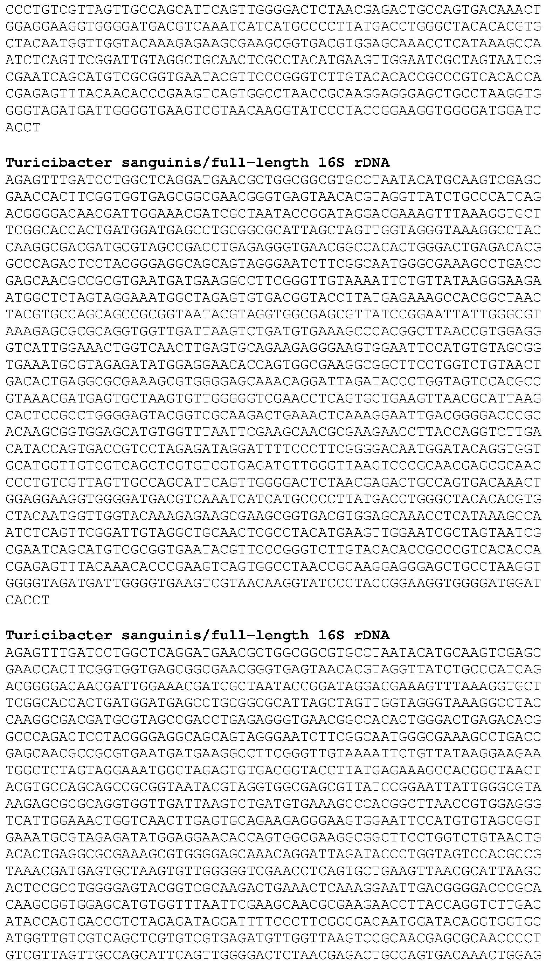

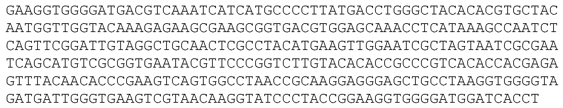

Accordingly, in a first aspect, the invention provides compositions including viable populations of bacteria, the viable populations containing the bacterial species of DBC 1, DBC 2, DBC 3, DBC 4, DBC 5, DBC 6, DBC 7, DBC 8, or DBC 9 of Table 3; or DBC S1, DBC S2, DBC S4, DBC S5, DBC S6, DBC S7, DBC S8, DBC S9, DBC S10, DBC S11, DBC S12, DBC S13, DBC S14, DBC S15, DBC S16, DBC S17, DBC S18, DBC S19, DBC S20, DBC S21, DBC S22, DBC S23, or DBC S24 of Table 4. Optionally, the 16S rDNA of one or more (e.g., each) bacterial species in the DBC has at least 97% (e.g., at least 98%, at least 99%, or 100%) identity to at least one 16S rDNA sequence in Figure 1.

In certain embodiments, the compositions consist of a viable population of bacterial species of DBC 1, DBC 2, DBC 3, DBC 4, DBC 5, DBC 6, DBC 7, DBC 8, DBC 9, DBC S1, DBC S2, DBC S4, DBC S5, DBC S6, DBC S7, DBC S8, DBC S9, DBC S10, DBC S11, DBC S12, DBC S13, DBC S14, DBC S15, DBC S16, DBC S17, DBC S18, DBC S19, DBC S20, DBC S21, DBC S22, DBC S23, or DBC S24, as defined herein. Optionally, the 16S rDNA of one or more (e.g., each) bacterial species in the DBC has at least 97% (e.g., at least 98%, at least 99%, or 100%) identity to at least one 16S rDNA sequence in Figure 1.

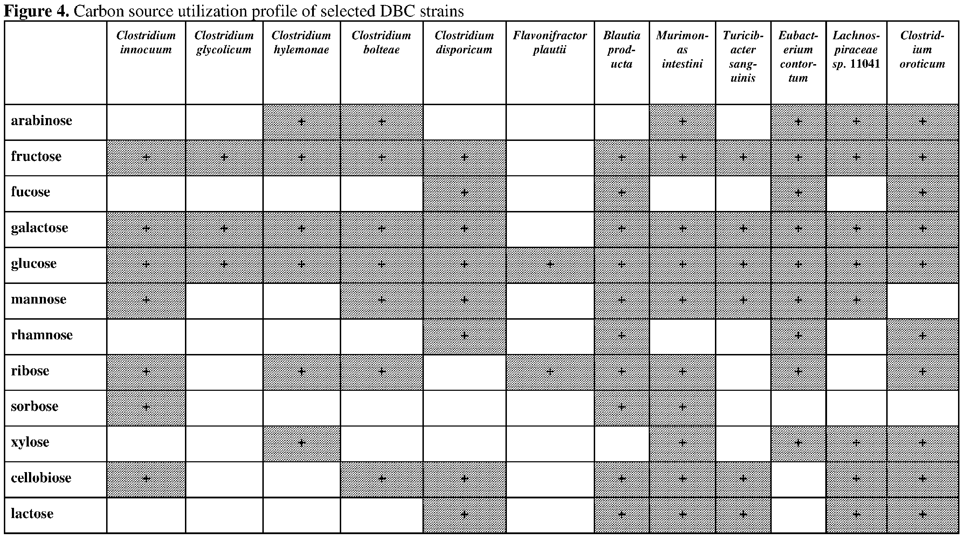

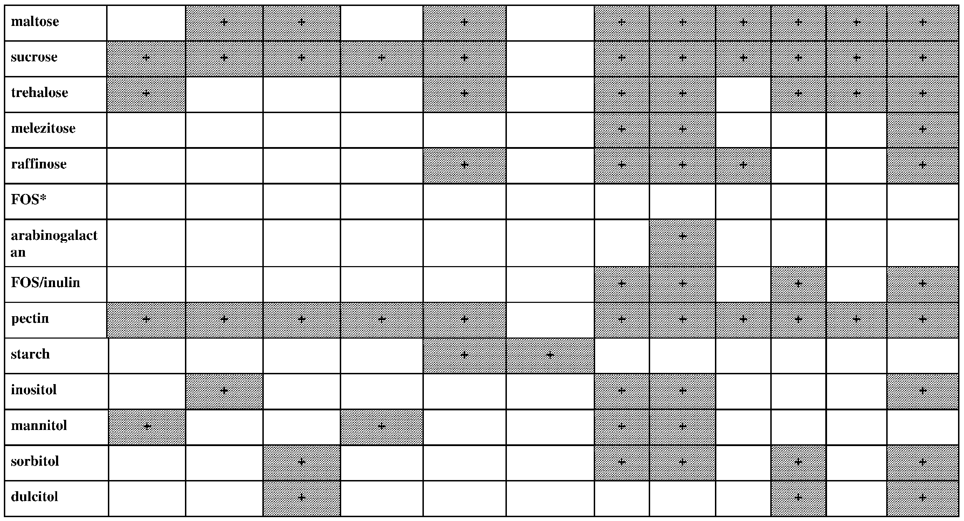

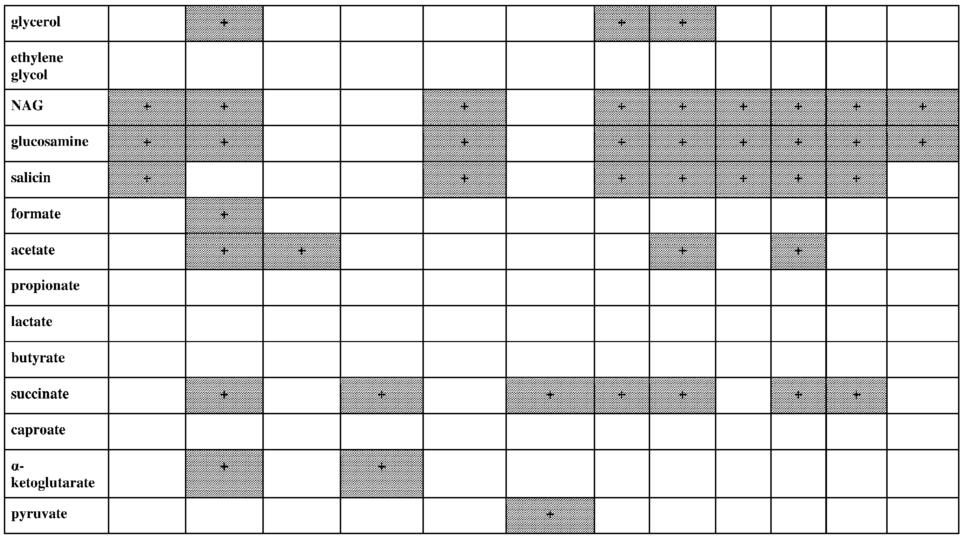

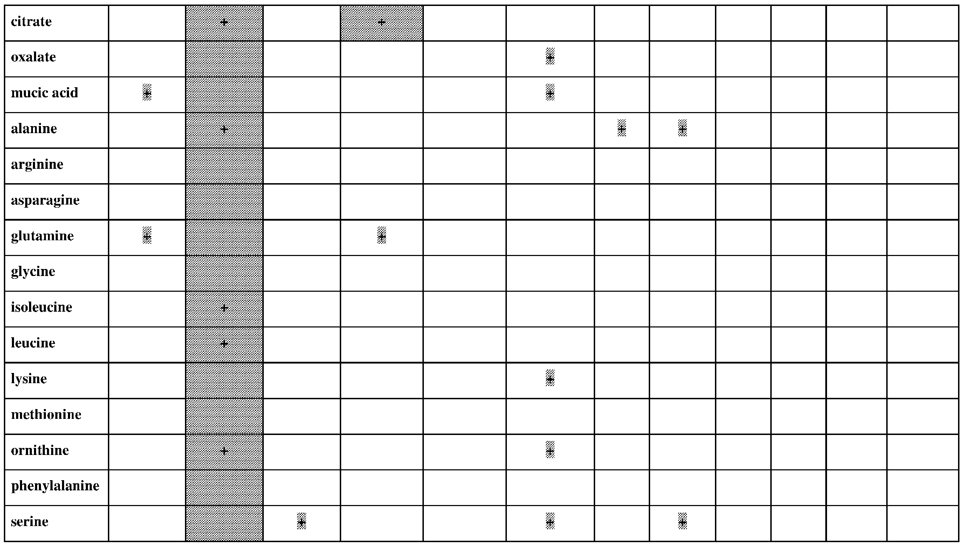

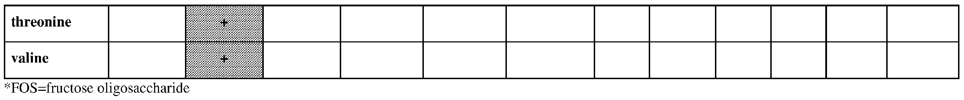

In some embodiments, the populations of bacterial species are able to utilize at least 90% (e.g., at least 95%, at least 98%, or 100%) of the carbon sources listed in Figure 4.

In another aspect, the invention provides compositions including populations of at least five (e.g., at least 5, 6, 7, 8, 9, 10, 11, 12, 13, 14, 15, 16, 17, 18, 19, or 20) species of viable bacteria, wherein at least one (e.g., at least 1, 2, 3, 4, 5, 6, 7, 8, 9, 10, 11, 12, 13, 14, 15, 16, 17, 18, 19, or 20) of the species of the population has one or more (e.g., 1, 2, 3, or 4) features selected from the group consisting of: (i) utilization of one or more carbon sources utilized by a pathogenic microorganism, (ii) production of an inhibitor of histone deacetylase (HDAC), (iii) production of indole or other tryptophan metabolites, and (iv) production of an enzyme that functions in bile acid regulation.

In various examples, the pathogenic microorganism is *C*. *difficile* and the population includes one or more (e.g., at least 1, 2, 3, 4, 5, 6, 7, 8, 9, 10, 11, 12, 13, 14, 15, 16, 17, 18, 19, or 20) species of viable bacteria that can utilize at least five (e.g., 5, 6, 7, 8, 9, 10, 11, 12, 13, 14, 15, 16, 17, 18, 19, or 20) different *C*. *difficile* carbon sources. For example, the *C. difficile* carbon sources can include or be selected from the group consisting of: taurocholate, glycocholate, glycochenodeoxycholate, taurochenodeoxycholate, cholate, chenodeoxycholate, and deoxycholate.

In further examples, the populations include one or more (e.g., at least 1, 2, 3, 4, 5, 6, 7, 8, 9, 10, 11, 12, 13, 14, 15, 16, 17, 18, 19, or 20) species of viable bacteria that produce an HDAC inhibitor, for example, an HDAC inhibitor selected from the group consisting of: butyrate, isovalerate, isobutyrate, propionate, and 2-methyl-butyrate.

In additional examples, populations include one or more (e.g., at least 1, 2, 3, 4, 5, 6, 7, 8, 9, 10, 11, 12, 13, 14, 15, 16, 17, 18, 19, or 20) species that produce one or more (e.g., 1, 2, 3, 4, 5, 6, 7, 8, 9, or 10) enzyme that functions in bile acid regulation, e.g., selected from the group consisting of bile hydrolase (BSH), 3-α-hydroxysteroid dehydrogenase (3-α-HSDH), 7-α-hydroxysteroid dehydrogenase (7-α-HSDH), and 12-α-hydroxysteroid dehydrogenase (12-α-HSDH).

In a further aspect, the invention provides compositions including at least one species from each of clades 86, 90, 101, 139, 195, 197, 206, 233, 238, 241, 244, and 290, and optionally a species from claim 202. In various examples, the compositions of the invention includes 5, 6, 7, 8, 9, 10, 11, or 12 species of bacteria. Furthermore, in various examples, at least 75% (e.g., at least 80%, 85%, 90%, or 95%) of the bacteria of the compositions are in the form of spores.

In another aspect, the invention provides formulations including a composition as described herein and a pharmaceutically acceptable excipient (e.g., a pharmaceutically acceptable excipient including glycerol, polyethylene glycol, or cocoa butter). The formulations can optionally be in a capsule or tablet for, e.g., oral delivery.

The invention further provides the use of a pharmaceutically effective amount of a composition as described herein preventing (e.g., decreasing the likelihood) or treating a dysbiosis in a subject at risk of or diagnosed with a dysbiosis. In various examples, the total concentration of bacteria in the composition is 10e1 to 10e9. Furthermore, the bacteria of the composition can optionally be provided in 1, 2, 3, 4, or 5 capsules.

In addition, the invention provides the use of a therapeutically effective amount of a composition as described herein for treating a *C. difficile* infection, or preventing (e.g., decreasing the likelihood of) a *C. difficile* infection, or preventing (e.g., decreasing the likelihood of) recurrence of a *C. difficile* infection.

The invention also provides methods of preventing or treating a dysbiosis in a subject at risk of or diagnosed with a dysbiosis, involving administering to the subject a pharmaceutically effective amount of a composition or formulation as described herein. In various examples, the total concentration of bacteria in the composition is 10e1 to 10e9. Furthermore, the bacterial of the composition can optionally be provided in 1, 2, 3, 4, or 5 capsules.

Also included in the invention are methods of treating a *C. difficile* infection, or preventing a *C. difficile* infection, or preventing recurrence of a *C. difficile* infection, the methods including administering a therapeutically effective amount of a composition or formulation as described herein.

In some embodiments of the methods, the compositions are useful for preventing recurrence of an infection, e.g., a *C. difficile* infection. In some embodiments, the composition is useful for decreasing gastrointestinal carriage of a pathogen, e.g. *C. difficile* carriage or vancomycin-resistant *Enterococcus* carriage.

### Definitions

A "therapeutically effective amount" of a designed bacterial composition (DBC) described herein can vary according to factors such as the disease state, age, sex, and weight of the individual, and the ability of the DBC to elicit a desired response in the individual, e.g., amelioration of at least one sign or symptom of a disorder (and optionally, the effect of any additional agents being administered). In some embodiments, a therapeutically effective amount of a DBC can prevent or reduce the risk of at least one sign or symptom of a disorder. For example, in some embodiments, a DBC can reduce the risk of recurrence of an infection associated with a dysbiosis. Dysbiosis can include the loss of function that a healthy microbiome confers to a host, including without limitation colonization resistance, protection from infection, regulation of immune homeostasis, metabolic functions, synthesis of essential metabolites and vitamins, modulation of gut motility or neurologic function, or any number of other properties that are now associated with a healthy microbiome and maintenance of intestinal barrier. A therapeutically effective amount is also one in which any toxic or detrimental effects of the composition are outweighed by the therapeutically beneficial effects. A composition as described herein is generally administered in a therapeutically effective amount.

"Carriage" means the condition of harboring a microorganism in or on the body of a subject, for example, carriage of a pathogen such as *C. difficile.*

The entire disclosure of each patent document and scientific article referred to herein, and those patent documents and scientific articles cited thereby, is expressly incorporated by reference herein for all purposes.

Additional features and advantages of the invention are more particularly described below.

### BRIEF DESCRIPTION OF THE DRAWINGS

Figure 1 is an exemplary set of full-length 16S rDNA sequences associated with bacteria useful in a DBC (SEQ ID NOs: 1-124).
Figure 2 is a drawing depicting an experimental design using a murine model of *C. difficile* infection resulting from antibiotic-induced dysbiosis.
Figure 3 is a bar graph depicting the results of experiments testing designed bacterial compositions (DBCs) in a murine model of a dysbiosis. Data depict minimum body weight per experimental group of mice compared to pre-infection baseline.
Figure 4 is a table showing carbon source utilization profiles of selected DBC species, based on OD₆₀₀ in medium with indicated carbon source, normalized for OD₆₀₀ in base medium without a carbon source. An empty cell indicates a negative result. Full cells indicate utilization of the listed carbon sources. Of the 59 carbon sources tested, only 29 used by *C. difficile* are presented.
Figure 5 is a table showing bile acid enzymatic activities of selected DBC species.
Figure 6 is a bar graph showing HDAC assay results for pure culture supernatants grown in PYG.
Figure 7 is a bar graph showing a comparison of HDAC inhibition activity and butyrate concentrations by GC.

### DETAILED DESCRIPTION

Reportedly, a healthy gastrointestinal (GI) microbiome in a mammal has between about 300 and 1000 different bacterial species. Surprisingly, Applicants have discovered that designed bacterial compositions, referred to herein as "DBCs," which contain a limited number of defined bacterial species, some of which are reportedly present in human microbiomes in very low relative amounts, are able to provide recovery or amelioration from insult to the GI tract of an animal, e.g., recovery or amelioration of *Clostridium difficile* infection (CDI). In some embodiments, such compositions can inhibit the recurrence of an infection associated with a dysbiosis, e.g., by reducing or eliminating carriage of a pathogenic organism induced by dysbiosis prior to infection.

Furthermore, Applicants have discovered new species present in extremely low levels in a healthy human GI microbiome, for example, detected in feces from a healthy human at less than 2%, e.g., less than 1%, less than 0.5%, less than 0.1%, less than 0.01%, less than 0.001%, less than 0.0001%, or less than 0.00001% of total bacteria. In particular examples, the new species may be present in an amount less than 1e9 CFU/gram of stool, 1e6 CFU/gram of stool, less than 1e5 CFU/gram of stool, less than 1e4 CFU/gram of stool, or less than 1e3 CFU/gram of stool. Despite their extremely low levels in human microbiomes, surprisingly, these species are able to contribute to the therapeutic or prophylactic utility of DBCs, e.g., a therapeutically effective DBC. DBCs provide an improvement over methods of treating a dysbiosis that use, e.g., preparations directly derived from human feces. For example, because humans have variable GI microbiomes, there can be problems with consistency in the numbers or proportions of organisms present in preparations from human feces. Furthermore, such preparations have a risk of infecting the patient receiving such treatments with a pathogen. Applicants' DBCs address the issue of patient diversity by the inclusion of phylogenetically diverse organisms in the therapeutic compositions that increase the likelihood of providing the functional characteristics necessary to promote colonization resistance across a broad range of patients.

DBCs described herein provide solutions to these problems with treating a dysbiosis as well as other issues with treating a dysbiosis-associated disorder.

### Compositions

Compositions comprise at least 2, 3, 4, 5, 6, 7, 8, 9, 10, 11, 12, 13, 14, 15, 16, 18, 20, 25, or 30 types of bacteria. A bacterial type can be a family, genus, clade, species, or strain. In one example, a composition comprises at least one species from each of clades 86, 90, 101, 139, 195, 197, 206, 233, 238, 241, 244, and 290, and optionally, clade 202, examples of which are provided in Table 2, *infra.* In some embodiments, the composition consists of at least one species from each of clades 86, 90, 101, 139, 195, 197, 202, 206, 233, 238, 241, 244, and 290.

In some embodiments, a composition comprises at least one species of bacterium from each of the families Erysipelatrichaceae, Lachnospiraceae, Peptostreptococaceae, Clostridiaceae, and the genus Flavonifractor.

In some embodiments, the species in a composition are selected from those identified in Table 1 and/or Table 2. Clades are bacterial species that are evolutionarily related and therefore have a high likelihood of sharing functional features. Clades are defined based on the topology of a phylogenetic tree that is constructed from full-length 16S sequences using maximum likelihood methods familiar to individuals with ordinary skill in the art of phylogenetics. Clades are constructed to ensure that all OTUs in a given clade are: (i) within a specified number of bootstrap supported nodes from one another, and (ii) within 5% genetic similarity. OTUs that are within the same clade can be distinguished as genetically and phylogenetically distinct from OTUs in a different clade based on 16S-V4 sequence data, while OTUs falling within the same clade are closely related. OTUs falling within the same clade are evolutionarily closely related. Members of the same clade, due to their evolutionary relatedness, play similar functional roles in a microbial ecology such as that found in the human gut. Compositions substituting one species with another from the same clade are likely to have conserved ecological function and therefore are useful in the present invention. In some embodiments, the composition comprises one, two, or three species from each clade in Table 1 or Table 2.

One example of a composition is a composition that includes 1, 2, 3, 4, 5, 6, 7, 8, 9, 10, 11, 12, 13, 14, 15, 16, 17, 18, 19, or 20 species of bacteria selected from Table 1 and/or Table 2. In some embodiments, a composition consists of 1, 2, 3, 4, 5, 6, 7, 8, 9, 10, 11, 12, 13, 14, or 15 species of bacteria selected from Table 1 and/or Table 2. In some embodiments, a bacterial species is identified by homology to 16S rDNA. An exemplary list of such 16S rDNA sequences is provided in Figure 1.

**Table 1**

| **Clade** | **Example Species [OTU] (Alternate name)** | **NCBI Genus** | **NCBI Family** | **Example of public database accession no. for Example Species** |
|---|---|---|---|---|
| 197 | Blautia producta (Blautia coccoides) | Blautia | Lachnospiraceae | producta: ATCC 27340 |
| | | | | coccoides: ATCC 29236 |
| 233 | Clostridium bolteae (Clostridium clostridioforme) | Lachnoclostridium | Lachnoclostridium | bolteae: ATCC BAA-613 |
| | | | | clostridioforme: ATCC 25537 |
| 244 | Clostridium butyricum | Terrisporobacter | Peptostreptococcaceae | ATCC 19398 |
| 244 | Clostridium disporicum (Clostridium celatum) | Clostridium | Clostridiaceae | ATCC 43838 |
| 90 | Clostridium hylemonae | Clostridium | Clostridiaceae | hylemonae: DSM 15053 |
| 139 | Clostridium innocuum | Erysipelatoclostridium | Erysipelotrichaceae | ATCC 14501 |
| 195 | Clostridium glycolicum | Terrisporobacter | Peptostreptococcaceae | glycolicum: ATCC 14880 |
| | (Clostridium mayombei) | | | mayombei: ATCC 51428 |
| 101 | Flavonifractor plautii (Clostridium orbiscindens; Eubacterium plautii) | | Unclassified Clostridiales | ATCC 29863 |
| 86 | Clostridium oroticum | Lachnoclostridium | Lachnospiraceae | ATCC 13619 |
| 86 | Eubacterium contortum | - | Lachnospiraceae | ATCC 25540 |
| 233 | Clostridium symbiosum | Lachnoclostridium | Lachnospiraceae | ATCC 14940 |
| 241 | Lachnospiraceae bacterium 11041 | - | Lachnospiraceae | Previously unknown: See Figure 1 for 16S rDNA sequence; |
| | | | | ATCC PTA-123576* |
| 290 | Turicibacter sanguinis | Turicibacter | Erysipelotrichaceae | DSM 14220 |
| 206 | Eubacterium sp WAL 14571 | Eubacterium | Eubacteriaceae | See Figure 1 for 16S rDNA sequences; |
| | | | | ATCC PTA-123577* (Clost 10316) |
| 90 | Lachnospiraceae bacterium 5_1_57FAA | | Lachnospiraceae | See Figure 1 for 16S rDNA sequences |
| 202/23 8 | Lachnospiraceae bacterium 10972 | | Lachnospiraceae | Previously unknown: See Figure 1 for 16S rDNA sequence |
| 238 | Murimonas intestini (Lachnospiraceae bacterium A4) | | Lachnospiraceae | Previously unknown: See Figure 1 for 16S rDNA sequence; DSM 26524 |

| | | | | |
|---|---|---|---|---|
| ^{∗} strains PTA-123576 and PTA-123577 were deposited with the American Type Culture Collection (ATCC), 10801 University Boulevard, Manassas, VA 20110 USA, on October 26, 2016, and tested for viability on November 8, 2016, as per an ATCC letter dated November 11, 2016 | | | | |

**Table 2: Additional bacterial species by clade**

| **Clade** | **Species** |
|---|---|
| 86 | Clostridium oroticum |
| | Clostridium_sp_D5 |
| | Eubacterium contortum |
| | Ruminococcus_lactaris |
| | Clostridium_glycyrrhizinilyticum |
| | Eubacterium_contortum |
| | Eubacterium_fissicatena |
| | Lachno spiraceae_bacterium_1 _1_57FAA |
| | Lachnospiraceae_bacterium_1_4_56FAA |
| | Lachnospiraceae_bacterium_8_1_57FAA |
| | Ruminococcus_torques |
| 90 | Clostridium hylemonae (Clostridium leptum) |
| | Lachnospiraceae_bacterium_5_1_57FAA |
| | Clostridium_scindens |
| | Dorea_formicigenerans |
| | Dorea_longicatena |
| | Lachnospiraceae_bacterium_2_1_46FAA |
| | Lachnospiraceae_bacterium_4_1_37FAA |
| | Lachnospiraceae_bacterium_9_1_43BFAA |
| 101 | Clostridium orbiscindens (Flavonifractor plautii) |
| | Clostridium_sp_NML_04A032 |
| | Clostridium_viride |
| | Flavonifractor_plautii |
| | Oscillibacter_sp_G2 |
| | Oscillibacter_valericigenes |
| | Oscillospira_guilliermondii |
| | Papillibacter_cinnamivorans |
| | Pseudoflavonifractor_capillosus |
| | Ruminococcaceae_bacterium_D16 |
| | Sporobacter_termitidis |
| 139 | Clostridium innocuum |
| | Clostridiaceae_bacterium_JC13 |
| | Clostridium_sp_HGF2 |
| | Clostridium_sp_MLG055 |
| | Erysipelotrichaceae_bacterium_3_1_53 |
| | Erysipelotrichaceae_bacterium_5_2_54FAA |
| | Eubacterium_biforme |
| | Eubacterium_cylindroides |
| | Eubacterium_dolichum |
| | Eubacterium_sp_3_1_31 |
| | Eubacterium_tortuosum |
| 195 | Clostridium mayombei (Clostridium glycolicum) |
| | Clostridium_bartlettii |
| | Clostridium_bifermentans |
| | Clostridium difficile |
| | Clostridium_ghonii |
| | Clostridium_glycolicum |
| | Clostridium_hiranonis |
| | Clostridium_irregulare |
| | Clostridium_sordellii |
| | Clostridium_sp_MT4_E |
| | Eubacterium_tenue |
| | Peptostreptococcus_anaerobius |
| | Peptostreptococcus_stomatis |
| 197 | Blautia_producta |
| | Blautia coccoides |
| | Blautia_glucerasea |
| | Blautia_glucerasei |
| | Blautia_hansenii |
| | Blautia_hydrogenotrophica |
| | Blautia_luti |
| | Blautia_schinkii |
| | Blautia_sp_M25 |
| | Blautia_stercoris |
| | Blautia_wexlerae |
| | Clostridium_coccoides |
| | Lachnospiraceae_bacterium_6_1_63FAA |
| | Ruminococcus_hansenii |
| | Ruminococcus_obeum |
| | Ruminococcus_sp_5_1_39BFAA |
| | Ruminococcus_sp_K_1 |
| 206 | Eubacterium sp WAL 14571 |
| | Alkaliphilus_metalliredigenes |
| | Alkaliphilus_oremlandii |
| | Caminicella_sporogenes |
| | Clostridiales_bacterium_9400853 |
| | Eubacterium_brachy |
| | Eubacterium_infirmum |
| | Eubacterium_nodatum |
| | Eubacterium_saphenum |
| | Eubacterium_sp_oral_clone_JH012 |
| | Eubacterium_sp_oral_clone_JS001 |
| | Mogibacterium_diversum |
| | Mogibacterium_neglectum |
| | Mogibacterium_pumilum |
| | Mogibacterium_timidum |
| 233 | Clostridium bolteae (Clostridium clostridioforme) |
| | Clostridium symbiosum |
| | Acetivibrio_ethanolgignens |
| | Anaerosporobacter_mobilis |
| | Anaerostipes_caccae |
| | Anaerostipes_sp_3_2_56FAA |
| | Clostridiales_sp_1_7 _47 |
| | Clostridiales_sp_SM4_1 |
| | Clostridiales_sp_SSC_2 |
| | Clostridium_aerotolerans |
| | Clostridium_aldenense |
| | Clostridium_algidixylanolyticum |
| | Clostridium_aminovalericum |
| | Clostridium_amygdalinum |
| | Clostridium_asparagiforme |
| | Clostridium_celerecrescens |
| | Clostridium_citroniae |
| | Clostridium_clostridiiformes |
| | Clostridium_clostridioforme |
| | Clostridium_hathewayi |
| | Clostridium_indolis |
| | Clostridium_lavalense |
| | Clostridium_phytofermentans |
| | Clostridium_saccharolyticum |
| | Clostridium_sp_M62_1 |
| | Clostridium_sp_SS2_1 |
| | Clostridium_sphenoides |
| | Clostridium_xylanolyticum |
| | Eubacterium_hadrum |
| | Eubacterium_ventriosum |
| | Eubacterium_xylanophilum |
| | Lachnospiraceae_bacterium_5_1_63FAA |
| 238 | Lachnospiraceae_bacterium_A4 (Murimonas intestini) |
| | Bryantella_formatexigens |
| | Lachnospiraceae_bacterium_3_1_57FAA |
| | Lachnospiraceae_bacteriumDJF_VP30 |
| | Marvinbryantia_formatexigens |
| 241 | Lachnospiraceae_bacterium_oral_taxon_F15_UY03 8 |
| | Coprococcus_catus |
| 244 | Clostridium butyricum |
| | Clostridium disporicum (Clostridium celatum) |
| | Clostridicum_butyricum |
| | Clostridium_disporicum |
| | Clostridium_acetobutylicum |
| | Clostridium_argentinense |
| | Clostridium_baratii |
| | Clostridium_beijerinckii |
| | Clostridium_botulinum |
| | Clostridium_carboxidivorans |
| | Clostridium_carnis |
| | Clostridium_celatum |
| | Clostridium_chauvoei |
| | Clostridium_cochlearium |
| | Clostridium_colicanis |
| | Clostridium_estertheticum |
| | Clostridium_favososporum |
| | Clostridium_felsineum |
| | Clostridium_gasigenes |
| | Clostridium_histolyticum |
| | Clostridium_isatidis |
| | Clostridium_kluyveri |
| | Clostridium_limosum |
| | Clostridium_magnum |
| | Clostridium_malenominatum |
| | Clostridium_paraputrificum |
| | Clostridium_quinii |
| | Clostridium_sardiniense |
| | Clostridium_sartagoforme |
| | Clostridium_septicum |
| | Clostridium_sp_7_2_43FAA |
| | Clostridium_sp_HPB_46 |
| | Clostridium_sp_JC122 |
| | Clostridium_sp_NMBHI_1 |
| | Clostridium_sporogenes |
| | Clostridium_subterminale |
| | Clostridium_sulfidigenes |
| | Clostridium_tertium |
| | Clostridium_tetani |
| | Clostridium_tyrobutyricum |
| | Eubacterium_budayi |
| | Eubacterium_moniliforme |
| | Eubacterium_multiforme |
| | Eubacterium_nitritogenes |
| 290 | Turicibacter sanguinis |
| | Desulfitobacterium_frappieri |
| | Desulfitobacterium_hafniense |
| | Desulfotomaculum_nigrificans |
| | Heliobacterium_modesticaldum |
| | Peptococcus_niger |
| | Peptococcus_sp_oral_taxon_167 |

Non-limiting examples of compositions using bacterial species listed in Table 1 are provided in Table 3.

**Table 3: Examples of Designed Compositions (DBCs)**

| **Example Species** | **DBC 1** | **DBC 2** | **DBC 3** | **DBC 4** | **DBC 5** | **DBC 6** | **DBC 7** | **DBC 8** | **DBC 9** |
|---|---|---|---|---|---|---|---|---|---|
| Blautia producta | X | X | X | X | X | X | X | X | X |
| Clostridium bolteae | X | | X | X | | X | X | X | |
| Clostridium butyricum | X | X | | X | | | X | | |
| Clostridium disporicum | X | X | X | X | | X | X | X | |
| Clostridium hylemonae | X | X | X | X | X | X | X | X | X |
| Clostridium innocuum | X | X | X | | | X | X | X | |
| Clostridium glycolicum (formerly referenced as Clostridium mayombei) | X | X | X | X | X | X | X | X | X |
| Flavonifractor plautii (formerly referenced as Clostridium orbiscindens) | X | X | X | X | X | X | X | X | X |
| Clostridium oroticum | | X | X | X | X | X | | X | X |
| Eubacterium contortum | | X | X | X | X | X | | X | X |
| Clostridium symbiosum | X | X | | X | | | X | | |
| Murimonas intestini (formerly referenced as Lachnospiraceae bacterium A4) | | | X | | X | X | | X | X |
| Lachnospiraceae bacterium 11041 | | X | X | | X | X | | X | X |
| Turicibacter sanguinis | | X | X | X | | X | | X | X |
| Eubacterium sp WAL 14571 | X | | X | | | | X | X | |
| Lachnospiraceae 5_1_57FAA | X | | X | | X | | | | |

Additional DBC compositions (DBC S 1 - DBC S24) are listed below, in Table 4. Table 4 shows the bacterial compositions of DBC S 1 to DBC S24 and shows features of DBC S 1 to DBC S24 under the conditions tested. Features are as follows: Cluster IV: none of the species are in Clostridial Cluster IV; No Cluster IV or XIVa: none of the species are in Clostridial Cluster IV or XIVa; High engrafting, High prevalence: in experiments with a complex composition of spores isolated from healthy human feces, these are examples of bacterial species that were high engrafters (species that were in the complex spore composition that were then later detected in subjects dosed with the spore composition) and species that were high prevalence (species commonly found in the HMP dataset); HDAC Activity: all species produce a short chain fatty acid (butyrate and/or propionate, and/or isobutyrate and isovalerate) capable of inhibiting histone deacetylase activity; No HDAC Activity: none of the species produce a short chain fatty acid (butyrate and/or propionate and/or isobutyrate and isovalerate) capable of inhibiting histone deacetylase activity; BSH Activity: all species have bile salt hydrolase activity; No BSH Activity: none of the species have bile salt hydrolase activity; 7α-HSDH Activity: all of the species have 7-α-hydroxysteroid dehydrogenase activity; No 7-α-HSDH Activity: none of the species have 7-α-hydroxysteroid dehydrogenase activity; 3-α or 12-α-HSDH Activity: all species have 3-α/12 α-hydroxysteroid dehydrogenase activity; Growth on the Amino Acids alanine, arginine, asparagine, glutamine, glycine, histidine, isoleucine, leucine, methionine, ornithine, phenylalanine, serine, and valine: all species can use these amino acids as a carbon source; No Growth on the Amino Acids alanine, arginine, asparagine, glutamine, glycine, histidine, isoleucine, leucine, methionine, ornithine, phenylalanine, serine, and valine: none of the species used these amino acids as a carbon source; Indole producing: all species can produce indole or an indole derivative; Non-indole producing: none of the species can produce indole; Growth on sugar alcohols: all species can use sugar alcohols as a carbon source; No growth on sugar alcohols: none of the species can use sugar alcohols as a carbon source; Growth on fucose: all species can use fucose as a carbon source; No growth on fucose: none of the species can use fucose as a carbon source; Growth on NAG: all species can use N-acetylglucosamine (NAG) as a carbon source; No growth on NAG: none of the species were predicted to be able to use NAG as a carbon source. The predictions were based on the results of that specific experiment/screen.

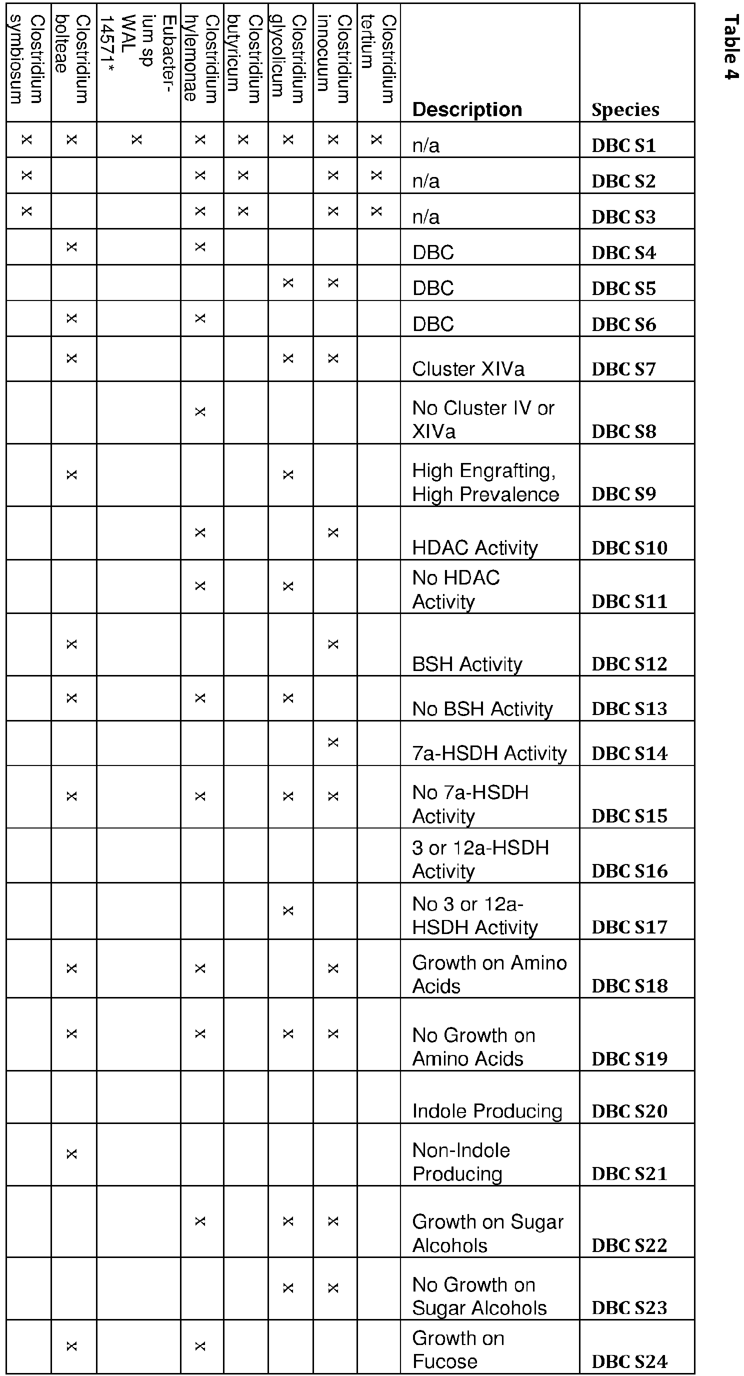

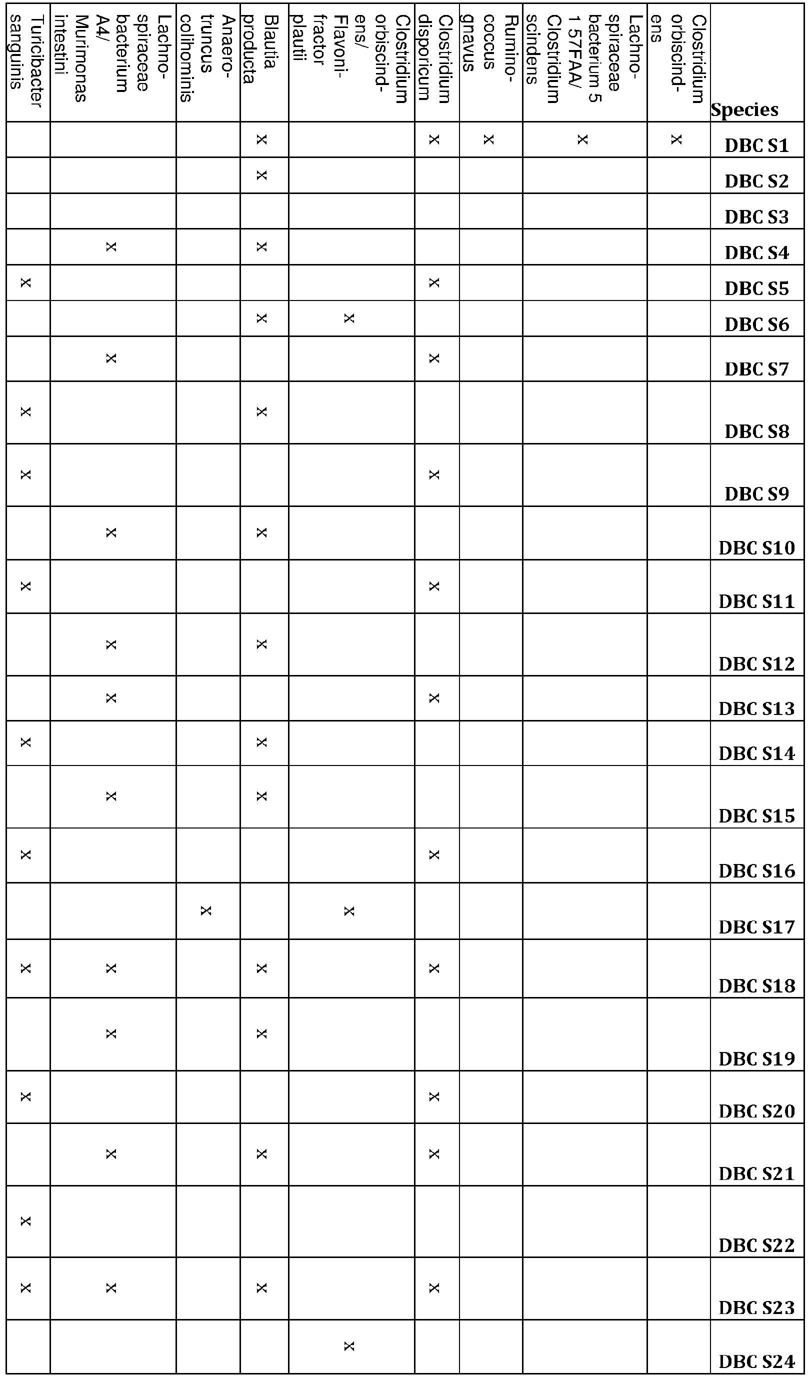

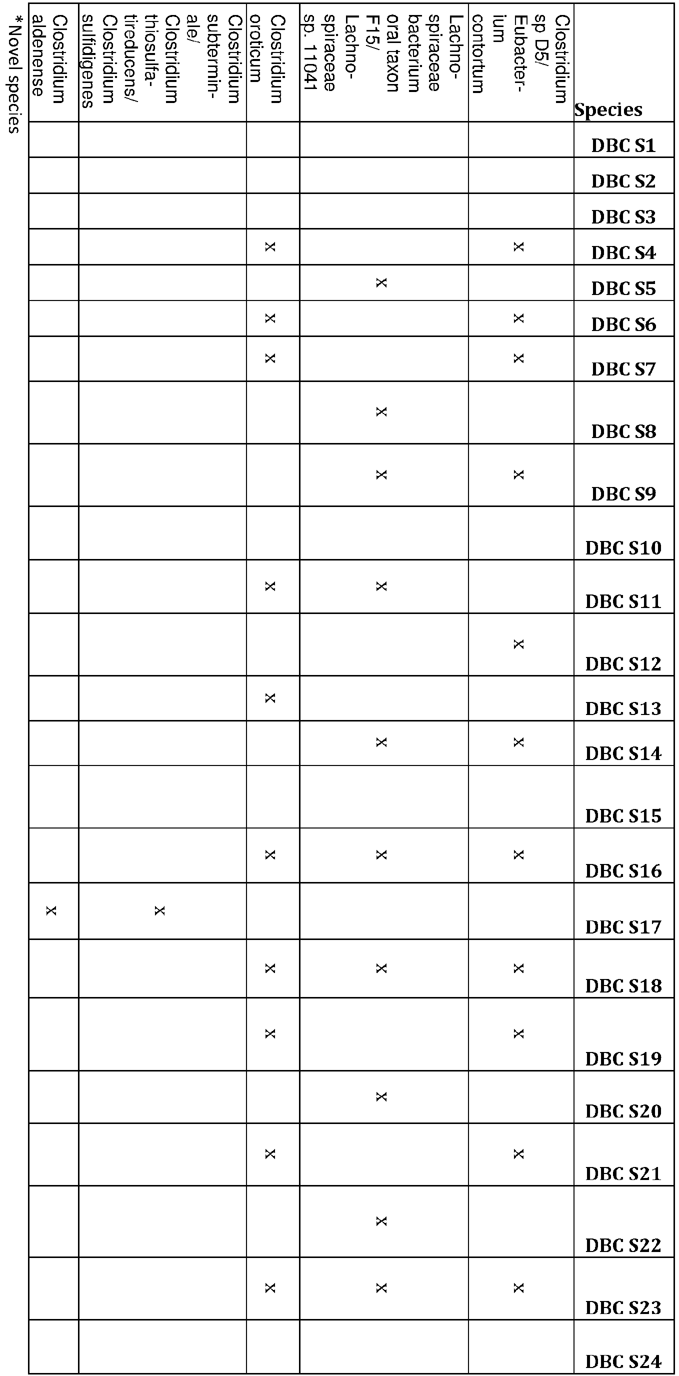

In some embodiments, all organisms in a DBC are obligate anaerobes. In some embodiments, the bacteria in a composition are species that can be cultured *in vitro* to form spores and such spores can be germinated *in vitro.* In some embodiments, the bacteria in a composition are spores. In some embodiments, the bacteria in a composition are in vegetative form. It is to be understood that a composition of bacterial spores or a composition of vegetative bacteria means that while the majority of bacteria are in the specified form (i.e., spore or vegetative), a small number may be in a different form, e.g., in the case of spores, some cells in a composition may be vegetative, while in the case of vegetative bacteria, some cells may be in the form of spores. For example, the composition may be at least 100%, at least 99%, at least 97%, at least 95%, at least 90%, at least 85%, at least 80 %, or at least 75% spores, or the composition may be at least 100%, at least 99%, at least 97%, at least 95%, at least 90%, at least 85%, at least 80 %, or at least 75% vegetative bacteria. In some embodiments, the individual species are present as a mixture of vegetative bacteria and spores in a ratio, for example, 74:26, 70:30, 60:40, 50:50, 40:60, 30:70, and 26:74. Typically the ratios are assessed using colony-forming units (cfu), although other methods known in the art can be used. The assessment of percent of bacteria in a vegetative or spore specific form may be referenced as of the date of preparing the composition in a dosage form or as of the date or administration of the dosage form.

In other embodiments, a DBC includes at least two organisms from Table 1 and one or more of the following: *Lachnospiraceae bacterium A4, Lachnospiraceae bacterium 5 1 57FAA,* and *Ruminococcus lactaris.*

Another example of compositions are compositions containing 2, 3, 4, 5, 6, 7, 8, 9, 10, 11, or 12 of the following species: *Clostridium glycolicum, Clostridium mayombei, Clostridium hylemonae, Clostridium bolteae, Clostridium disporicum, Clostridium innocuum, Flavonifractor plautii, Clostridium orbiscindens, Blautia producta, Turicibacter sanguinis, Eubacterium contortum, Murimonas intestini, Lachnospiraceae bacterium A4, Lachnospiraceae bacterium 11041,* and *Clostridium oroticum.*

Surprisingly, Applicants have found that is not necessary to provide organisms in proportions that reflect those of a healthy microbiome or that are otherwise found in nature; Applicants found that doses of a DBC containing approximately the same number of each species of bacteria (e.g., the same number of *in vitro* germinable spores) were effective, even though the composition does not contain organisms in naturally occurring proportions. Furthermore, Applicants are not aware of any individual human reported as having detectable levels of all organisms in any of the DBCs provided in Tables 3 and 4, i.e., Applicants believe that the compositions are non-naturally occurring.

Applicants also note that the total number of bacteria effective in a treatment is far below the total number of organisms in the gastrointestinal tract of a healthy human, i.e., it is not necessary to administer a complete healthy microbiome to achieve a therapeutic effect, not only in terms of the diversity of the species provided in a composition, but also in the total number of organisms provided.

It is to be understood that "consisting of" in these examples refers to the bacteria types that are present in a composition. A bacterial formulation may contain additional non-bacterial materials such as one or more excipients (including, for example, one or more capsules), an aqueous or non-aqueous medium (for example, glycerol, polyethylene glycol, cocoa butter, water, and/or buffer), as well as one or more prebiotics or small molecule drugs.

### Species used in compositions

In general, species used in a DBC are identified as within a selected clade based on their 16S rDNA sequence (e.g., full-length sequence, or one or more variable region sequences (V1-V9, e.g., V4 sequence, or V6 sequence)).

In some embodiments, a species useful in a DBC is a species having a full-length 16S rDNA with at least 95% sequence identity ("identity") to 16S rDNA of a reference species, e.g., a species identified in Table 1 or Table 2. In some embodiments, a species useful in a DBC is a species having a full-length 16S rDNA with at least 97% sequence identity ("identity") to 16S rDNA of a reference species, e.g., a species in Table 1 or Table 2. In some embodiments, a useful species has V4 region 16S rDNA having 95% identity to a V4 region of 16S rDNA of a reference species, e.g., a species identified in Table 1 or Table 2. In some embodiments, a useful species has V4 region 16S rDNA having 97% identity to a V4 region of 16S rDNA of a reference species, e.g., a species identified in Table 1 or Table 2. In some embodiments a useful species has a genomic sequence having at least 95% identity to the full length genomic DNA of a reference species, e.g., a species identified in Table 1 or Table 2. In some embodiments a useful species has a genomic sequence having at least 97% identity to the full-length genomic sequence of a reference species, e.g., a species identified in Table 1 or Table 2. In the event that a sequence is not provided herein, e.g., a V4 sequence, methods are well known in the art for identifying such sequences. Figure 1 provides non-limiting examples of full-length 16S rDNA sequences that can be used as reference sequences. In general, identity or percent identity with a reference species means identity or percent identity with at least one 16S rDNA sequence found in an organism.

In some cases, strains of bacterial species useful in an invention, e.g., species disclosed herein, can be obtained from a public biological resource center such as the ATCC (atcc.org), the DSMZ (dsmz.de), or the Riken BioResource Center (en.brc.riken.jp). 16s rDNA sequences useful for identifying species or other aspects of the invention can be obtained from public databases, e.g., the Human Microbiome Project (HMP) web site or GenBank.

Methods of determining sequence identity are known in the art and examples are provided *infra.*

### Species/naming information

Names and classification of bacteria are subject to changes that may not be reflected in the literature. For convenience, alternate names for some bacterial species are provided, herein, but are not intended to be a comprehensive set of alternative names. In some embodiments, species are identified by sequence identity of all or a portion of a 16S rDNA sequence, e.g., at least 90%, 93% 95%, 96%, 97%, 98%, 99%, or 100% identity.

### Determination of identity

Clades, operational taxonomic units (OTUs), species, and strains are, in some embodiments, identified by their 16S rDNA sequence. The relatedness of clades, OTUs, species, and strains can be determined by the percent identity between clades, OTUs, species, or strains. Percent identity between a reference and query sequence can be determined using methods known in the art. Non-limiting examples of methods for such determinations are provided below. As used herein, the relatedness between two nucleotide sequences is described by the parameter "identity."

In one embodiment, the degree of sequence identity between a query sequence and a reference sequence is determined by 1) aligning the two sequences by any suitable alignment program using the default scoring matrix and default gap penalty, 2) identifying the number of exact matches, where an exact match is where the alignment program has identified an identical nucleotide in the two aligned sequences on a given position in the alignment and 3) dividing the number of exact matches with the length of the reference sequence.

In another embodiment, the degree of sequence identity between a query sequence and a reference sequence is determined by 1) aligning the two sequences by any suitable alignment program using the default scoring matrix and default gap penalty, 2) identifying the number of exact matches, where an exact match is where the alignment program has identified an identical nucleotide in the two aligned sequences on a given position in the alignment and 3) dividing the number of exact matches with the length of the longest of the two sequences.

In another embodiment, the degree of sequence identity between the query sequence and the reference sequence is determined by 1) aligning the two sequences by any suitable alignment program using the default scoring matrix and default gap penalty, 2) identifying the number of exact matches, where an exact match is where the alignment program has identified an identical amino acid or nucleotide in the two aligned sequences on a given position in the alignment and 3) dividing the number of exact matches with the "alignment length," where the alignment length is the length of the entire alignment including gaps and overhanging parts of the sequences.

Sequence identity comparisons are, generally, with the aid of a sequence comparison program. These commercially or publicly available computer programs use complex comparison algorithms to align two or more sequences that best reflect the evolutionary events that might have led to the difference(s) between the two or more sequences. Therefore, these algorithms operate with a scoring system rewarding alignment of identical or similar amino acids and penalizing the insertion of gaps, gap extensions and alignment of non-similar amino acids. The scoring system of the comparison algorithms include:
i) assignment of a penalty score each time a gap is inserted (gap penalty score),
ii) assignment of a penalty score each time an existing gap is extended with an extra position (extension penalty score),
iii) assignment of high scores upon alignment of identical amino acids, and
iv) assignment of variable scores upon alignment of non-identical amino acids.

In general, the default values of the alignment program are used for sequence comparisons. Suitable computer programs useful for determining identity include, for example, BLAST (blast.ncbi.nlm.nih.gov).

In an embodiment of the present invention, the alignment program optimizes the alignment over the full-length of selected sequences, e.g., full-length, V4, or V6 16S rDNA sequence. For example, the global alignment program is based on the Needleman-Wunsch algorithm (Needleman and Wunsch, 1970, J Mol Biol 48: 443-53). Non-limiting examples of such programs are EMBOSS Needle and EMBOSS Stretcher programs, available at ebi.ac.uk/Tools/psa/.

In one embodiment, the sequences are aligned by a global alignment program and the sequence identity is calculated by identifying the number of exact matches identified by the program divided by the "alignment length," where the alignment length is the length of the entire alignment including gaps and overhanging parts of the sequences. In a further embodiment, the global alignment program uses the Needleman-Wunsch algorithm and the sequence identity is calculated by identifying the number of exact matches identified by the program divided by the "alignment length," where the alignment length is the length of the entire alignment including gaps and overhanging parts of the sequences.

In yet a further embodiment, the global alignment program is selected from the group consisting of EMBOSS Needle and EMBOSS stretcher and the sequence identity is calculated by identifying the number of exact matches identified by the program divided by the "alignment length," where the alignment length is the length of the entire alignment including gaps and overhanging parts of the sequences.

Once the software has produced an alignment, it is possible to calculate percent (%) similarity and percent sequence identity.

### Methods of testing a candidate DBC

### In vivo methods

Candidate DBCs can be tested using animal models of diseases associated with a dysbiosis, e.g., Hutton et al. (2014, FEMS Microbiol. Lett. 352:140-149), Best et al. (2012, Gut Microbes 3:145-167) and Chen et al. (2008, Gastroenterology 135:1984-92). The use of one such model is described in the Examples, *infra.* A candidate DBC that improves a sign or symptom in such a model is useful as a DBC for treating a dysbiosis, e.g., in a human.

### Features of compositions

As described herein, a DBC generally is tested for the ability, *in vivo* and/or *in vitro* to inhibit the growth of a pathogenic organism, the ability to kill a pathogenic organism, the ability to prevent or ameliorate the effects of a pathogenic organism, or other such suitable activity. In some embodiments, bacteria in a DBC are selected for features that they exhibit either in the composition, in a sub-composition, or as individual bacteria. Examples of such features include the abilities to inhibit histone deacetylase, metabolize bile acids, utilize nutrient sources (e.g., nutrient sources used by a targeted pathogenic organism), and metabolize tryptophan to produce indole and other tryptophan metabolites. Details of these features are set forth as follows and in the Examples, below.

### Histone deacetylase activity

Histone deacetylases (HDACS) are a family of enzymes that can remove acetyl residues from specific sites in the N-terminal end of histones, which are part of the DNA chromatin structure in eukaryotic cells (reviewed in Davie). The steady state of histone acetylation is a result of the balance of acetylation by histone acetyltransferase (HAT) enzymes and deacetylation by HDACs. When HDACs are inhibited but HATs activity continues, histones become hyperacetylated, thus disrupting high order chromatin structure and stimulating transcription by RNA polymerase III. The effect of HDAC inhibition in gene expression is not generalized, as only 2% of mammalian genes are affected by HDAC inhibition.

Some short chain fatty acids (SCFAs) produced by the intestinal human microbiome are HDAC inhibitors. Butyrate in particular has been identified as an HDAC inhibitor *in vitro* and *in vivo,* leading to the accumulation of hyperacetylated histones H3 and H4 (Candido et al. 1978 Cell 14:105-113; Boffa et al. 1978 J Biol Chem 253:3364-3366; Vidali et al. 1978 Proc Natl Acad Sci USA 75:2239-2243; Davie. 2003 J Nutrition 133:2485S-2493S). Other SCFAs, propionate, isobutyrate, isovalerate, valerate, lactate, and acetate also inhibit histone deacetylation although reportedly less effectively than butyrate (Sealy and Chalkley. 1978 Cell 14:115-121; Latham et al. Nucl Acids Res 40:4794-4803, Waldecker et al. 2008 J Nutr Biochem 19:587-593). Certain therapeutic effects of butyrate are reportedly mediated, at least in part, by inhibition of HDACs. Such studies clearly demonstrate an important role of HDAC across therapeutic areas and directly link the SCFAs butyrate and propionate, which are produced by the human microbiome, to desirable functional outcomes via HDAC inhibition.

In some embodiments, the HDAC activity of bacteria considered for assignment to a DBC is assayed. Such bacteria are useful in DBCs in which HDAC inhibition is desirable to achieve a therapeutic outcome or in which a role for an SCFA has been identified. Examples of methods for such selections are provided in the Examples.

Applicants have demonstrated that the ability of a bacterial species to inhibit HDAC is influenced by the nutrient source provided to the bacteria. Accordingly, in some indications, a prebiotic may be provided as part of the therapeutic use of a DBC. The prebiotic is selected, at least in part, based on the ability of bacteria in the DBC to use the prebiotic as a nutrient source to increase SCFA production.

### Indoles and tryptophan metabolism

Indole and indole derivatives have been implicated in host physiology (reviewed in Zhang and Davies, 2016 Genome Medicine 8:46). For example, indole and indole-3-aldehyde are agonists of the aryl hydrocarbon receptor (AhR), which reportedly induce IL-22 expression and increase Th-17 activity. Indole has also been reported to increase epithelial tight junction resistance, mucin production, and colonization resistance (Bansal et al. 2010 Proc Nat Acad Sci 107:228-233). Indole-3-acetate, tryptamine, and skatol are also AhR ligands (Hubbard et al. 2015. Drug Metabolism and Disposition 43:1522-35) and therefore may play similar roles in host biology. Indole-3-propionate, also a product of tryptophan metabolism by commensal bacteria, is a ligand for the pregnane H receptor (PXR). PXR activation downregulates TNFα and upregulates tight junction proteins thus promoting immune homeostasis and improved intestinal barrier function (Venkatesh et al. 2014. Immunity 41:296-310 and Romagnoli et al. 2016. J Immunol 196 Suppl. 67.10). Furthermore, an AhR agonist has been reported to ameliorate the impact of *C*. *difficile* infection in a murine model (Julliard et al., Ann. Srg. PMID 27280500). Furthermore, in a study in which bacterial spores derived from feces of healthy humans were used to treat recurrent *C*. *difficile* infection, tryptophan metabolism was observed to be increased; the fecal concentration of tryptophan decreased and there was an increase in some indole derivatives (data not shown).

Accordingly, applicants have discovered that selected bacteria are capable of metabolizing tryptophan and as such are useful additions to a DBC. Example 8, *infra,* provides an example of a method for identifying and characterizing such bacteria as well as providing specific examples of such bacteria.

### Carbon source utilization

Another useful feature of a DBC useful for treating or preventing a disorder associated with the presence of a pathogen is carbon source competition, e.g., inclusion of bacteria in a DBC that can use one or more carbon sources in common with those used by the pathogen, e.g., a *C. difficile.* Examples 3A and 3B provide an examples of how this feature can be identified and provides examples of bacterial species that are useful, e.g., for treating or preventing CDI, and are suitable for use in a DBC.

### Formulations

In some embodiments, treatment includes administering a DBC to a subject (for example, a patient at risk for, recently treated for, or that has been diagnosed with *C*. *difficile* infection), the composition comprising the DBC and a pharmaceutically acceptable carrier. In some embodiments, the DBC is an oral dosage form. In some embodiments, the DBC comprises, as the active component a consortium of bacteria as described herein in combination with one or more pharmaceutically acceptable carriers (excipients). In making the compositions of the invention, the DBC is typically mixed with an excipient, diluted by an excipient or enclosed within such a carrier in the form of, for example, a capsule, sachet, paper, or other container. When the excipient serves as a diluent, it can be a solid, semisolid, or liquid material, which acts as a vehicle, carrier or medium for the active component. Thus, a formulation can be in the form of a tablet, pill, powder, lozenge, sachet, cachet, elixir, suspension, emulsion, solution, syrup, aerosol (as a solid or in a liquid medium), ointment containing, for example, up to 10% by weight of the active component, soft capsule, hard capsule, gel-cap, tablet, suppository, solution, or packaged powder. Suitable excipients include, for example, PBS, glycerol, cocoa butter, or polyethylene glycol.

In preparing a formulation, the DBC can be milled to provide the appropriate particle size prior to combining with the other ingredients.

The compositions can be formulated so as to provide quick, sustained or delayed release of the active component after administration to the patient, for example, for release in the colon, by employing methods and forms known in the art.

A DBC can be formulated in a unit dosage form, each dosage form containing from about 10² to about 10⁹ spores, for example, about 10⁴ to about 10⁸ spores. The term "unit dosage forms" refers to physically discrete units suitable as unitary dosages for human subjects and other mammals, each unit containing a predetermined quantity of active component calculated to produce the desired therapeutic effect, in association with a suitable pharmaceutical excipient. In some cases, more than one unit dosage form constitutes a dose. For example, a single dose can be one unit dosage form, two dosage unit forms, three dosage unit forms, four unit dosage forms, five unit dosage forms or more. In some cases, the number of unit dosage forms constituting a single dose is 1, 2, 3, 4, 5, 6, 7, 8, 9, 10, 11, 12, 13, 14, 15, 20, 25, or 30 unit dosage forms. A single dose can be, e.g., 10³ to about 10⁹ spores, for example, about 10⁴ to about 10⁸ spores. In an example, a dose is 1, 2, 3, or 4 capsules containing a total of between 10e2 and 10e8 spores in the dose. In the case of a single dose having multiple dosage forms, the dosage forms are generally delivered within a prescribed period, e.g., within 1 hour, 2 hours, 5 hours, 10 hours, 15 hours, or 24 hours.

A composition described herein can be effective over a wide dosage range and is generally administered in a pharmaceutically effective amount.

A tablet or pill comprising a composition described herein can be coated or otherwise compounded to provide a dosage form, for example, to ease delivery (for example, by improving swallowability) or to improve delivery to a targeted area of the gastrointestinal tract such as the colon.

In some embodiments, the tablet or pill comprises an inner component surrounding the composition and an outer component, the latter serving as an envelope over the former. The two components can be separated by an enteric coating layer that may resist disintegration in the stomach and permits the inner component to pass intact into the duodenum or to be delayed in release.

In some embodiments, a formulation comprising a DBC is administered via a nasogastric route, by endoscopy or other suitable method of delivering the formulation at or near a desired site, for example, the upper intestinal tract (e.g., stomach and/or duodenum) or the lower intestinal tract (e.g., small intestine and/or large intestine). Effective doses can be extrapolated from dose-response curves derived from in vitro or animal model test systems or from clinical studies.

Furthermore, the formulations can optionally be administered in combination with antacids that are known in the art.

### Methods of treating a dysbiosis

DBCs as described herein are useful for administration to a subject, e.g., a mammal such as a human in need of treatment, e.g., to prevent or treat a dysbiosis. In some embodiments, the mammalian subject is a human subject who has one or more symptoms of a dysbiosis, including but not limited to overgrowth of an undesired pathogen or an undesired taxon such as the *Enterobacteriaceae* or vancomycin-resistant Enterococcus, reduced representation of one or more key bacterial taxa such as the Bacteroidetes or Firmicutes or genera or species thereof, or reduced diversity of microbial species compared to a healthy individual, reduced overall abundance of anaerobic bacteria, or reduced overall abundance of bacteria capable of carrying out a particular function, e.g., bile acid metabolism. As used herein, "treating a dysbiosis" includes, for example, treating a dysbiosis-associated disease such as *C*. *difficile* infection, preventing recurrent *C*. *difficile* infection, or treating or preventing vancomycin-resistant Enterococcus infection (VRE). It is understood that preventing can mean reducing the risk of a dysbiosis.

In some embodiments, the subject receives an antibiotic treatment prior to administration of the DBC. In some embodiments, the subject receives an antibiotic treatment and does not receive the DBC until at least one day, two days, three days, 5 days, one week, two weeks, three weeks, or four weeks has elapsed since the antibiotic treatment and prior to administration of the DBC. In some embodiments, the subject receives multiple doses of DBC to ensure coverage of the dosing period. In some embodiments, the subject has symptoms of a dysbiosis prior to administration of the DBC. In other embodiments, the subject does not exhibit symptoms of the dysbiosis prior to administration of the DBC, e.g., the DBC is administered prophylactically to reduce the risk that a dysbiosis will result in clinical symptoms, e.g., infection with a pathogen.

In some embodiments, the DBC is administered to ameliorate the effects of a gastrointestinal disease, disorder or condition, for example, *C. difficile* infection, inflammatory bowel disease (IBD) (e.g., ulcerative colitis and Crohn's disease), irritable bowel disorder or other disorder associated with a GI dysbiosis. In some embodiments, the DBC is administered to a subject exhibiting diarrhea or other symptom caused by, e.g., *C*. *difficile* including recurrent *C*. *difficile* infection, inflammatory bowel disease (e.g., ulcerative colitis, Crohn's disease) or colitis.

In some embodiments, the DBC is administered only once prior to improvement of the disease, disorder or condition. In some embodiments the therapeutic composition is administered at intervals greater than two days, such as once every three, four, five or six days, or every week or less frequently than every week, e.g., every two weeks, every three weeks, every 4 weeks, every six weeks, every eight weeks, every twelve weeks, once per month, once per two months, once per three months, once per four months, or once per six months. In some cases, the DBC is administered intermittently according to a set schedule, e.g., once a day, once weekly, or once monthly, or when the subject relapses from the primary illness. In another embodiment, the DBC is administered on a long-term basis to individuals who are at risk for infection with or who may be carriers of these pathogens, including individuals who will have an invasive medical procedure (such as surgery), who will be hospitalized, who live in a long-term care or rehabilitation facility, who are exposed to pathogens by virtue of their profession (livestock and animal processing workers), or who could be carriers of pathogens (including hospital workers such as physicians, nurses, and other healthcare professionals). In some cases, the DBC is administered following the use of antibiotics to prevent the development of a dysbiosis and its symptoms, such as antibiotic-associated diarrhea.

Also provided are methods of treating or preventing a subject suffering from or at risk of developing a metabolic disease, and disorder or condition selected from the group consisting of diabetes, metabolic syndrome, obesity, heart disease, autoimmune disease, liver disease, and autism using the therapeutic compositions provided herein.

In embodiments, the bacterial spore composition is generally administered enterically. For example, administration can be oral administration via a swallowed form (e.g., a pill, sachet, capsule, syrup or the like), or by an oral or nasal tube (including nasogastric, nasojejunal, oral gastric, or oral jejunal). In other embodiments, administration includes rectal administration (for example, by enema, suppository, or colonoscopy). The DBC can be administered to at least one region of the gastrointestinal tract, including the mouth, esophagus, stomach, small intestine, large intestine, or rectum. A DBC can be administered orally in the form of a medicament such as a powder, one or more capsules, one or more tablets, a gel or a liquid. A DBC can also be administered in gel or liquid form by the oral route or through a nasogastric tube, or by the rectal route in a gel or liquid form, by enema or instillation through a colonoscope or by a suppository.

The subject may have a colonic-cleansing preparation prior to administration of a DBC. Methods of colonic-cleansing are known in the art such as those used to prepare a subject for a colonoscopy. Also, the subject may optionally be treated with an antacid or buffering agent to increase stomach pH at the time of DBC administration, as is known in the art and determined to be appropriate for the subject.

To evaluate a subject, signs or symptoms of a dysbiosis are evaluated post-treatment ranging from 1 day to 6 months after administration of the DBC. One method of evaluation involves obtaining fecal material from the subject and assessment of microbes present in the gastrointestinal tract, e.g., using 16S rDNA or metagenomic shotgun sequencing analysis or other analyses known in the art. Population of the gastrointestinal tract by bacterial species present the DBC as well as augmentation by commensal microbes not present in the DBC can be used to indicate an improvement in the dysbiosis. In the case of infection, a decrease in the level of the infective entity, e.g., *C. difficile,* after treatment with a DBC compared to the level of the infective entity prior to treatment can be used to indicate efficacy of the DBC treatment.

The embodiments within the specification provide an illustration of embodiments and should not be construed to limit the scope. The skilled artisan readily recognizes that many other embodiments are encompassed. All publications and patents cited in this disclosure are incorporated by reference in their entirety. To the extent the material incorporated by reference contradicts or is inconsistent with this specification, the specification will supersede any such material. The citation of any references herein is not an admission that such references are prior art.

### EQUIVALENTS

All technical features can be individually combined in all possible combinations of such features.

The invention may be embodied in other specific forms without departing from the spirit or essential characteristics thereof. The foregoing embodiments are therefore to be considered in all respects illustrative rather than limiting on the invention described herein.

### EXAMPLES

The following non-limiting examples further illustrate embodiments of the inventions described herein.

### Example 1: Murine model of Clostridium difficile infection (CDI)

The efficacy of test compositions was investigated using a mouse challenge model of CDI (Chen et al., 2008, Gastroenterology 135:1984-1992). The mouse CDI model is used to demonstrate prevention of infection. In this model, mice receive an antibiotic pretreatment to create a dysbiosis in the gut that increases susceptibility to CDI. When challenged with orally administered *C*. *difficile* spores, mice exhibit symptoms of CDI including body weight loss, diarrhea and lethargy with peak disease between days 2-3 post-*C*. *difficile* inoculation. Infection can be lethal and death occurs during peak disease. In mice surviving the infection, symptoms are mainly resolved by day 6 after inoculation. Animals are kept in a bioBubble Clean Room or equivalent facility for the duration of the experiment.

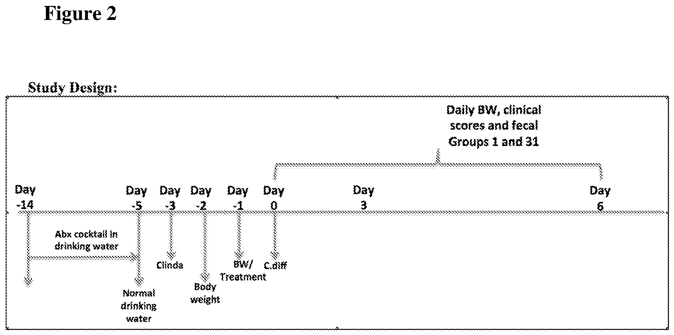

Briefly, on Days -14 to -6, animals (female C57BL/6 mice, nine-ten weeks old) received an antibiotic cocktail in their drinking water consisting of 1% glucose, kanamycin (0.5 mg/mL), gentamicin (0.044 mg/mL), colistin (1062.5 U/mL), metronidazole (0.269 mg/mL), ciprofloxacin (0.156 mg/mL), ampicillin (0.1 mg/mL) and vancomycin (0.056 mg/mL). On Day -3, animals received a dose of 10 mg/kg clindamycin by oral gavage. On Day -1, test articles, a human feces-derived spore preparation (study efficacy control; HBS) and FSV (human FMT control) were thawed on ice, centrifuged for five minutes at 12,100 RCF, decanted to remove the supernatant, and resuspended in sterile PBS. Test articles (DBCs) were designed consortia of bacteria. The control for a test article inoculum was sterile PBS. Animals were dosed by oral gavage with a volume of 0.2 mL of appropriate treatment. Figure 2 provides a schematic of the study design.

On Day 0, mice were challenged by administration of approximately 4.5 log10 spores of *C*. *difficile* or sterile PBS (for the naive control arm) via oral gavage. Infection and its consequences were evaluated by daily assessment of mortality, weight loss and clinical signs and symptoms (the clinical score; scoring of lethargy, grooming, wet tail/abdomen, and hypothermia).

### Example 2: Test articles

Applicants have tested about 100 different DBCs. In one example of such an experiment, the murine model described in Example 1 was used to evaluate the efficacy of various orally administered microbial spore preparations for treating/preventing *Clostridium difficile* infection (CDI). Nine compositions were tested at estimated doses ranging from 1e2 to 1e5 per individual species in a composition. Estimates of the number of bacteria were based on spore colony forming unit (SCFU) assays (i.e., number of spore-derived colonies grown on a plate from a stock). Methods of performing such assays are known in the art, typically including a germinant appropriate for the species to be grown. Some compositions tested are provided in Table 3 (*supra*). Negative controls included PBS alone as treatment and naïve animals (not infected with *C*. *difficile*)*.* Positive controls included treatment with a slurry of healthy human feces (FSV), and treatment with a population of bacterial spores derived from human feces (HBS).

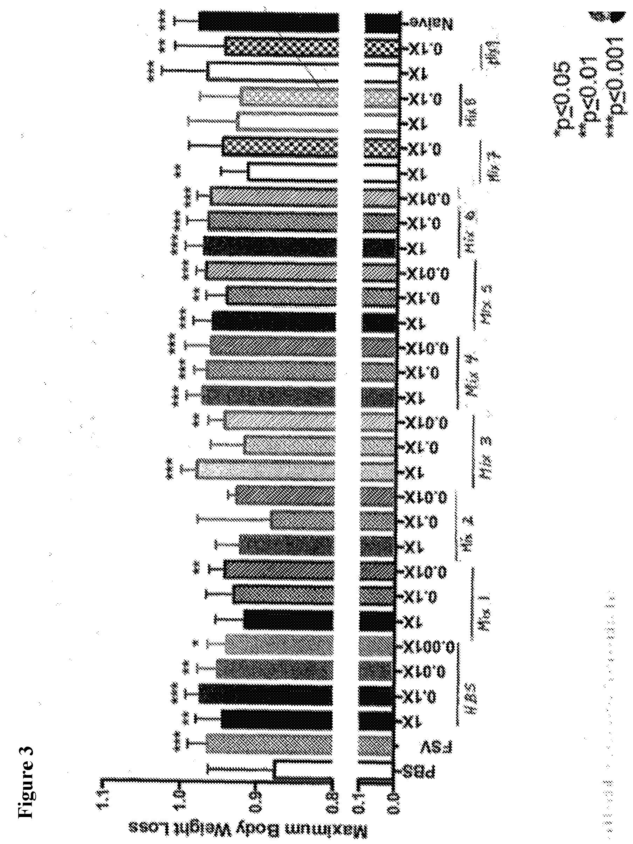

Mortality in the untreated group of mice challenged with *C*. *difficile* was at least 20%. Surprisingly, it was found that some test compositions were at least as effective in inhibiting/preventing CDI in the murine model as a composition prepared from feces of healthy humans or a bacterial spore preparation prepared from healthy human feces (HBS). In general, tested compositions varied in their ability to ameliorate the effects of *C. difficile* infection in the murine model, e.g., maximum decrease in body weight from pre-treatment baseline (Figure 3), lethality, and clinical signs. Some compositions were observed to be effective over a broad concentration range (1e2 to 1e5 SCFU per species of the composition per mouse). Examples of effective compositions include DBC 4, DBC 6, and DBC 9.

In further experiments, it was demonstrated that a DBC was able to prevent CDI-related mortality, prevent CDI-related symptoms, and significantly decrease the negative change in body weight compared to the PBS-treated mice challenged with *C. difficile.*

Accordingly, in some embodiments of the invention, a DBC can effectively prevent or inhibit a range of features caused by CDI infection, for example one or more of weight loss, diarrhea, and mortality.

These data demonstrate that a designed bacterial composition containing a relatively limited number of specific bacterial species can be used as a treatment for prevention of a dysbiosis, as demonstrated by treatment of CDI in a murine model.

### Example 3A: Carbon utilization

Antibiotics are well known to disrupt the gut microflora resulting in loss of colonization resistance and setting the stage for infections by pathogens, including *C. difficile* (reviewed by Keeney et al. 2014 Ann Rev Microbiol, June 2, 2014. doi:10.1146/annurev-micro-091313-103456). The contribution of nutrient competition to the resistance to colonization by *C. difficile* was suggested in early experiments in continuous flow models in which glucose, N-acetylglucosamine, and a sialic acid (N-acetylneuraminic acid) were identified as *C. difficile* carbon sources that were of limited availability due to catabolism by other gut organisms (Wilson and Perini, 1988 Infection and Immunity 56: 2610-14). More recently, in a mouse model, sialic acid utilization by *C. difficile* was associated with higher *C. difficile* levels (Ng et al. 2013 Nature advance online publication (September 1, 2013). doi:10.1038/nature12503). Accordingly, a useful feature of a DBC is bacteria that have the ability to utilize carbon sources that overlap with those used by one or more *C. difficile* strains (e.g., toxigenic *C. difficile* strains).

In some embodiments of the invention, selection of compositions includes selection of strains, OTUs, or species that utilize at least one carbon source that is used by a targeted pathogen. To demonstrate this, the species used in a DBC effective for inhibiting or preventing *C. difficile* infection were tested for utilization of carbon sources used by *C. difficile,* termed herein *"C. difficile* carbon sources". In these experiments, a carbon source utilization profile was determined for strains in a DBC. Growth on a panel of 59 carbon sources was assayed in a 96-well plate format by measuring optical density of cultures. Briefly, 1% solutions of 59 carbon sources were prepared and filter sterilized. 96-deep well plates were filled with 0.4 mL of 2X MCB base medium per well (composition 1X: 10 g/L beef extract, 3 g/L yeast extract, 10 g/L peptone, 5 g/L NaCl, 6.8 g/L KH₂PO₄, 2 mM MgSO₄, 0.5 mM CaCl₂, 0.1 mM MnClz, 0.25 g/L cysteine (added prior to inoculation, see below), 10 mg/L hemin, 1 mg/L menadione, 10.5 g/L MOPS, pH 7). Each carbon source was added into 3 wells. Two plates were prepared for each strain. Each plate contained 29 carbon sources, glucose and 3 wells with base medium only. 8 µL of 2.5% cysteine solution was added to each well before inoculation.

Bacterial strains to be used for inoculation were streaked from frozen stocks onto brain heart infusion (BHI) plates and incubated for 24 hours at 37 °C. A loopful of cell material was transferred into 5 mL of pre-reduced PBS and resuspended.

8 µL of inoculum was added to 95 wells of each plate. 1 well on each plate was a control receiving no cells. Plates were covered with a breathable membrane and incubated at 37 °C in a ziplock pack for 72 hours. 0.2 mL from each well was transferred into a clear 96-well flat-bottom plate and OD₆₀₀ (optical density at 600 nm wavelength) was measured in a spectrophotometer. OD₆₀₀ for each well was corrected for the value of inoculated wells with base medium only. Average OD₆₀₀ was calculated for each carbon source for each strain. A result was considered positive if the average corrected OD₆₀₀ was ≥15% of the maximum corrected OD₆₀₀ recorded for a given strain.

Each strain grew on multiple carbon sources (ranging from 10 to 30 sources). All strains were saccharolytic, using from 10 to 24 different sugars or sugar alcohols out of 29 tested, with the exception of one strain, which used only 3 sugars in the panel. Glucose was used by all strains. Seven strains were able to use at least one amino acid (out of 15 tested) as a carbon source. Data from these experiments are shown in Figure 4.

The selected DBC strains were shown to be capable of utilizing 26 of the 29 (90%) tested carbon sources used by *C. difficile,* comprising 19 of the 19 (100%) of the carbohydrate and carboxylic acid sources tested. Accordingly, in some embodiments, a DBC useful for treating *C. difficile* comprises types of bacteria capable of using carbon sources also used by *C. difficile,* for example at least 90% of the panel of carbon sources disclosed herein.

Accordingly, in some embodiments, a DBC includes non-pathogenic bacteria that can utilize amino acids and other materials (e.g., sugars, sugar alcohols, carbohydrates, and carboxylic acids) as carbon sources.

### Example 3B: Additional carbon utilization

Experiments supplementing those described in Example 3A were carried out to extend knowledge of the carbon sources that can be used by *C. difficile* strains and the repertoire of carbon utilization for bacteria that may be useful in a DBC.

Three *C. difficile* strains and 12 non-*C*. *difficile* bacterial strains were profiled using a panel of carbon sources. As used herein, unless otherwise indicated, carbon sources includes all sources tested as growth substrates in the experiments herein. The three *C. difficile* strains were *Clostridium difficile* ATCC 9689, *Clostridium difficile* ATCC 43593, and *Clostridium difficile* ATCC 43255, all of which are available from the American Type Culture Collection (ATCC).

Growth of the strains was tested on 87 different carbon sources in a 96-well plate format. The panel contained 34 carbon sources reported to be utilized by C. *difficile* in the literature (Hafiz and Oakley. 1976 J Med Microbiol 9:129-36.; Wilson and Perini. 1988 Infection and Immunity 56:2610-14; Bergey and Holt. Bergey's Manual of Determinative Bacteriology. Baltimore: Williams & Wilkins, 1994; Sebaihia et al. 2006 Nature Genetics 38:779-786) including amino acids, mono-, di-, tri- and polysaccharides, glucoside and sugar alcohols. Carbon sources were added at 0.5% into three different base media (Base medium 1 SSNC composition: beef extract 1 g/L, yeast extract 2 g/L, bacto soytone 6 g/L, NaCl 4 g/L, KH₂PO₄ 5.44 g/L, MgSO₄ 2 mM, CaCl₂ 1 mM, MnCl₂ 0.1 mM, cysteine hydrochloride 0.25 g/L, hemin 10 mg/L, menadione 1 mg/L, MOPS 6.3 g/L, pH 7; Base medium 2 SPOSCR composition: beef extract 1 g/L, yeast extract 2 g/L, bacto soytone 6 g/L, NaCl 4 g/L, KH₂PO₄ 5.44 g/L, MgSO₄ 2 mM, CaCl₂ 1 mM, MnCl₂ 0.1 mM, cysteine hydrochloride 0.25 g/L, hemin 10 mg/L, menadione 1 mg/L, MOPS 8.4 g/L, NaHCO₃ 0.4%, pH 7; Base medium 3 YCFA: casitone 10 g/L, yeast extract 2.5 g/L, cysteine hydrochloride 1 g/L, hemin 10 mg/L, ATCC vitamin solution 10 mL/L, ATCC trace element solution 10 mL/L, K₂HPO₄ 0.45 g/L, KH₂PO₄ 0.45 g/L, (NH₄)₂SO₄ 0.9 g/L, MgSO₄x7H₂O 90 mg/L, CaCl₂ 90 mg/L, acetic acid 33 mM, propionic acid 9 mM, isobutyric acid 1 mM, isovaleric acid 1 mM, valeric acid 1 mM, pH 7).

Positive growth was scored by measuring optical density at 600 nm and comparing it to growth in base medium without an added carbon source. For two, base media acid production was also scored by measuring optical density at 558 nm of pH-indicator phenol red mixed with spent medium from each culture.

All three *C. difficile* strains metabolized a variety of carbon sources; 53 out of 87 carbon sources tested were used by at least one strain (Table 5). All three strains were saccharolytic, using different sugars or sugar alcohols, and all three strains used amino acids as a carbon source (Table 5 and Table 6). Four of the carbon sources reported to be utilized by *C. difficile,* as noted above, had no hits for the three *C. difficile* strains tested in this assay (starch, sucrose, glycogen, and dulcitol), 23 other carbon sources in the panel had at least one hit for at least one of the three *C. difficile* strains, bringing the total number of *C. difficile* carbon sources detected to 53.

Of 53 carbon sources used by *C. difficile* in this assay, individual bacterial strains tested used from 16 to 29 in at least one base medium. The carbon source utilization patterns of non-*C. difficile* strains differed from those of the *C. difficile* strains with overlapping carbon sources ranging from 30-55%. All tested bacterial strains together can utilize 52 of the 53 (98%) carbon sources used by *C. difficile.* These data demonstrate that non-*C. difficile* bacterial strains can utilize multiple *C. difficile* carbon sources. This feature of bacteria that can compete for multiple carbon sources with a pathogen is useful for designing a DBC, for example by inclusion of bacteria in a DBC that can utilize multiple carbon sources that can also be used by a *C. difficile.*

The data of Table 6 indicate that a useful feature of a DBC is inclusion of bacteria that can together utilize a variety of carbon sources, particularly given the variability in carbon source use by different strains of *C. difficile.*

Carbon sources reported to be utilized by *C. difficile* but negative in this experiment are each utilized by at least four of the non-*C. difficile* strains. Therefore, even when multiple strains of a pathogen are characterized for carbon source utilization, a DBC having a broad carbon utilization profile covering even some sources not used by test *C. difficile* strains can be desirable.

These data show that selected bacterial strains can utilize multiple *C. difficile* carbon sources, including mono-, di-, tri- and polysaccharides, sugar alcohols, glycosides, carboxylic acids, alcohols, bile salts and amino acids. Microbiota-liberated host sugars such as sialic acid have been reported to facilitate expansion of *C. difficile* in animal models of infection. Ethanolamine is another host derived compound that can give pathogens a competitive advantage in the GI environment, and *C. difficile* has an operon for ethanolamine utilization (Ng et al. Nature advance online publication (September 1, 2013). doi:10.1038/nature12503; Garsin Nat Rev Microbiol. 2010 Apr;8(4):290-5. doi: 10.1038/nrmicro2334). In these experiments, *C. difficile* was confirmed to metabolize both sialic acid and ethanolamine. Accordingly, in some cases, a DBC strains that can utilize, e.g., sialic acid and/or ethanolamine are useful. Eight and three strains out of the 12 selected strains were also able to utilize sialic acid and ethanolamine, respectively. These data support a model in which carbon source competition may contribute to the mechanism of efficacy against *C. difficile* of a DBC that includes bacteria having the ability to utilize sialic acid and ethanolamine.

**Table 5. Carbon sources used by the three C. difficile strains tested and the number of strains testing positive for a given carbon source.**

| **C-source** | **n** | **C-source** | **n** |
|---|---|---|---|
| **apple pectin** | **2** | **arginine** | **1** |
| **pectin** | **2** | **glycine** | **3** |
| **polygalacturonate** | **2** | **ornithine** | **3** |
| **galactomannan** | **1** | **leucine** | **3** |
| **guar** | **1** | **isoleucine** | **2** |
| **cyclodextrin** | **3** | **alanine** | **3** |
| **melezitose** | **2** | **threonine** | **2** |
| **cellobiose** | **2** | **serine** | **2** |
| **trehalose** | **3** | **valine** | **3** |
| **ribose** | **3** | **asparagine** | **3** |
| **xylose** | **3** | **aspartic acid** | **2** |
| **fructose** | **3** | **methionine** | **3** |
| **glucose** | **2** | **phenylalanine** | **3** |
| **mannose** | **3** | **glutamine** | **1** |
| **rhamnose** | **1** | **lysine** | **1** |
| **sialic acid** | **2** | **tryptophan** | **3** |
| **N-acetylglucosamine** | **3** | **cysteine** | **3** |
| **mucic acid** | **1** | **succinate** | **3** |
| **mannitol** | **2** | **α-ketoglutarate** | **1** |
| **sorbitol** | **2** | **citrate** | **1** |
| **xylitol** | **2** | **oxalate** | **2** |
| **salicin** | **3** | **formate** | **1** |
| **esculin** | **3** | **ethanolamine** | **2** |
| **arbutin** | **3** | **propanediol** | **1** |
| **oxgall** | **2** | **taurochenodeoxycholate** | **1** |
| **glycocholate** | **1** | **glycochenodeoxycholate** | **1** |
| **mucin** | **2** | | |

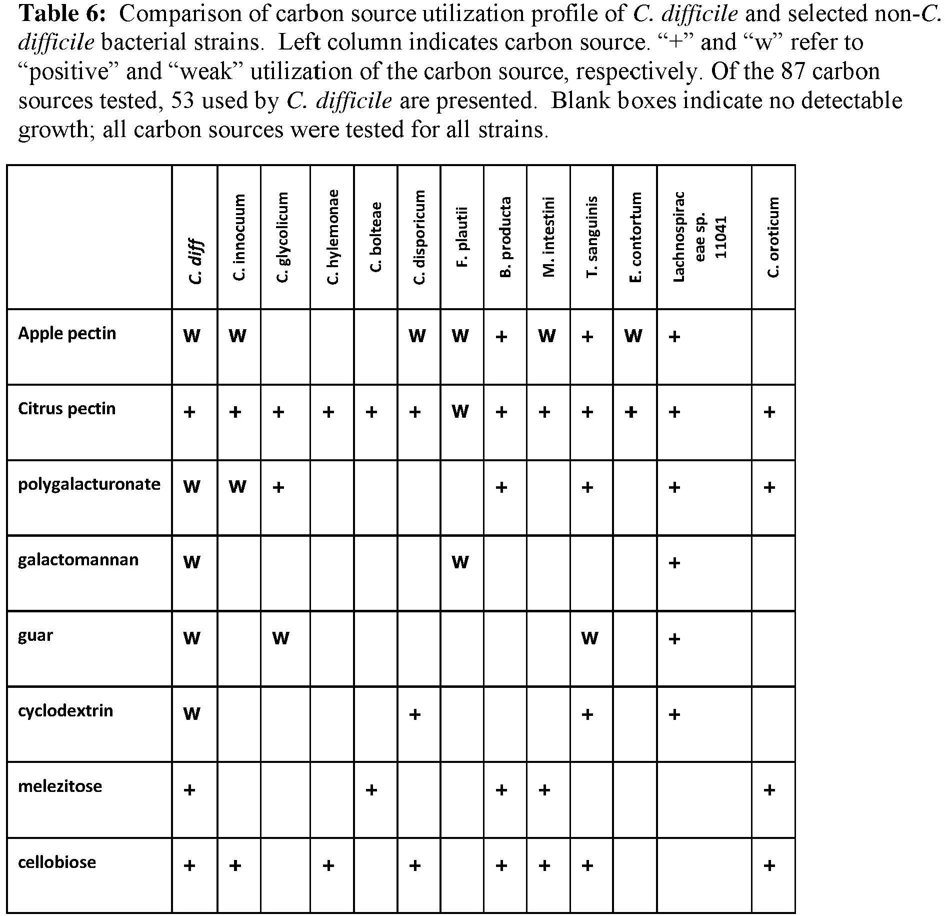

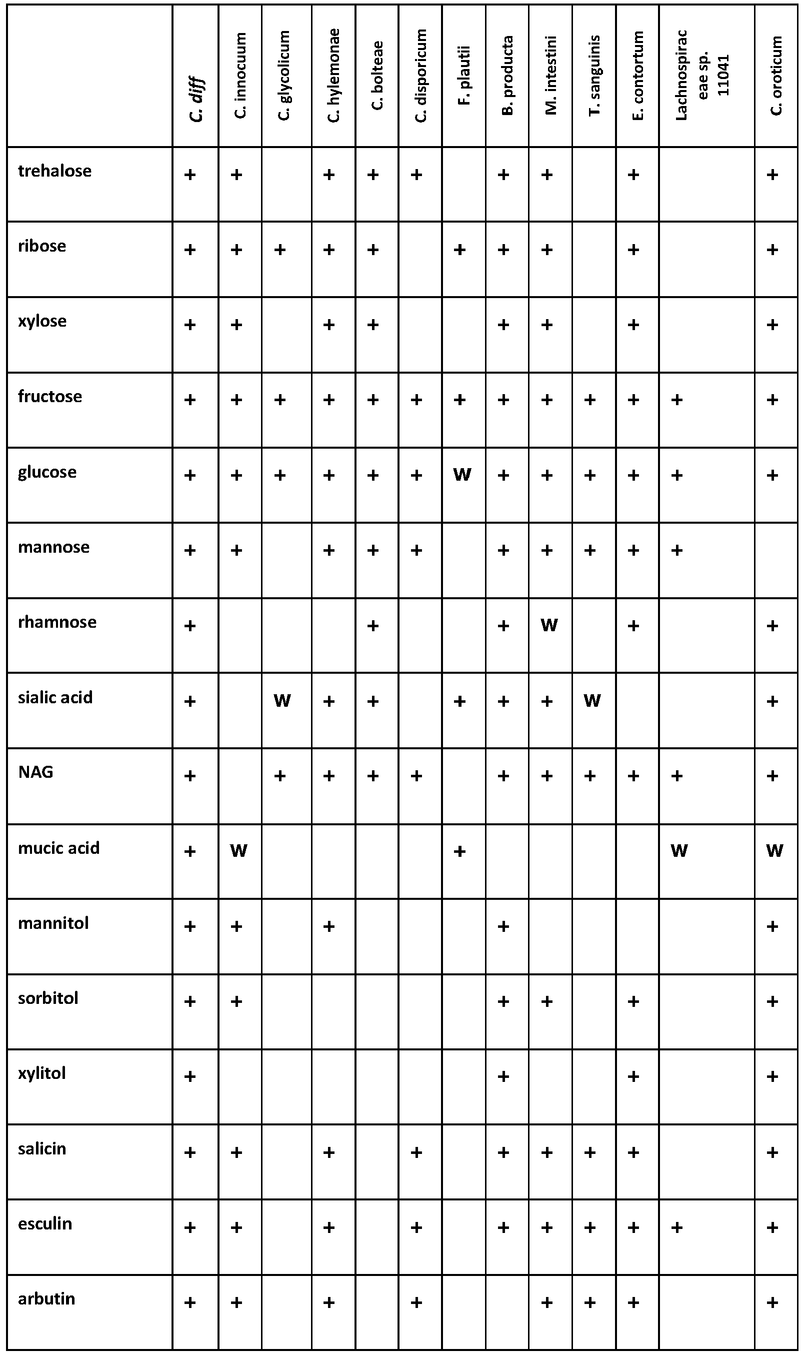

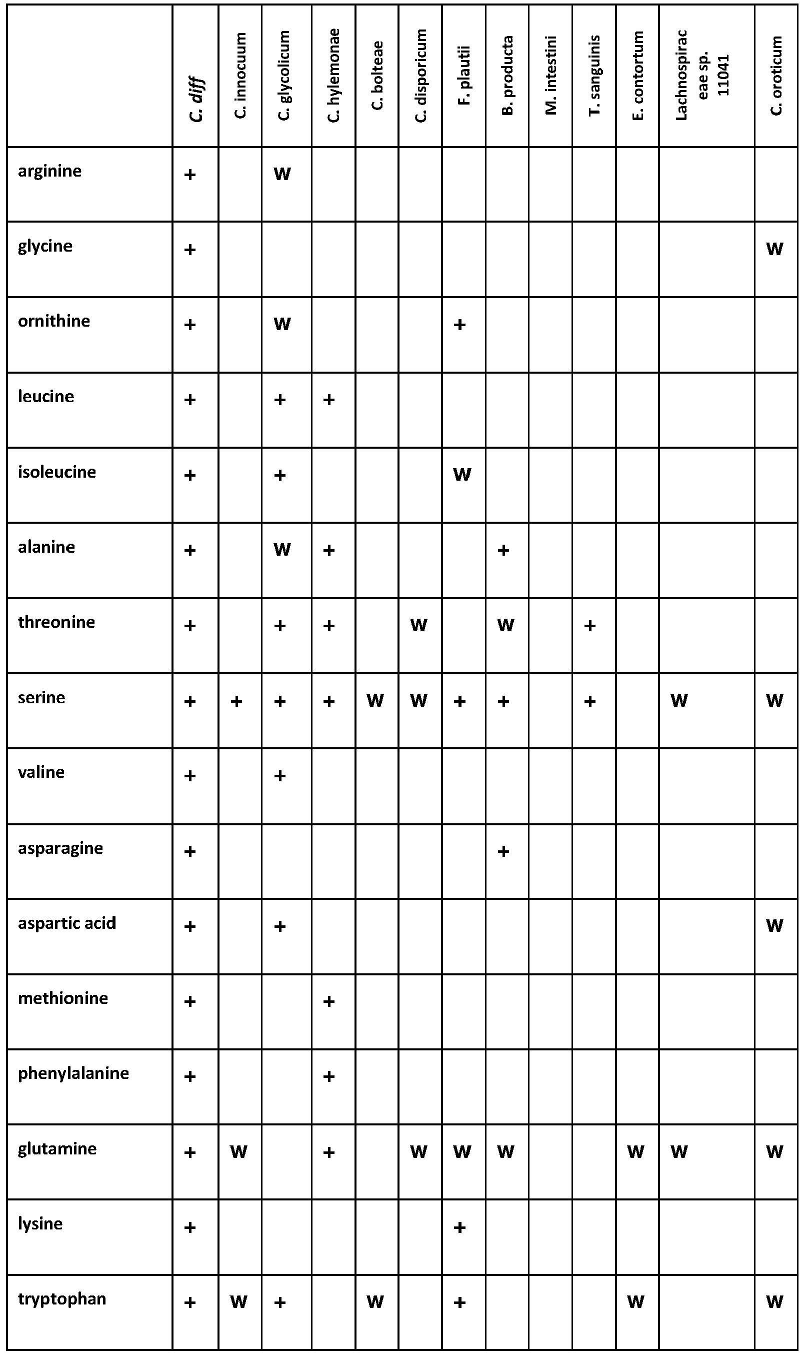

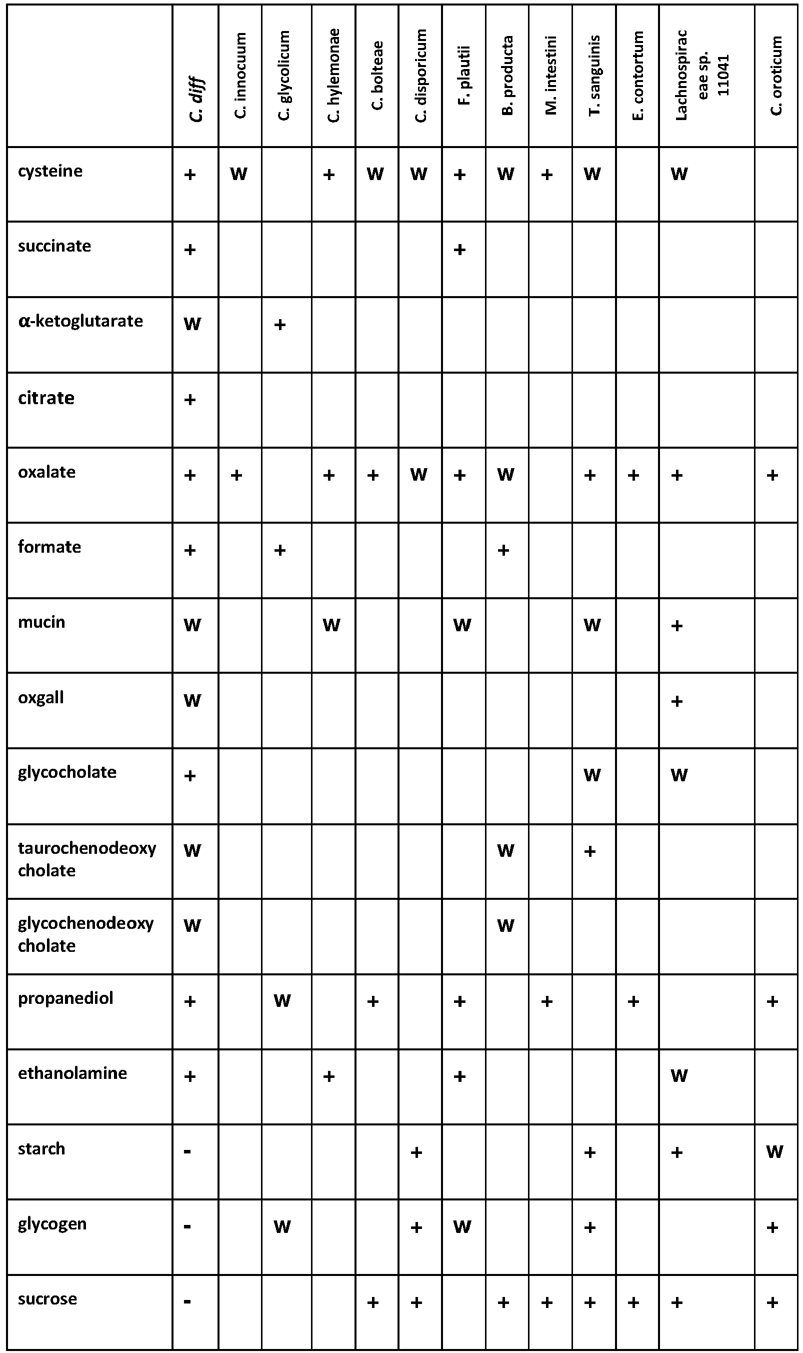

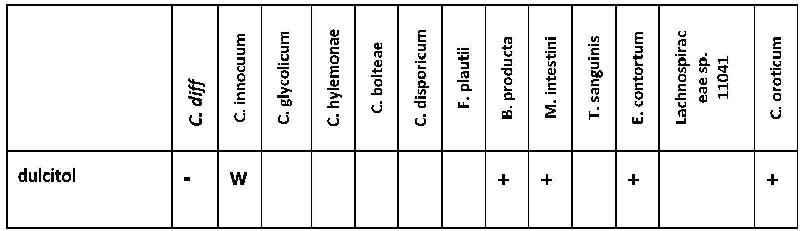

### Example 4: Bile Acid Metabolism/Thin Layer Chromatographic analyses

The production of secondary and tertiary bile acids results from the sequential modification of primary bile acids through a series of reactions catalyzed by specific bacterial enzymes (e.g., Ridlon et al., 2006, J Lipid Res 47: 241-259). To cause disease *C. difficile* spores first germinate in the host GI tract and germination of *C. difficile* spores can be promoted by certain bile acids. In addition, vegetative *C. difficile* growth is limited by certain bile acid metabolites. Accordingly, a useful functional feature of a DBC is the ability to catalyze the conversion of bile acid pools that promote *C. difficile* germination to pools that do not promote germination and/or inhibit germination or the outgrowth of *C. difficile.* For example, in some embodiments, bacteria that can express enzymes that convert *C. difficile*-promoting bile acids to bile acids that do not promote *C. difficile* germination are included in a DBC. Compositions that include such bacteria are useful for treating or preventing *C. difficile* infection; e.g., preventing recurrence of *C. difficile* infection. In one example, bacteria selected for a DBC can express at least one, two, or three enzyme activities that are key in bile acid regulation, e.g., bile salt hydrolase (BSH), hydroxysteroid dehydrogenase (7, 3, or 12 α-HSDH) and 7α-dehydroxylase. Methods of identifying such bacteria are provided in non-limiting examples, *infra.*

### Bile salt hydrolase (BSH) activity screen using thin layer chromatography (TLC)

Deconjugation of conjugated bile acids is catalyzed by bile salt hydrolases (BSH), which are expressed by some bacterial species isolated from human and animal GI tracts. Bacterial species as diverse as *Clostridia, Bacteroides, Bifidobacteria,* and *Lactobacilli* may express a functional BSH. Deconjugation is a requisite step for further processing of conjugated bile acids by the GI microbiome, and therefore plays an important role in shaping the bile acid composition of the GI tract. Some conjugated bile acids have been shown to promote *C. difficile* spore germination, and thus it may be beneficial to decrease their concentration.

In experiments to test the ability of candidate DBC bacteria for their ability to demonstrate BSH activity, bacteria were grown for 24 to 48 hours to generate sufficient biomass. For the selected bacterial strains, streak plates were generated on brain heart infusion (BHI) agar or brucella blood agar (BBA), depending upon the strain being tested. Bacterial lawns were resuspended in phosphate buffered saline (PBS) to create a homogenous suspension that was then used for the BSH assay. To assay for BSH activity, bacterial suspensions were separately incubated with four different conjugated bile acids, each bile acid at concentration of 5 mg/mL. Strains were incubated with respective bile acids for 4 hours at 37°C under anaerobic conditions. Bile acids tested in this assay include glyco-cholic acid (glycocholate; gCA), taurocholic acid (taurocholate; tCA), glycochenodeoxycholic acid (glycochenodeoxycholate; gCDCA), and tauro-chenodeoxycholic acid (taurochenodeoxycholate; tCDCA).

After incubation, plates were spun down to pellet bacteria and the supernatant from each well was loaded onto silica coated TLC plates. Samples were chromatographed in buffer (benzene:isopropanol:acetic acid; 30:30:1) and developed with *p*-anisaldehyde stain using methods known in the art. BSH activity was determined by the formation of deconjugated bile acid products appearing as additional bands on the TLC plates. Bile acids were verified by comparison to known standards run in parallel on each TLC plate.

### Hydroxysteroid Dehydrogenase (HSDH) activity screen using thin layer chromatography (TLC)

Deconjugated secondary bile acids are further modified into what some refer to as tertiary bile acids through reactions catalyzed by bacterial enzymes. This includes oxidation of bile acids to their keto-forms through the activity of bacterial HSDH enzymes specific to the 7-, 3- or 12- hydroxyl groups. HSDH enzymes can be present in a number of gut bacterial species including, for example, *Clostridia, Ruminococci* and *Eggerthella* (Tanaka et al., 1999, J Dairy Sci 82:2530-2535; Baron et al., 1991, J Bact 173:4559-4569).

### 7α-HSDH activity assay using TLC

Selected bacterial candidates for inclusion in a DBC were grown and resuspended as described *supra.* To assay for 7α-HSDH activity, bacterial suspensions were incubated aerobically with either cholic acid (cholate; CA) or chenodeoxycholic acid (chenodeoxycholate; CDCA), each at a concentration of 200 µM, for 4 hours at 37°C. Each bile acid was separately tested with each strain.

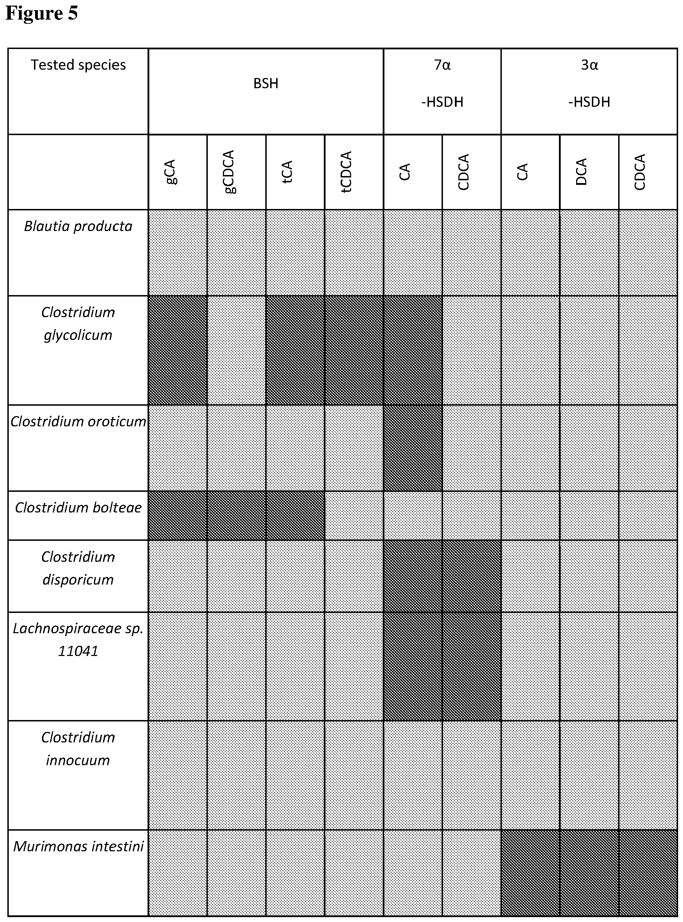

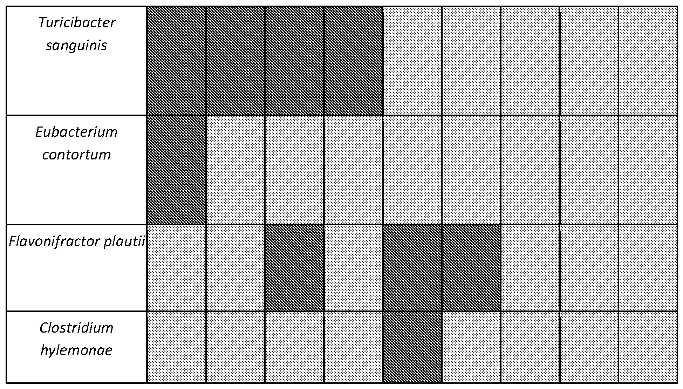

After incubation, plates were spun down to pellet bacteria and the supernatant from each well was loaded onto silica coated TLC plates. Samples were chromatographed in buffer (benzene: dioxane: acetic acid; 70:20:4) and developed with a cerium-ammonium-molybdate stain using methods known in the art. HSDH activity was determined by the formation of predicted 7-oxo-bile acid products that appeared as additional bands on the TLC plates. Bile acids were verified by comparison to known standards run in parallel on each TLC plate. Results are illustrated in Figure 5.

### 3α-HSDH activity assay using TLC

Bacteria were grown on appropriate media plates for 24 to 48 hours to generate sufficient biomass. For the candidate bacterial strains, streak plates were grown on BHI agar. Bacterial lawns were resuspended in PBS to create a homogenous suspension that was then used for the HSDH assay. To assay for 3α-HSDH activity, bacterial suspensions were incubated aerobically with either cholic acid, deoxycholic acid (deoxycholate; DCA) or chenodeoxycholic acid, each at a concentration of 100 µM, for 18-24 hours at 37°C. Each bile acid was separately tested with each strain.

After incubation, plates were spun down to pellet bacteria and the supernatant from each well was loaded onto silica coated TLC plates. Samples were chromatographed in buffer (benzene: dioxane: acetic acid) and first developed with a phosphomolybdic acid stain to detect the formation of oxo-bile acid products using methods known in the art. Candidate strains that showed HSDH activity were then specifically tested for 3-oxo HSDH activity using a second run on fresh TLC plates. The second set of plates was developed with p-anisaldehyde stain to distinguish 3-oxo bile acids (stained pink) from other bands (Devlin et al., 2015, Nat Chem Biol DOI:1-.1038/NCHEMBIO.1864). The formation of predicted 3-oxo-bile acid products that appeared as additional bands on the TLC plates confirmed the presence of 3α-HSDH activity in a strain. Bile acids were verified by comparison to known standards run in parallel on each TLC plate. Results are illustrated in Figure 5.

### Results

Overall, ten of twelve bacterial species tested in the bile acid activity assays displayed at least one of the three classes of bile acid metabolizing activity, i.e., BSH, 7α-HSDH, or 3α-HSDH activity (Figure 5) by TLC. In Figure 5, darkened boxes represent a positive result for activity metabolizing the specific indicated substrate. The light boxes represent the absence of a detectable positive result using this method. Applicants note that using more sensitive methods such as LCMS, as demonstrated in Example 5, *infra,* can reveal additional lower level activities.

Surprisingly, these data also indicate that there may be varying substrate specificities within each enzyme activity type. For example, only *T. sanguinis* demonstrated the ability to deconjugate all four conjugated bile acids that were tested and *M. intestini* was the only tested species demonstrating 3α-HSDH activity with all three bile acids tested.

These data demonstrate methods that can be used to select a consortium of bacteria to be included in a DBC that will provide a high level of one or more bile acid metabolizing activities, thereby altering the pool of bile acids available to promote *C. difficile* germination, or to inhibit *C. difficile* germination or growth.

Other methods of evaluating bile acid activities can be used, e.g., LCMS methods.

### Example 5: Bile acid metabolism/assayed using LC-MS

To improve the sensitivity of detection of enzymatic activity related to bile acid metabolism compared to the TLC method, a method using liquid chromatography mass spectroscopy (LCMS) was used. Bile acids and other small molecules can be accurately identified and quantified using a Liquid Chromatography-Mass Spectrometry (LC-MS) based approach (Kakiyama et al. 2014 J Lipid Res 55: 978-990). For most bile acids, the TLC assay demonstrates a lower limit of detection in 10-50 µM range, while the LC-MS approach extends this lower range of detection to 50-100 nM, increasing sensitivity by 100 fold or greater in many cases.

### Assessing secondary bile acid metabolism in the GI microbiome

### LC-MS screen for bile salt hydrolase (BSH) activity

The production of secondary and tertiary bile acids results from the sequential modification of primary bile acids through a series of reactions catalyzed by specific bacterial enzymes. The first step of this process, resulting in the removal of glycine or taurine groups from primary bile acids to release free unconjugated bile acids, is termed deconjugation. Deconjugation of primary bile acids is catalyzed by bile salt hydrolase (BSH). Deconjugation is a requisite step for further processing of secondary bile acids by the gut microbiome, and therefore plays an important role in shaping the bile acid composition of the gut.

To conduct the assay, briefly, bacteria were grown on permissive agar media plates for 24 to 48 hours to generate sufficient biomass to carry out assays. The bacterial lawns were resuspended in PBS to create a homogenous suspension that was then used for the BSH assay. To assay for BSH activity, bacterial suspensions were incubated with two separate mixtures of four distinct conjugated bile acids, each at concentration of 150 µg/ml, for 4 hours at 37°C under anaerobic conditions. The reaction mixture was then extracted with an equal volume of acetonitrile, spun down to pellet bacteria and the supernatant filtered through a 0.2 µM filter to generate a sample for LC-MS analysis. Bile acids were determined using an Agilent 1260 HPLC coupled to a Bruker Compact qTOF MS. Samples were run on a bidentate C18 column using an acetonitrile:water buffer system to separate bile acids which were then ionized in negative mode and identified using a qTOF-MS. BSH activity was determined by the formation of deconjugated bile acid products that were verified by comparison to known standards and quantified, if necessary, using standard curves. Primary bile acids tested in this assay included glyco/tauro-cholic acid, glyco/tauro-chenodeoxycholic acid, and glyco/tauro-muricholic acid.

### LC-MS screen for Hydroxysteroid Dehydrogenase (HSDH) activity

Deconjugated secondary bile acids are further modified into tertiary bile acids through reactions catalyzed by bacterial enzymes. This includes oxidation of bile acids to their keto-forms through the activity of bacterial HSDH enzymes specific to the 7-, 3- or 12-hydroxyl groups.

To assay for HSDH activity, bacterial suspensions were prepared as described above for the LC-MS BSH assay. To assay for HSDH activity, bacterial suspensions are first incubated with a mixture of cholic acid, chenodeoxycholic acid, deoxycholic acid and lithocholic acid at 75 µg/ml each for 18 to 24 hours at 37°C under anaerobic conditions. Assay plates were then removed from the anaerobic chamber and incubated aerobically at 37°C for 4 hours to promote the oxidation of bile acids. Once the incubation was completed, the reaction mixture was extracted with an equal volume of acetonitrile, spun down to pellet bacteria and the supernatant filtered through a 0.2 µM filter to generate a sample ready for LC-MS analysis. Bile acids in the samples were assayed as described above for the LC-MS BSH assay. HSDH activity was determined by the formation of keto-bile acid products that were verified by comparison to known standards and quantified, if necessary, using standard curves.

### LC-MS screen for 7α-dehydroxylation (7α-OH) activity

In addition to oxidation, secondary bile acids can also be modified by the removal of the hydroxyl group at the 7α position, resulting in the generation of tertiary bile acids. This includes the conversion of cholic acid into deoxycholic acid, and the conversion of chenodeoxycholic acid to lithocholic acid. The 7α-dehydroxylation reaction is catalyzed by a multi-step process, including a number of enzymes that remain uncharacterized.

Bacterial suspensions were prepared as described above for the LC-MS BSH screens. To assay for dehydroxylation activity, bacterial suspensions were incubated with a mixture of cholic acid and chenodeoxycholic acid at 150 µg/mL each for 18 to 24 hours at 37°C under anaerobic conditions. This step is necessary for the induction of enzymes involved in the reaction. Assay plates were then removed from the anaerobic chamber and incubated aerobically at 37°C for 4 hours to promote dehydroxylation of bile acids. After the incubation was complete, the reaction mixture was extracted with an equal volume of acetonitrile, spun down to pellet bacteria and the supernatant filtered through a 0.2 µM filter to generate a sample ready for LC-MS analysis. Bile acids were analyzed using LC-MS as described above. 7α-OH activity was determined by the formation of deoxycholic acid and lithocholic acid, verified by comparison to known standards, and quantified if necessary using standard curves.

### Results

Tables 7 and 8 provide the results of the LC-MS analyses of bile acid production by various bacterial strains. Table 7 lists the results of LC-MS bile acid analysis for the subset of strains mentioned in Table 9, demonstrating the increased sensitivity and coverage available with an LC-MS based assay. Table 8 describes bile acid activity of a number of additional strains, based on analysis by LC-MS. In the Tables, `+' indicates greater than 50-fold increase in levels of product bile acid, suggesting strong activity, `+/-` indicates ≤ 50-fold change in product bile acid levels, suggesting weak activity, while `-` indicates a complete lack of activity under the conditions tested. Strains with known/published activity were used as controls to monitor each reaction type.

In general, the data generated using LC-MS to analyze the metabolism of bile acids by bacterial species were consistent with the data obtained using TLC. However, the increased sensitivity of the LC-MS method revealed additional activities; for example *C. glycolicum* was demonstrated to have no BSH activity on gCDCA by TLC, but the increased resolution of LC-MS analysis showed that the bacterium does retain BSH activity on gCDCA. In addition, the LC-MS approach allowed characterization of an additional activity, 7α-dehydroxylation, which was previously difficult to resolve by TLC.

These data demonstrate that the use of the LC-MS assay in identifying the bile acid metabolizing activities of bacterial species provides greater precision when designing a bacterial composition, such as designing a DBC to have a broad range of bile acid metabolizing activities. For example, treatment with this DBC may decrease levels of primary bile acids that promote *C. diff* germination while simultaneously increasing secondary bile acids that inhibit *C. diff* growth. Preferentially selecting strains that maximize these activities in a DBC may increase the efficacy of a composition for treating *C. diff* infections. In some cases, the use of the LC-MS assay or other assay with equivalent sensitivity can facilitate the design of a bacterial composition that covers a range of bile acid metabolizing activities with fewer species than would be used in a composition if a less sensitive assay were used. The minimization of the number of species in a composition can be a useful feature, for example, because it can reduce the number of bacteria that must be delivered making the dose easier to deliver (for example as an oral dosage form) and can be more efficient for manufacturing as well as composition costs as fewer separate fermentations must be carried out. In addition, when assayed *in vitro,* mixtures of bacteria can display bile metabolizing activities that the individual strains do not. This can allow modulation of compositions and composition size, as the mixtures may have activities the individual strains do not.

**Table 7**

| **Species** | **BSH** | | | | | | **HSDH** | | | **7a Dehyrdox** | |
|---|---|---|---|---|---|---|---|---|---|---|---|
| | **gCA** | **tCA** | **gCDCA** | **tCDCA** | **t-aMCA** | **t-bMCA** | **7a** | **3a** | **12a** | **CA** | **CDCA** |
| Blautia producta | - | - | - | - | - | - | - | - | - | - | - |
| Clostridium bolteae | + | + | + | + | + | + | - | + | - | - | - |
| Clostridium glycolicum | + | + | + | + | + | + | + | + | - | - | - |
| Clostridium dipsoricum | - | +/- | +/- | + | + | + | + | - | +/- | - | - |
| Clostridium hylemoeae | - | - | - | - | - | - | + | - | + | - | - |
| Clostridium innocuum | - | - | - | - | - | - | - | - | - | - | - |
| Clostridium oroticum | - | - | - | +/- | - | - | + | - | + | - | - |
| Eubacterium contortum | + | + | + | + | + | + | - | - | - | - | - |
| Turicibacter sanguinis | + | + | + | + | + | + | +/- | - | - | + | - |
| Lachnospiraceae sp. 11041 | - | +/- | - | +/- | + | - | +/- | - | - | - | - |
| Murimonas intestinii | - | - | - | - | - | - | - | - | - | - | - |
| Flavonifractor plautii | - | - | +/- | + | + | + | + | - | + | - | - |

**Table 8**

| | **BSH** | | | | | | **HSDH** | | | **7a Dehyrdox** | |
|---|---|---|---|---|---|---|---|---|---|---|---|
| **Species** | **gCA** | **tCA** | **gCDCA** | **tCDCA** | t- **aMCA** | **t-bMCA** | **7a** | **3a** | **12a** | **CA** | **CDCA** |
| Eubacterium contortum | | | | | | | - | - | +/- | - | - |
| Ruminococcus lactaris | + | + | + | + | + | + | - | - | - | - | - |
| Lachnospiraceae bacterium 5_1_5_57FAA | - | - | - | - | - | - | + | + | + | + | + |
| Clostridium hylemoeae | | | | | | | + | - | + | - | - |
| Clostridium scindens | | | | | | | + | + | + | + | + |
| Dorea formicigenerans | + | - | + | - | - | - | | | | | |
| Dorea longicatena | + | + | + | + | + | + | - | - | - | - | - |
| Clostridium orbiscindens | | | | | | | + | - | + | - | - |
| Clostridium sp_NML_04A032 | - | - | - | - | - | - | - | - | - | - | - |
| Clostridium viride | - | - | - | - | + | - | - | - | - | - | - |
| Pseudoflavonifractor capillosus | + | + | + | + | + | + | - | + | - | - | - |
| Clostridium innocuum | - | +/- | - | + | + | - | - | - | - | - | - |
| Clostridium_sp_HGF2 | +/- | +/- | + | +/- | +/- | - | - | - | - | - | - |
| Erysipelotrichaceae_bacterium_3_1_5 3 | - | - | - | - | - | - | + | - | - | - | - |
| Eubacterium_dolichum | - | - | - | - | - | - | - | | - | - | - |
| Eubacterium_sp_3_1_31 | - | +/- | - | +/- | - | - | - | + | + | - | - |

| **Species** | **BSH** | | | | | | **HSDH** | | | **7a Dehyrdox** | |
|---|---|---|---|---|---|---|---|---|---|---|---|
| | **gCA** | **tCA** | **gCDCA** | **tCDCA** | **t- aMCA** | **t-bMCA** | **7a** | **3 a** | **12 a** | **CA** | **CDCA** |
| Clostridium mayombei (Clostridium glycolicum) | + | + | + | + | + | + | | | | | |
| Clostridium_ghonii | + | + | + | + | + | + | + | + | - | + | + |
| Clostridium_glycolicum | + | + | + | + | + | + | | | | | |
| Clostridium_sordellii | + | + | + | + | + | + | - | +/ - | - | - | - |
| Blautia_producta | | | | | | | - | - | - | - | - |
| Blautia_schinkii | | | | | | | - | - | - | + | - |
| Blautia_sp_M25 | + | + | + | + | + | + | | | | | |
| Blautia_wexlerae | + | + | + | + | + | + | - | - | - | - | - |
| Lachnospiraceae_bacterium_6_1_63F AA | | | | | | | | | | | |
| Ruminococcus_hansenii | | | | | | | - | - | - | - | - |
| Ruminococcus_obeum | + | + | + | + | + | + | | | | | |
| Ruminococcus_sp_5_1_39BFAA | | | | | | | - | - | - | - | - |
| Eubacterium sp WAL 14571 | | | | | | | - | - | - | + | - |
| Clostridium bolteae (Clostridium clostridioforme) | | | | | | | - | + | - | - | - |

| **gCA** | **tCA** | **gCDCA** | **tCDCA** | **t- aMCA** | **t-bMCA** | **7a** | **3a** | **12a** | **CA** | **CDCA** | |
|---|---|---|---|---|---|---|---|---|---|---|---|
| Clostridium symbiosum | +/- | - | + | +/- | - | - | + | +/- | - | - | - |
| Clostridiales_sp_SM4_1 | - | +/- | - | +/- | +/- | - | | | | | |
| Clostridium_asparagiforme | - | +/- | - | + | + | + | + | +/- | + | - | - |
| Clostridium_lavalense | - | +/- | - | + | + | + | + | - | + | - | - |
| Lachnospiraceae_bacterium_5_1_63F AA | - | - | - | - | - | - | | | | | |
| Lachnospiraceae_bacterium_3_1_57F AA | - | - | - | - | - | - | | | | | |
| Clostridium butyricum | | | | | | | + | - | - | - | - |
| Clostridium disporicum | | | | | | | + | - | - | - | - |
| Clostridium paraputrificum | + | + | + | + | + | + | | | | | |
| Clostridium sp_7_2_43FAA | | | | | | | + | +/- | - | - | - |

### Example 6: Bacterial compositions selected for functional features

### Experimental design

To further investigate functionality of a DBC, additional DBCs were designed that possessed or did not possess selected features. The efficacy of additional test compositions was investigated using the mouse challenge model of CDI essentially as described *supra* (Chen et al., 2008, Gastroenterology 135:1984-1992). The mouse CDI model is used to demonstrate prevention of infection. In this model, mice receive an antibiotic pretreatment to create a dysbiosis in the gut that increases susceptibility to CDI. When challenged with orally administered *C*. *difficile* spores, mice exhibit symptoms of CDI including body weight loss, diarrhea and lethargy with peak disease between days 2-3 post-*C*. *difficile* inoculation. Infection can be lethal and death occurs during peak disease. In mice surviving the infection, symptoms are mainly resolved by day 6 after inoculation. Animals are kept in a bioBubble Clean Room or equivalent facility for the duration of the experiment.

Briefly, in these experiments, on Days -14 to -6, animals (female C57BL/6 mice, nine-ten weeks old) received an antibiotic cocktail in their drinking water consisting of 1% glucose, kanamycin (0.5 mg/mL), gentamicin (0.044 mg/mL), colistin (1062.5 U/mL), metronidazole (0.269 mg/mL), ciprofloxacin (0.156 mg/mL), ampicillin (0.1 mg/mL) and vancomycin (0.056 mg/mL). On Day -3, animals received a dose of 10 mg/kg clindamycin by oral gavage. On Day -1, test articles were centrifuged for five minutes at 12,100 RCF to pellet bacteria, decanted to remove the supernatant, and resuspended in sterile PBS. Test articles included FSV (a human FMT control), SC (a human-derived microbial spore composition to compare study efficacy across studies), and designed consortia of bacteria (DBCs). The control for the test article inocula was sterile PBS. Animals were dosed by oral gavage with a volume of 0.2 mL of appropriate treatment. Figure 2 provides a schematic of the study design.

On Day 0, mice were challenged by administration of approximately 4.5 log10 spores of *C*. *difficile* or sterile PBS (for the naive control arm) via oral gavage. Infection and its consequences were evaluated by daily assessment of mortality, weight loss and clinical signs and symptoms (the clinical score; scoring of lethargy, grooming, wet tail/abdomen, and hypothermia).

### Test articles

As described in Example 2, applicants have tested additional DBCs using the murine model described in Examples 1 and 2, *supra* to evaluate the efficacy of various orally administered microbial spore preparations for treating/preventing *Clostridium difficile* infection (CDI). Twenty-four additional compositions were tested as described in this Example at estimated doses of 1e5 spore colony forming units (SCFU) per individual species in a composition. Twenty of these compositions were selected such that in each of ten pairs, strains in one composition all shared a selected phenotype while in the corresponding composition, all strains lacked that phenotype. Some compositions tested are provided in Table 4. Negative controls included PBS alone as treatment and naïve animals (not infected with *C*. *difficile*). Positive controls included treatment with a slurry of healthy human feces (FSV), and treatment with a population of bacterial spores derived from human feces (SC) with doses from 1E4 to 1E7 SporQ per mouse.

### Results

Results of this experiment are presented in Table 9. Mortality in the untreated group of mice challenged with *C. difficile* was 20% and their mean minimum relative weight was 0.81. Surprisingly, it was found that some compositions were effective in inhibiting/preventing CDI in the murine model and some were at least as effective as a composition prepared from feces of healthy mice or a bacterial spore preparation prepared from healthy human feces (SC). In general, tested compositions varied in their ability to ameliorate the effects of *C. difficile* infection in the murine model, e.g., change in minimum body, lethality, and clinical signs. In some embodiments, a DBC effective for treating or preventing CDI is a DBC having a minimum relative weight P-value relative to the PBS control of <0.0001. Non-limiting examples of such compositions include DBC S1, DBC S2, DBC S4, DBC S6, and DBC S7. In other embodiments, a DBC effective for treating or preventing CDI is a DBC having a minimum relative weight P-value relative to the PBS control of <0.05. Other embodiments include a DBC having a specific feature having a low cumulative mortality score generally (e.g., DBC S6, which is composed of bacterial species reported to have a high rate of engraftment in a Ph 1b/2 study of multiply recurrent CDI subjects that received a complex microbial spore preparation derived from healthy human stool and a high prevalence in healthy human microbiota (HMP cohort). In some embodiments, a useful DBC having a specific feature and a low cumulative mortality compared to a DBC lacking the feature, for example, DBC S19 (selected for the feature of growth on sugar alcohols) and DBC S17 (selected for the feature of indole production), DBC S7 (selected for HDAC activity, which is indicative of SCFA production), and DBC S4 (strains in clostridial cluster XIVa).

In some cases, pairs designed to have contrasting features were both effective. For example DBC S21 and DBC S22 were designed to have the ability to use fucose as a nutrient source and the inability to use fucose as a nutrient source, respectively. Applicants note that the ability of a composition lacking a feature to be effective does not indicate that the feature is not useful, but merely that there may be alternate functions and pathways available to those species resulting in the phenotype of ameliorating *C. difficile* infection. In some embodiments, selection of species for a DBC that have such multiple capabilities is useful, for example, to limit the number of bacterial types (e.g., species) needed to provide multiple functions.

An example of a composition pair that had discordant results was DBC S19 and DBC S20, designed such that all members of DBC S19 could utilize the sugar alcohols that were tested and all members of DBC S20 are unable to utilize those sugar alcohols. In this case, DBC S19 was effective in preventing symptoms of CDI and DBC S20 was not; a comparison of the two DBCs showed they were statistically different from each other with respect to the minimum relative weight (p < 0.0001). Therefore, it can be advantageous to include one or more bacterial species that can utilize sugar alcohols in a composition, e.g., a composition for treating or preventing *C. difficile* infection.

Another example of a discordant DBC pair is DBC S17 (indole producing) and DBS S18 (non-indole producing). The data from this pair indicates that inclusion of at least one indole-producing bacterium in a DBC is useful in a composition, e.g., a composition for treating or preventing *C. difficile* infection. The results of the HDAC activity versus no HDAC activity pair is consistent with data provided in Example 7, *infra.* Moreover, HDAC plus bacteria (activity) as well as indole producing bacteria were effective preventing C. difficile-associated weight loss (maximum weight loss for HDAC positive and indole-positive DBC not significantly different from the PBS control that had not been challenged with C. difficile). Similarly, inclusion of a strain of Clostridium cluster XIVa can be a useful feature for such compositions. Although not as robust an effect as some (P = 0.82 for BSH compared to no BSH), the presence of BSH activity resulted in advantageous results and accordingly, the inclusion of one or more bacteria having BSH activity can be useful. Applicants note that these are useful guides to inclusion of bacteria in the design of a DBC, but are not to be construed as a basis to exclude bacteria that do not have such features.

Accordingly, in some embodiments of the invention, a DBC having certain defined features can effectively treat or prevent a range of features associated with CDI infection, for example one or more of weight loss, diarrhea, and mortality. In some cases, selected phenotypes discriminate between compositions that are efficacious and those that are not, while in yet other embodiments, pairs of compositions that contain strains with contrasting phenotypes are both efficacious.

These data demonstrate that a designed bacterial composition containing a relatively limited number of specific bacterial species can be used as a treatment for prevention of a dysbiosis, as demonstrated by treatment of CDI in a murine model.

Furthermore, such DBC compositions selected for certain features and shown to have a desired activity, e.g., amelioration of CDI, can be used as a basis for a more complex composition to which additional bacterial species are added, including those having additional selected features. DBC4, DBC6, and DBC9 are non-limiting examples of such compositions as are other compositions disclosed herein and that can be constructed using the guidance provided herein.

**Table 9**

| **Composition** | **Description** | **Mean Min. RelWeight** | **STDEV Min, RelWeight** | **Mean Clinical Score** | **STDEV Clinical Score** | **Cumulative Mortality** | **Min. Rel Weight P-value relative to PBS** | **Min. Rel Weight P-value relative to corresponding composition** |
|---|---|---|---|---|---|---|---|---|
| PBS | Vehicle | 0.81 | 0.05 | 2.4 | 0.8 | 20% | n/a | n/a |
| FSV | FMT Control | 0.99 | 0.02 | 0 | 0 | 0% | < 0.0001 | n/a |
| SC (1E7) | SC Control | 0.95 | 0.03 | 0 | 0 | 0% | < 0.0001 | n/a |
| SC (1E6) | SC Control | 0.95 | 0.03 | 0 | 0 | 0% | < 0.0001 | n/a |
| SC (1E5) | SC Control | 0.86 | 0.06 | 0 | 0 | 0% | 0.0679 | n/a |
| SC (1E4) | SC Control | 0.79 | 0.06 | 1.2 | 1.9 | 30% | 0.2758 | n/a |
| DBC S1 | DBC | 0.98 | 0.02 | 0 | 0 | 0% | < 0.0001 | n/a |
| DBC S2 | DBC | 0.86 | 0.07 | 0.4 | 1.3 | 10% | 0.0435 | n/a |
| DBC S3 | DBC | 0.81 | 0.09 | 2 | 2.1 | 50% | 0.8456 | n/a |
| DBC S4 | Cluster XIVa | 0.92 | 0.04 | 0 | 0 | 0% | 0.0015 | 0.0285 |
| DBC S5 | No Cluster IV or XIVa | 0.83 | 0.04 | 0.8 | 1.7 | 20% | 0.6126 | |
| DBC S6 | High Engrafting, High Prevalence | 0.93 | 0.06 | 0 | 0 | 0% | < 0.0001 | n/a |
| DBC S7 | HDAC Activity | 0.96 | 0.04 | 0 | 0 | 0% | < 0.0001 | 0.0032 |
| DBC S8 | No HDAC Activity | 0.87 | 0.05 | 0 | 0 | 0% | 0.0397 | |
| DBC S9 | BSH Activity | 0.91 | 0.05 | 0 | 0 | 0% | 0.0002 | 0.8214 |
| DBC S10 | No BSH Activity | 0.89 | 0.07 | 0.4 | 1.3 | 10% | < 0.0001 | |
| DBC S11 | 7a-HSDH Activity | 0.94 | 0.07 | 0 | 0 | 0% | < 0.0001 | 0.7806 |
| DBC S12 | No 7a-HSDH Activity | 0.93 | 0.05 | 0 | 0 | 0% | < 0.0001 | |
| DBC S13 | 3 or 12a-HSDH Activity | 0.92 | 0.05 | 0 | 0 | 0% | 0.0002 | 0.7001 |
| DBC S14 | No 3 or 12a-HSDH Activity | 0.91 | 0.07 | 0 | 0 | 0% | < 0.0001 | |
| DBC S15 | Growth on Amino Acids | 0.96 | 0.03 | 0 | 0 | 0% | < 0.0001 | 0.0998 |
| DBC S16 | No Growth on Amino Acids | 0.91 | 0.06 | 0 | 0 | 0% | 0.0001 | |
| DBC S17 | Indole Producing | 0.98 | 0.02 | 0 | 0 | 0% | < 0.0001 | 0.0022 |
| DBC S18 | Non-Indole Producing | 0.87 | 0.05 | 0.4 | 1.3 | 10% | 0.0298 | |
| DBC S19 | Growth on Sugar Alcohols | 0.89 | 0.09 | 0.4 | 1.3 | 10% | 0.0067 | 0.0026 |
| DBC S20 | No Growth on Sugar Alcohols | 0.79 | 0.05 | 2 | 2.1 | 40% | 0.2131 | |
| DBC S21 | Growth on Fucose | 0.92 | 0.04 | 0 | 0 | 0% | 0.0001 | 0.4219 |
| DBC S22 | No Growth on Fucose | 0.94 | 0.06 | 0.4 | 1.3 | 10% | < 0.0001 | |
| DBC S23 | Growth on NAG | 0.96 | 0.04 | 0 | 0 | 0% | < 0.0001 | 0.7969 |
| DBC S24 | No Growth on NAG | 0.97 | 0.07 | 0 | 0 | 0% | < 0.0001 | |
| Naïve | Naïve | 1 | 0 | 0 | 0 | 0% | < 0.0001 | n/a |

### Example 7: Functional properties of DBC species: histone deacetylase (HDAC) activity in DBC species

Applicants have identified inhibition of histone deacetylase activity via SCFA production as a useful function of some DBCs. Accordingly, Applicants assayed various bacteria for their ability to inhibit HDAC activity.

### HDAC inhibition assay can detect SCFAs in vitro

To determine whether a commercially available HDAC screening system was useful for testing HDAC inhibition by SCFAs, HDAC-Glo I/II assay kit Promega (see, Halley et al. 2011 J Biomol Screen 16:1227-35) was tested. For this experiment, the HeLa nuclear extract provided in the kit was used as the source of HDAC enzymes. The HDAC-Glo I/II kit was chosen because butyrate has been reported to inhibit most HDAC I and II family enzymes, with the exception of HDAC6 and HDAC10 (Davie 2003 J Nutrition 133:2485S-2493S). In this assay, deacetylation of the provided substrate by HDACs results in a luminescence signal that is detected with a suitable plate reader (Spectramax m5). HDAC inhibition decreases the luminescence signal compared to the no inhibitor control. The experiment was carried out according to the manufacturer's instructions as follows: 50 µL of HeLa nuclear extract diluted 1 :3000 in assay buffer was mixed with 50 µL of inhibitor solution (serial dilutions of SCFAs in assay buffer) and incubated for 30 minutes. 100 µL of developer solution were added to each well, and luminescence was measured after 45 minutes of incubation at room temperature. Results (not shown) demonstrated that butyrate and propionate are powerful HDAC inhibitors with IC50s of 0.8 and 2.4 mM, respectively, under the assay conditions. Lactate and acetate had minimal HDAC inhibition activity at the highest tested concentration (3.75 mM).

### HDAC inhibition assay to measure butyrate production in culture supernatants

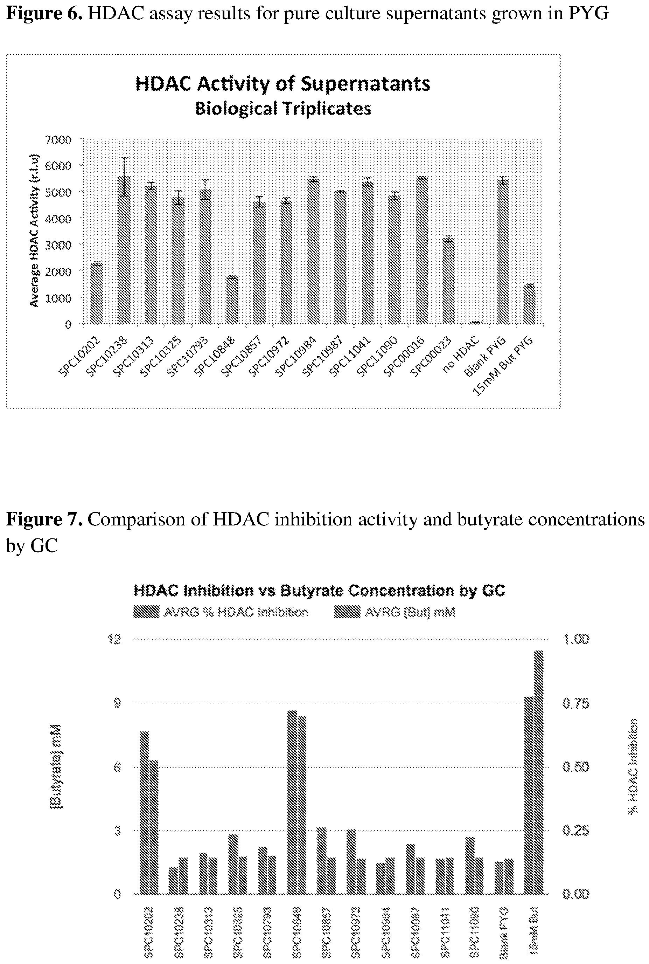

To examine the correlation between an HDAC assay and assay of an SCFA, twelve microbiome bacterial isolates from healthy human donors (HHD strains) and two publicly available strains not known to produce butyrate, *Escherichia coli* and *Bifidobacterium adolescentis,* were evaluated for their ability to inhibit HDAC activity and for butyrate production by gas chromatography. Cultures of all strains were grown in peptone yeast glucose medium (PYG, Anaerobe Systems) at 37°C for 5 days in triplicate. A blank culture (uninoculated sterile medium) and a sterile culture spiked with 15 mM butyrate were included as controls. After 5 days of incubation, microbial cells were removed by centrifugation (4000 rpm for 15 minutes) and supernatants (bacterial growth medium) were filtered through a 0.22 µM filter (Millipore). Supernatants were submitted for measurement of butyrate by gas chromatography (GC-FID by MRL, Woburn MA) and assayed for HDAC inhibition using the HDAC-Glo I/II Assay Kit (Promega). For this assay, 10 µL of culture supernatant, 40 µL of assay buffer, and 50 µL of diluted HeLa nuclear extract (1:3000 dilution) were incubated for 30 minutes prior to addition of 100 µL developer reagent. Luminescence was measured after 45 minutes at room temperature. The results indicated that the culture supernatants of three strains, as well as the supernatant spiked with 15 mM butyrate, had significant reduction in HDAC-derived luminescence under these conditions, i.e., demonstrated inhibition of HDAC (Figure 6). The assay is robust as was indicated by the small standard deviation between biological replicates. Because this assay is not specific for any particular HDAC inhibitor, it is useful for determining the general ability of a bacterial species or bacteria composition and may be useful, e.g., for identifying this ability even if a non-typical HDAC inhibitor (other than butyrate) is produced by the bacteria.

As indicated above, the bacterial supernatants were assayed for butyrate concentrations using GC. Comparison of the HDAC inhibition results to the butyrate concentrations measured by GC showed a significant correlation for the twelve HHD strains (Figure 7). These results indicate that the HDAC inhibition assay performed as described can detect butyrate concentrations as low as 6 mM in bacterial supernatants under these conditions. In some embodiments of the invention, more than 10 µL of supernatant is used in a reaction, resulting in a lower level of detection, e.g., 1 mM, 5 µM, or 1 µM.

### HDAC inhibition activity in supernatants of cultures grown in different carbon sources

To determine whether the HDAC inhibition was affected by the type and level of carbon source available to a bacterial species, HDAC inhibition by supernatants derived from a selection of bacterial species used in DBCs was determined. In these experiments, bacteria were grown in a variety of carbon sources including mono-, di-, polysaccharides, and porcine mucine. Briefly, 600 µL bacterial cultures in peptone/yeast extract medium (PY) supplemented with 0.5% of the selected carbon sources were inoculated in 96 deep well plates, grown for 4 days, then the microbial cells were pelleted by centrifugation, and 15 µL of the culture medium (supernatant) was used for the HDAC assay with HeLa nuclear extract as described above. Because HDAC activity is reduced at low pH (Latham et al. 2012 Nucl Acid Res 40:4794-803) and the microbial cultures had pH as low as 5, to ensure that the final pH of the assay was not reduced by the addition of supernatant, 10 µL of a 1M Tris pH 8 solution was added per well to bring the final Tris buffer concentration to 75 mM. Thus, assays were performed with 15 µL supernatant, 10 µL 1M Tris pH 8, 25 µL of assay buffer and 50 µL of diluted HeLa nuclear extract, which were preincubated for 30 minutes prior to the addition of developing reagent. Luminescence was measured after 30 minutes.

Results of these experiments are provided in Table 10, below. Under the experimental conditions, a sterile supernatant spiked with 15 mM butyrate resulted in 62% HDAC inhibition. Using 25% HDAC inhibition as cut off (1.8 mM butyrate under the experimental conditions), as above, only *Clostridium innocuum* and *Clostridium orbiscindens* exhibited HDAC inhibition activity when grown in glucose medium. However, when alternative nutrient sources are tested, 4 additional strains were positive for HDAC inhibition: *Clostridium glycolicum, Clostridium bolteae, Clostridium disporicum,* and *Eubacterium contortum. Murimonas intestinii* and *Clostridium oroticum* supernatants also showed HDAC inhibition although slightly below the 25% cutoff. These data demonstrate that to understand the ability of a species to produce HDAC inhibitors, it is important that HDAC inhibition assays be performed with a variety of carbon sources.

Furthermore, these data indicate that it can be advantageous to provide a composition that can use a broad range of carbon sources, thereby increasing the likelihood that, when administered as a treatment for an indication in which inhibition of HDAC is desirable, the composition will produce HDAC inhibitors. Such indications include those in which production of short chain fatty acids is desirable, e.g., for the treatment or prevention of C. *difficile* infection. These data also provide support for the use of a prebiotic that is a nutrient source for one or more species in a DBC.

**Table 10: HDAC inhibition activity in cultures grown with diverse carbon sources**

| **Clade** | **Species** | **HDAC Inhibition basal** | **HDAC Inhibition gluc** | **HDAC Inhibition fucose** | **HDAC Inhibition sucrose** | **HDAC Inhibition pectin** | **HDAC Inhibition fos/inulin** | **HDAC Inhibition starch** | **HDAC Inhibition mucin** |
|---|---|---|---|---|---|---|---|---|---|
| clade_139 | Clostridium innocuum | 0.27 | 0.54 | 0.28 | 0.38 | 0.54 | 0.48 | 0.28 | 0.34 |
| clade_195 | Clostridium glycolicum | 0.42 | 0.08 | 0.42 | 0.42 | 0.15 | 0.10 | 0.43 | 0.54 |
| clade_90 | Clostridium hylemonae | 0.10 | 0.06 | 0.06 | 0.09 | 0.16 | 0.07 | 0.09 | 0.11 |
| clade_233 | Clostridium boiteae | 0.20 | 0.12 | 0.29 | 0.16 | 0.22 | 0.13 | 0.25 | 0.30 |
| clade_244 | Clostridium disporicum | 0.12 | 0.08 | 0.37 | 0.21 | | 0.19 | 0.11 | 0.17 |
| clade_101 | Flavonifractor piautii | 0.51 | 0.55 | 0.50 | 0.51 | 0.55 | 0.57 | 0.66 | 0.63 |
| clade_197 | Blautia producta | 0.11 | 0.06 | 0.14 | 0.06 | 0.10 | 0.07 | 0.11 | 0.20 |
| clade_238 | Murimonas intestini | 0.07 | 0.13 | 0.23 | 0.07 | 0.11 | 0.08 | 0.10 | 0.13 |
| clade_290 | Turicibacter sanguinis | 0.04 | 0.06 | 0.05 | 0.05 | 0.06 | 0.04 | 0.04 | 0.07 |
| clade_86 | Eubacterium contortum | 0.08 | 0.15 | 0.32 | 0.15 | 0.13 | 0.09 | 0.06 | 0.09 |
| clade_241 | Lachnospiraceae sp. 11041 | 0.00 | 0.00 | 0.00 | -0.02 | 0.07 | 0.01 | 0.01 | 0.06 |
| clade_86 | Clostridium oroticum | 0.06 | 0.07 | 0.19 | 0.09 | 0.11 | 0.08 | 0.05 | 0.08 |

### SCFA profile of bacterial strains

The same bacterial culture supernatants used for HDAC inhibition assays described *supra* were submitted to GC-MS analysis to determine which SCFAs underlie the observed inhibition of HDAC. The method employed (MSOmics, Denmark) measures formate, acetate, propionate, butyrate, isobutyrate (2-methyl-propionate), isovalerate (3-methyl-butyrate), valerate (pentanoate), 4-methyl-pentanoate, caproate (hexanoate), and heptanoate. The GC-MS results (Table 11) demonstrate that there is an excellent correlation between HDAC inhibition results and production of SCFA associated with HDAC inhibition in the literature. Numbers in Table 11 indicate detected SCFA concentration. B=butyrate; P=propionate; IB=isobutyrate, and IV=isovalerate.

*Clostridium innocuum* and *Flavonifractor plautii* produced butyrate in all media tested. For *Clostridium innocuum,* the concentration of butyrate was higher on sucrose, pectin, and FOS/inulin than in basal medium indicating that this strain can produce butyrate from these complex polysaccharides. *Flavonifractor plautii* produced additional butyrate on FOS/inulin, starch, and mucin, and to a lesser extent, with glucose. Accordingly, inclusion of both species in a composition will enable butyrate production from a wide range of complex substrates.

*C. glycolicum* produced isovalerate, isobutyrate and propionate. Isobutyrate and isopropionate are branched chain fatty acids (BCFAs) produced in the dissimilatory metabolism of branched amino acids (valine and leucine respectively), and have been shown to exhibit HDAC inhibitory activity in HeLa cells (Waldecker et al., 2008 J Nutritional Biochem 19:587-593). The inclusion of such strains, e.g., as in DBC6, for which *C. glycolicum* is the only strain shown to be capable of utilizing valine and leucine as a carbon source (*supra*) and thus can provide a strain that can compete with *C. difficile* for these substrates while modulating HDAC activity. Applicants note that 2-methyl-butyrate, the product of isoleucine metabolism, was not part of the SCFA panel in these experiments. However, *C. glycolicum* also can use Ile as carbon source as did the *C. difficile* strains tested in the carbon source panel. Therefore, *C. glycolicum* can also be used in a DBC as a source of 2-methyl-butyrate.

*C. bolteae, C. disporicum, Eubacterium contortum, Murimonas intestini,* and *C. oroticum* produced propionate but only when supplied with fucose as a carbon source. Fucose is an important component of glycans in the intestinal epithelium lumen and mucosal secretions. Intestinal fucose has been identified as a mediator of host-microbiome symbiosis (Pickard and Chervonsky 2015 J Immunol 194:5588-5593). Accordingly, including at least one fucose-utilizing species in a DBC can support colonization of the host by such bacteria, and propionate utilization near the epithelial surface can have significant beneficial effects in epithelial barrier function.

Growth of *F. plautii* in PY (basal) supplemented with glucose, FOS/In, start, or mucin result in increased levels of butyrate compared to levels produced when grown only in the basal medium. Similarly, *C. innocuum* grown in medium supplemented with glucose or pectin and to lesser extents in sucrose or FOS/In produce increased levels of butyrate.

In some cases, a species produces less of an SCFA when the basal medium is supplemented with a carbon source. Without committing to any particular theory, it may be that in such cases, the supplemental carbon source is a preferred source to the extent that bacteria shift their metabolism to use of the supplemental carbon source and thereby make less of the SCFA detected when only basal medium is used for growth.

These data indicate that selected species can be used to increase the likelihood that an SCFA will be produced when a bacterium is administered in a DBC. Furthermore, the data related to HDAC inhibition and production of SCFAs using various carbon sources support the use of DBCs as opposed to single species compositions for treatments in which it is desirable to express HDAC inhibitory molecules, e.g., SCFAs or other unidentified molecules produced by a bacterium.

**Table 11: SCFA profiles of bacterial species grown in diverse carbon sources. Numbers indicate measured concentration (mM) in culture supernatants for butyrate (B), propionate (P), isobutyrate (IB) or isovalerate (IV). Limits of detection (LOD) were 0.3 mM (B), 0.2 mM (P), 0.3 mM (IB), and 0.6 mM (IV). Empty cells indicate that concentration was below LOD.**

| **Species** | **Basal** | **Glucose** | **Fucose** | **Sucrose** | **Pectin** | **FOS/in** | **Starch** | **Mucin** |
|---|---|---|---|---|---|---|---|---|
| Clostridium innocuum | 4.5 (B) | 13.3 (B) | 5.4 (B) | 7.3 (B) | 9.7 (B) | 8.5 (B) | 3.9 (B) | 4.0 (B) |
| | 3.1 (P) | 0.3 (P) | 3.2 (P) | 1 3.0 (P) | 0.4 (P) | 0.2 (P) | 3.0 (P) | 3.5 (P) |
| | 6.5 (IB) | 0.5 (IB) | 6.7 (IB) | 16.6 (IB) | 0.3 (IB) | 0.3 (IB) | 6.5 (IB) | 6.8 (IB) |
| Clostridium glycolicum | 13.7 (IV) | 1.8 (IV) | 13.9 (IV) | 1 13.2 (IV) | 0.7 (iv) | 0.8 (IV) | 13.6 (IV) | 13.7 (IV) |
| Clostridium hylemonae | | | | | | | | |
| Clostridium bolteae | | | 6.9 (P) | | | | | |
| Clostridium disporicum | | | 3.1 (P) | | | | | |
| Flavonifractor plautii | 9.2 (B) | 11.0 (B) | 8.7 (B) | 8.8 (B) | 8.9 (B) | 12.4 (B) | 16.3 (B) | 12.2 (B) |
| Blautia producta | | | | | | | | |
| Murimonas intestini | | | 6.9 (P) | | | | | |
| Turicibacter sanguinis | | | | | | | | |
| Eubacterium contortum | | | 8.9 (P) | | | | | |
| Lachnospiraceae sp. 11041 | | | | | | | | |
| Clostridium oroticum | | | 4.9 (P) | | | | | |

### Example 8. Functional properties of DBC species; production of indole and other tryptophan metabolites

As shown in Example 6, a bacterial composition that can produce indole or indole derivatives s was effective in ameliorating *C. difficile* infection compared to a bacterial composition that did not produce such indoles. In view of this discovery, Applicants developed an assay for indole and indole metabolite production that can be used to test bacteria for these activities and were able to identify bacterial species having the activity. Such species are useful additions to construct a DBC that can be used, e.g., to treat or prevent a *C. difficile* infection.

### Assay for indole and indole-metabolite production in SER-262 bacterial supernatants

For the indole test, a drop of Indole Reagent (Anaerobe Systems, Morgan Hill, CA) was added to 100 µL of bacterial cultures of bacterial strains grown in eight different carbon sources in duplicate. Of the 12 strains tested, only *Clostridium glycolicum* and *Flavonifractor plautii* were positive in the indole test under these conditions (data not shown). *F. plautii* cultures supplemented with each of the 8 carbon sources utilized in Example 7 turned dark blue, which is characteristic of the presence of indole. *C. glycolicum* turned red (different colors have been described for distinct indole derivatives in this test (Lombard and Dowell, 1983 J Clin Microbiol 18:609-613)) in all C-sources except glucose, pectin, and FOS/inulin. For these strains, the pattern of indole phenotype by carbon source is identical to that of the HDAC and SCFA phenotypes and not correlated with biomass, suggesting overlapping regulation. Supernatants were also assayed again using a spot test (4 µL of supernatant spotted in a paper soaked with Indole Reagent (Anaerobe Systems, Morgan Hill, CA) along with purified indole, 3-methyl-indole, indole-3-butyric acid, tryptamine, and indole-3-propionic acid as positive controls). Indole and the supernatant of *F. plautii* produced light blue color, while *C. innocuum,* indole-3-propionic acid, 3-methyl-indole, indole-3-butyric acid, and tryptamine produced purple spots. These results imply that *F. plautii* is an indole producer while *Clostridium innocuum* is a producer of an indole derivative that cannot be specified with this method. Evidence for a role of indoles in immunomodulation and epithelial barrier function has been reported in the literature. In view of this and of the data presented herein, it is useful to include at least one or both of these two strains in a DBC designed to treat or prevent infection, e.g., *C. difficile* infection. Without committing to any particular theory, Applicants believe the inclusion of such species in a DBC is useful for immunomodulation and restoration of epithelial barrier function.

### Example 9: DBC clinical trial

A clinical trial is conducted using a DBC in human subjects diagnosed with a primary (first) episode of CDI. An example of such a trial is described at clinicaltrials.gov, trial number NCT02830542.

An effective DBC reduces recurrence of one or more CDI signs and/or symptoms in this population.

Other embodiments are within the scope of the following claims.

Preferred embodiments of the present invention are described below and are referred to as embodiments E1 to E20:
1. A composition comprising a viable population of bacteria, the viable population containing the bacterial species of DBC 1, DBC 2, DBC 3, DBC 4, DBC 5, DBC 6, DBC 7, DBC 8, or DBC 9 of Table 3, or DBC S1, DBC S2, DBC S4, DBC S5, DBC S6, DBC S7, DBC S8, DBC S9, DBC S10, DBC S11, DBC S12, DBC S13, DBC S14, DBC S15, DBC S16, DBC S17, DBC S18, DBC S19, DBC S20, DBC S21, DBC S22, DBC S23, or DBC S24 of Table 4.
2. The composition of E1, wherein the 16S rDNA of each bacterial species in the DBC has at least 97% identity to at least one 16S rDNA sequence in Figure 1.
3. The composition of E1, wherein the composition consists of a viable population of bacterial species of DBC 1, DBC 2, DBC 3, DBC 4, DBC 5, DBC 6, DBC 7, DBC 8, DBC 9, DBC S1, DBC S2, DBC S4, DBC S5, DBC S6, DBC S7, DBC S8, DBC S9, DBC S10, DBC S11, DBC S12, DBC S13, DBC S14, DBC S15, DBC S16, DBC S17, DBC S18, DBC S19, DBC S20, DBC S21, DBC S22, DBC S23, or DBC S24.
4. The composition of E3, wherein the 16S rDNA of each bacterial species in the DBC has at least 97% identity to at least one 16S rDNA sequence in Figure 1.
5. The composition of any one of E1 to 4, wherein the population of bacterial species is able to utilize at least 90% of the carbon sources listed in Figure 4.
6. A composition comprising a population of at least five species of viable bacteria, wherein at least one of the species of the population has one or more features selected from the group consisting of: (i) utilization of one or more carbon sources utilized by a pathogenic microorganism, (ii) production of an inhibitor of histone deacetylase (HDAC), (iii) production of indole or other tryptophan metabolites, and (iv) production of an enzyme that functions in bile acid regulation.
7. The composition of E6, wherein the pathogenic microorganism is *C. difficile* and the population comprises one or more species of viable bacteria that can utilize at least five different *C. difficile* carbon sources.
8. The composition of E7, wherein the *C. difficile* carbon sources are selected from the group consisting of: taurocholate, glycocholate, glycochenodeoxycholate, taurochenodeoxycholate, cholate, chenodeoxycholate, and deoxycholate.
9. The composition of E6, wherein the population comprises one or more species of viable bacteria that produce an HDAC inhibitor selected from the group consisting of: butyrate, isovalerate, isobutyrate, propionate, and 2-methyl-butyrate.
10. The composition of E6, wherein the population comprises one or more species that produces one or more enzyme selected from the group consisting of bile salt hydrolase (BSH), 3-α-hydroxysteroid dehydrogenase (3-α-HSDH), 7-α-hydroxysteroid dehydrogenase (7-α-HSDH), and 12-α-hydroxysteroid dehydrogenase (12-α-HSDH).
11. A composition comprising at least one species from each of clades 86, 90, 101, 139, 195, 197, 206, 233, 238, 241, 244, and 290, and optionally a species from claim 202.
12. The composition of any one of E6 to 11, wherein the composition comprises 5, 6, 7, 8, 9, 10, 11, or 12 species of bacteria.
13. The composition of any one of E1 to 12, wherein at least 75% of the bacteria are spores.
14. A formulation comprising the composition of any one of E1 to 13 and a pharmaceutically acceptable excipient.
15. The formulation of E14, wherein the pharmaceutically acceptable excipient comprises glycerol, polyethylene glycol, or cocoa butter.
16. The formulation of E14 or 15, wherein the formulation is in a capsule or tablet.
17. Use of a pharmaceutically effective amount of a composition of any one of E1 to 13 for preventing or treating a dysbiosis in a subject at risk of or diagnosed with a dysbiosis.
18. The use of E17, wherein the total concentration of bacteria in the composition is 10e1 to 10e9.
19. The use of E17 or 18, wherein the bacteria of the composition are provided in 1, 2, 3, 4, or 5 capsules.
20. Use of a therapeutically effective amount of a composition of any one of E1 to 13 for treating a *C. difficile* infection, or preventing a *C. difficile* infection, or preventing recurrence of a *C. difficile* infection.

## Claims

1. A composition comprising a viable population of bacteria, wherein the viable population comprises the bacterial species of DBC S13, DBC S15, DBC S4, DBC S7, DBC S21, DBC 1, DBC 2, DBC 3, DBC 4, DBC 5, DBC 6, DBC 7, DBC 8, DBC 9, DBC S1, DBC S2, DBC S5, DBC S6, DBC S8, DBC S9, DBC S10, DBC S11, DBC S12, DBC S14, DBC S16, DBC S17, DBC S18, DBC S19, DBC S20, DBC S22, DBC S23, or DBC S24.

2. The composition of claim 1, wherein the 16S rDNA of each bacterial species in the DBC has at least 97% identity to at least one 16S rDNA sequence in Figure 1.

3. The composition of claim 1 or 2, wherein at least 75% of the population of viable bacteria are spores.

4. The composition of claim 3, wherein at least 80%, at least 85%, at least 90%, at least 95%, at least 97%, at least 99%, or 100% of the population of viable bacteria are spores.

5. The composition of any one of claims 1 to 4, wherein the composition comprises at least 2, 3, 4, 5, 6, 7, 8, 9, 10, 11, 12, 13, 14, 15, 16, 17, 18, 20, 25, or 30 species of viable bacteria.

6. A formulation comprising the composition of any one of claims 1 to 5 and a pharmaceutically acceptable excipient.

7. The formulation of claim 6, wherein the pharmaceutically acceptable excipient comprises glycerol, polyethylene glycol, or cocoa butter.

8. The formulation of claim 6 or 7, wherein the formulation is in a capsule or tablet.

9. The composition of any one of claims 1 to 5, or the formulation of any one of claims 6 to 8, for use in a method of preventing or treating a dysbiosis in a subject in need thereof.

10. The composition or formulation for use of claim 9, wherein the dysbiosis is associated with (i) a *Clostridium difficile* infection, and/or (ii) a vancomycin-resistant *Enterococcus* infection.

11. The composition of any one of claims 1 to 5, or the formulation of any one of claims 6 to 8, for use in a method of treating a *C. difficile* infection, preventing a *C. difficile* infection, or preventing recurrence of a *C. difficile* infection in a subject in need thereof.

12. The composition or formulation for use of any one of claims 9 to 11, wherein the total concentration of bacteria is 10e1 to 10e9.

13. The composition or formulation for use of any one of claims 9 to 12, wherein the population of viable bacteria are provided in 1, 2, 3, 4, or 5 capsules or tablets.

14. The composition or formulation for use of any one of claims 9 to 13, wherein the subject has received an antibiotic treatment prior to administration of the composition or the formulation.

15. The composition or formulation for use of any one of claims 9 to 14, wherein the composition or the formulation is for oral administration in the form of a medicament.
